# EUROPÄISCHE PATENTANMELDUNG

(11) **EP 3 301 111 A1**
(43) Veröffentlichungstag der Anmeldung: **04.04.2018**
(21) Anmeldenummer: 16192002.0
(22) Anmeldetag: 02.10.2016
(51) Int. Cl.: C07K 14/415, C12N 15/82

(54) **PFLANZE DER GATTUNG TRITICUM, IN DER DAS TDF-GEN DURCH EIN MARKERGEN INAKTIVIERT IST**

(71) Anmelder: KWS SAAT SE, 37574 Einbeck (DE)
(72) Erfinder: SCHMIDT, Klaus, 31246 Lahstedt (DE)

(57) **Zusammenfassung**

Die Erfindung betrifft eine Pflanze der Gattung *Triticum,* in der ein Markergen in mindestens ein Allel des für das Protein *tapetal development and function* (TDF) codierenden Gens insertiert ist, wodurch das Allel inaktiviert ist. Desweiteren betrifft die Erfindung Nachkommen dieser Pflanze sowie Teile und Samen dieser Nachkommen, die ein inaktiviertes TDF-Gen enthalten, und Verfahren zu ihrer Herstellung. Die erfindungsgemäße Pflanze kann zur Herstellung von Pflanzen verwendet werden, bei denen die Sterilität bzw. Fertilität mit einem anderen phänotypischen Merkmal gekoppelt ist.

## Beschreibung

Die Erfindung betrifft eine Pflanze der Gattung *Triticum*, in der ein Markergen in mindestens ein Allel des für das Protein *tapetal development and function* (TDF) codierenden Gens insertiert ist, wodurch das Allel inaktiviert ist. Desweiteren betrifft die Erfindung Nachkommen dieser Pflanze sowie Teile und Samen dieser Nachkommen, die ein durch Insertion inaktiviertes TDF-Gen enthalten, und Verfahren zu ihrer Herstellung. Die erfindungsgemäße Pflanze kann zur Herstellung von Pflanzen verwendet werden, bei denen die Sterilität bzw. Fertilität mit einem anderen phänotypischen Merkmal gekoppelt ist.

Hybriden zeigen in der Regel deutlich höhere Erträge als Liniensorten, so dass bei zahlreichen Kulturpflanzen die Hybridzüchtung für die Sortenentwicklung genutzt wird. Die kommerzielle Nutzung von Hybridweizen / Hybriden der Gattung *Triticum* (Weizen) ist sehr begrenzt, da alle zur Zeit zur Verfügung stehenden Systeme mit erheblichen, technischen Problemen zu kämpfen haben.

Voraussetzung für die Entwicklung von Hybridsorten ist die Verfügbarkeit von männlich sterilen Linien, um die Befruchtung gezielt steuern zu können.

Häufig werden hierzu sogenannte CMS-Linien verwendet, die eine Mutation im mitochondrialen Genom aufweisen, was zu einer cytoplasmatisch bedingten, männlichen Sterilität (CMS) führt. Da diese Linien keinen Pollen mehr produzieren, können sie gezielt mit Pollen einer anderen Linie bestäubt werden, so dass auf den CMS-Linien das Hybridsaatgut geerntet werden kann.

Ein Problem stellt die Erhaltung und Vermehrung der CMS-Linien dar, da diese nicht durch Selbstbestäubung vermehrt werden können. Für die Erhaltung dieser Linien benötigt man daher sogenannte Maintainer-Linien. Diese Linien sind im Kerngenom genetisch identisch mit der männlich sterilen Linie, jedoch tragen sie nicht die mitochondriale Mutation, so dass diese Linien fertil sind. Durch Nutzung dieser Maintainer-Linie als Vater und der CMS-Linie als Mutter kann auf den Mutterpflanzen Saatgut erhalten werden, welches weiterhin steril ist, da der Sterilitätsphänotyp ausschließlich maternal vererbt wird. Diese maternale Vererbung bedingt aber auch, dass das Hybridsaatgut von CMS-Linien immer im Sterilitätsplasma vorliegt und somit männlich sterile Pflanzen hervorbringt. Bei Kulturpflanzen, in denen das Ernteprodukt ein vegetatives Organ ist, z.B. Zuckerrübe ist dies kein Problem. Bei Kulturarten, bei denen Saatgut das Ernteprodukt ist, muss durch Einfügen eines sogenannten Restorergens die Fertilität der Hybride wiederhergestellt werden. Das Restorergen ist kerncodiert und ist in der Lage die durch die Mutation im Mitochondrium hervorgerufenen Defekte in der Entwicklung der männlichen Blütenorgane zu kompensieren, und somit die Fertilität wiederherzustellen. Häufig ist es schwer, gut wirksame Restorergene zu identifizieren und für die Züchtung zu nutzen. Die bekannten, CMS-basierten Systeme sind in Weizen aufgrund der schwierigen Saatgutproduktionen (CMS-Linie; Maintainer-Linie; Restorerlinie) zu kostenintensiv, so dass eine kommerzielle Anwendung der Systeme in der Vergangenheit gescheitert ist. In geringem Maßstab werden alternativ Gametozide kommerziell genutzt: Durch Applikation einer chemischen Substanz wird die Entwicklung der männlichen Blütenorgane gestört, so dass es zu einer männlichen Sterilität kommt. Das Problem hierbei ist jedoch das enge Ausbringungsfenster des Gametozids im Feld. Durch ungeeignete Wetterbedingungen kann die Applikation des Gametozids verhindert werden, was somit zu einem Totalausfall der Hybridsaatgutproduktion führt (Whitford et al., 2013).

Neben den CMS-basierten, männlich sterilen Phänotypen sind ferner Systeme bekannt, die auf Mutationen im Kerngenom basieren und eine sogenannte Genetic Male Sterility (GMS) hervorrufen. Solche Mutationen sind meist rezessiv, so dass der männlich sterile Phänotyp nur im homozygot, rezessiven Zustand ausgebildet wird. Dadurch ist eine einfache Restoration der Fertilität gegeben, da durch Einbringen eines nicht mutierten Allels des GMS-Gens (z.B. durch die Vaterpflanze bei der Hybridsaatgutproduktion) die männliche Sterilität wieder aufgehoben werden kann, und die Hybriden somit voll fertil sind. Das Problem liegt allerdings auch hier in der Erhaltung und Vermehrung der männlich sterilen Linien (Kempe und Gils, 2011). Durch Kreuzung der sterilen GMS-Linien mit Pollen einer heterozygoten Mutante werden in der Nachkommenschaft immer nur maximal 50% der Nachkommen steril sein. Der sterile Phänotyp ist allerdings erst zum Zeitpunkt der Blüte festzustellen. Somit können GMS-Linien normalerweise nicht für die großflächige Produktion von Hybriden eingesetzt werden.

Die Nutzung von GMS-Linien ist nur dann möglich, wenn zu einem sehr frühen Zeitpunkt in der Entwicklung der Pflanzen eine Unterscheidung zwischen homozygot sterilen und heterozygotfertilen Pflanzen möglich ist. Um genetische Analysen in einem frühen Stadium der Pflanzenentwicklung, also vor Beginn der Blütenentwicklung, durchführen zu können, ist es jedoch zwingend notwendig, die Position der Mutation im Kerngenom der männlich sterilen Linie sowie die Sequenz des mutierten Gens zu kennen, um entsprechende DNA-Marker darauf anwenden zu können. Mit Hilfe dieser Marker ist es dann möglich sein, bereits an sehr jungen Sämlingen oder in einem noch früheren Stadium genetische Untersuchungen durchzuführen, auf deren Grundlage die fertilen Pflanzen verworfen und die sterilen Pflanzen für die Hybridsaatgutproduktion genutzt werden könnten.

US 2006288440 A1 offenbart ein System zur Nutzung von kerncodierter, männlicher Sterilität für Mais. Das System mit dem Namen SEED PRODUCTION TECHNOLOGY (SPT) basiert darauf, dass eine sterile Maismutante, deren Sterilität durch eine Mutation in einem bekannten, kerncodierten Gen verursacht wird, durch Einfügen eines Transgens restauriert werden kann. Das Transgen enthält dabei das nicht mutierte Allel des Sterilitäts- bzw. Fertilitätsgens, so dass das Transgen als Wildtyp-Allel fungiert. Das Fertilität restaurierende Transgen ist genetisch gekoppelt mit einem weiteren Transgen, welches die Weitergabe des Transgens über den Pollen verhindert (Pollenkiller). Dadurch entsteht bei Selbstbefruchtungen der transgenen Linie nur Saatgut, das hemizygot für das die Fertilität wiederherstellende Transgen ist. Für das Transgen homozygotes Saatgut kann nicht gebildet werden. Dies ist sehr wichtig für die Effizienz des Systems, da der Maintainer nur im hemizygoten Zustand sowohl fertiles, transgenes Saatgut als auch steriles, nicht transgenes Saatgut nach Selbstbefruchtung erzeugen kann. Liegt das Transgen hingegen im homozygoten Zustand vor, würden alle Nachkommen wieder fertil sein. Das Transgen enthält des Weiteren eine Expressionskassette, die zur Rotfluoreszenz der Samen führt. Somit lassen sich transgene von nicht transgenen Samen einfach unterscheiden. Da die transgenen Samen fertil sind, kann auf Basis der Fluoreszenz auch eine Separierung in fertile und sterile Pflanzen stattfinden. Durch Aussaat der nicht transgenen Samen erhält man somit die für die Hybridproduktion notwendigen Mutterpflanzen, die transgenen Samen können zum einen als Vaterlinie für die weitere Vermehrung der Mutterlinie verwendet werden (Maintainerlinie) und auch durch einfache Selbstung vermehrt werden (Whitford et al., 2013).

Das SPT System kann theoretisch in allen Pflanzenarten angewandt werden. Voraussetzung ist jedoch das Vorhandensein einer GMS-Linie sowie die Kenntnis über die Struktur desjenigen Gens, welches infolge einer Mutation die männliche Sterilität verursacht und somit für den GMS-Phänotyp verantwortlich ist.

Für Weizen sind kerncodierte Sterilitätssysteme auf Basis des Genlocus MS1 (Cornerstone) entwickelt worden (Zhou et al., 2006). Neben dem MS1-Locus sind weitere genomische Loci im Weizen bekannt, deren Modifikation zu männlicher Sterilität führen (Whitford et al., 2013). Bisher konnte nur im Fall von MS1 das für die Sterilität verantwortliche Gen identifiziert und kloniert werden. Eine kommerzielle Anwendung des MS1-Locus oder eines anderen Locus im Kontext einer SPT-System-basierten Hybridweizenherstellung ist bis heute nicht bekannt. Es scheint, dass die Etablierung mit weiteren technischen Schwierigkeiten verbunden ist.

Es ist daher die Aufgabe der vorliegenden Erfindung, eine Pflanze der Gattung *Triticum* zur Verfügung zu stellen, die eine Modifikation eines männlichen Fertilitätsgenes im Kerngenom aufweist und bei der eine phänotypische Unterscheidung und Selektion zwischen sterilen und fertilen Pflanzen möglich ist, und welche die Etablierung eines völlig neuen Systems zur effizienten und kommerziell-attraktiven Erzeugung von hybriden Weizenpflanzen ermöglicht.

Die Aufgabe wird durch den Gegenstand der Patentansprüche gelöst.

Die vorliegende Erfindung beruht auf der erstmaligen Klonierung eines Weizengens, dessen Inaktivierung zu einem männlich sterilen Phänotyp im Weizen führt. Die Sterilität liegt dabei rezessiv vor. Die durch die Inaktivierung des für *tapetal development and function* (TDF) codierenden Gens erzeugte männliche Sterilität wird erfindungsgemäß mit einer weiteren, phänotypischen Eigenschaft der Pflanze (z.B. Herbizidtoleranz) gekoppelt, wobei die Eigenschaft leicht phänotypisch selektiert werden kann (z.B. Herbizidtoleranz, morphologische Besonderheiten, Farbstoffe wie Anthocyane in Teilen der Samen).

Durch das von der vorliegende Erfindung zur Verfügung gestellte Wissen über das für die Sterilität verantwortliche Gen in Weizen können sehr spezifische DNA-Marker entwickelt werden, die zum molekularen Nachweis einer Modifikation des Gens (beispielsweise durch Insertion) eingesetzt werden können. Dies ist insbesondere auch in einem sehr frühen Stadium der Pflanzenentwicklung möglich. Gleichzeitig kann das Wissen über das Sterilitätsgen aber auch Anwendung finden bei der Unterscheidung von Samen, die sterile oder fertile Pflanzen hervorbringen. Dazu wird eine Gewebeprobe des Samens mittels *Seed clipping* Technologie abgenommen und mittels DNA Markern untersucht. Dadurch können die Samen sortiert werden in solche, die fertile bzw. solche, die sterile Pflanzen hervorbringen.

Die vorliegende Erfindung betrifft eine Pflanze der Gattung *Triticum*, in der ein Markergen in mindestens ein Allel des für *tapetal development and function* (TDF) codierenden Gens eines Subgenoms der Pflanze insertiert (integriert) ist und das Allel inaktiviert ist.

Eine "Pflanze der Gattung *Triticum"* ist beispielsweise eine Pflanze ausgewählt aus einer der Spezies: *Triticum aestivum, Triticum aethiopicum, Triticum antiquorum, Triticum baeoticum, Triticum carthlicum, Triticum compactum, Triticum dicoccoides, Triticum dicoccon, Triticum durum, Triticum ispahanicum, Triticum macha, Triticum monococcum, Triticum parvicoccum, Triticum* x *petropavlovskyi, Triticum polonicum, Triticum sinskajae, Triticum spelta, Triticum sphaerococcum, Triticum tetraurartu, Triticum timopheevii, Triticum turanicum, Triticum turgidum, Triticum urartu, Triticum vavilovii* oder *Triticum* × *zhukovskyi.*

Die Begriffe "für *tapetal development and function* codierendes Gen" / "für TDF codierendes Gen" und "TDF-Gen" werden hierin synonym verwendet. "TaTDF" bezeichnet das TDF-Gen aus Weizen. Gemeint ist jeweils ein funktionelles TDF-Gen bzw. ein Allel davon; dieses kann auch als "aktives TDF-Gen" bzw. "aktives TDF-Allel" bezeichnet werden, in Abgrenzung zum "inaktivierten TDF-Gen" oder "inaktivierten TDF-Allel".

Weizen verfügt über drei Subgenome: Subgenom A, B und D. Das TDF-Gen ist in jedem Subgenom einmal vorhanden, also insgesamt dreimal. Insgesamt gibt es in Weizen folglich sechs Allele des TDF-Gens.

Exemplarische genomische Sequenzen des Wildtyp Weizen-TDF-Locus aus *Triticum aestivum* der Sorte Taifun sind in den Sequenzen der SEQ ID NOs: 1-3 gezeigt. SEQ ID NO: 1 stammt aus dem Subgenom A, SEQ ID NO: 2 stammt aus dem Subgenom B, und SEQ ID NO: 3 stammt aus dem Subgenom D. SEQ ID NOs: 4, 5 und 6 zeigen die entsprechenden Promotorsequenzen. In SEQ ID NOs: 7, 8 und 9 ist die cDNA-Sequenz der TDF-Gene aus den WeizenSubgenomen A, B bzw. D gezeigt. Die Aminosäuresequenz des codierten TDF-Proteins ist in SEQ ID NO: 10 (Subgenom A), SEQ ID NO: 11 (Subgenom B) und SEQ ID NO: 12 (Subgenom D) wiedergegeben.

"Inaktiviertes TDF-Gen" oder "inaktiviertes TDF-Allel" bedeutet, dass die Funktion des TDF-Gens bzw. -Allels und/oder des TDF-Proteins inaktiviert ist (d.h. ausgeschaltet oder nicht mehr vorhanden) oder signifikant reduziert ist. Vorzugsweise ist die Funktion des TDF-Gens bzw. TDF-Allels und/oder des TDF-Proteins um mindestens 80%, oder mindestens 85% im Vergleich zum aktiven Gen/Allel/Protein reduziert. Besonders bevorzugt ist eine Reduzierung der Funktion um mindestens 90%, mindestens 95%, mindestens 98%, mindestens 99% oder 100% im Vergleich zum aktiven Gen/Allel/Protein. Die Funktion des TDF-Gens bzw. -Allels ist zum Beispiel signifikant reduziert, wenn die Expression des Gens nur 20% oder weniger, 15% oder weniger, 10% oder weniger, oder 5% oder weniger als die Expression eines aktiven Gens/Alles beträgt. Die Funktion des TDF-Proteins ist zum Beispiel signifikant reduziert, wenn das Proteins nur 20% oder weniger, 15% oder weniger, 10% oder weniger, oder 5% oder weniger der biologischen Aktivität eines aktiven Gens/Allels aufweist.

Ferner betrifft die vorliegende Erfindung eine Pflanze der Gattung *Triticum,* in der ein Markergen in mindestens ein Allel des TDF-Gens eines Subgenoms der Pflanze insertiert (integriert) ist und dadurch das Allel inaktiviert, und die Expression des Markergens zu einem unterschiedlichen Phänotyp führt, abhängig davon, ob das Markergen homozygot oder hemizygot vorliegt.

Markergene im Sinne der Erfindung sind Gene, die eine phänotypisch unterscheidbare Eigenschaft der Pflanze bedingen, das heißt, deren Expression einen bestimmten Phänotyp zur Folge hat, der unterschiedlich ausgeprägt ist, je nachdem, ob das Markergen homozygot oder hemizygot vorliegt.

Als Markergen kann ein Herbizidtoleranz-Gen, ein Farbmarker-Gen oder ein Gen, welches eine Unterscheidung anhand der Pflanzenmorphologie (z.B. Pflanzenhöhe) zulässt, verwendet werden. Beispiele für geeignete Herbizidtoleranz-Gene sind: P450, oder AHAS mit einer Herbizidtoleranz-vermittelnden Mutation (Jimenez et al., 2016). Beispiele für geeignete Farmarker-Gene sind: blue-aleuron (bla; Abdel-Aal & Hucl, 1999) oder Ba1 (Burešová et al., 2015) und für morphologische Marker rht--B1a bzw. rht-B1 b, wobei erstere allelische Variante eine große Pflanzenhöhe und zweitere allelische Variante eine kleine Pflanzenhöhe bewirkt; ebenso sind rht-D1 a bzw. rht-D1b einsetzbar (Ellis et al, 2002).

In bevorzugten Ausführungsformen der Erfindung ist das Markergen ein Herbizidtoleranz-Gen Ein besonders bevorzugtes Markergen ist das Herbizidtoleranz-Gen P450 aus *Cynodon dactylon.* In einer bevorzugten Ausführungsform umfasst das P450-Gen eine Sequenz ausgewählt aus folgenden Sequenzen: Nukleinsäuresequenz der SEQ ID NO: 26; codonoptimierte Nukleinsäuresequenz der SEQ ID NO: 27; Nukleinsäuresequenz kodierend für eine Aminosäuresequenz gemäß SEQ ID NO: 28 oder einen funktionellen Teil davon oder ein Homolog davon, oder einer Nukleotidsequenz, die unter stringenten Bedingungen mit einer Sequenz der SEQ ID NO: 26 oder 27 oder einer komplementären Sequenz dazu hybridisiert.

"Homozygot" bedeutet, dass beide Allele desselben Subgenoms das Markergen aufweisen; "hemizygot" bedeutet, dass nur ein Allel des Subgenoms das Markergen aufweist; "azygot" bedeutet, dass das Genom der Pflanze kein Markergen enthält.

Die Pflanze gemäß der Erfindung kann weitere Mutationen im TDF-Gen aufweisen. In einer bevorzugten Ausführungsform sind die Allele der beiden "übrigen Subgenome" (das heißt, derjenigen Subgenome, in denen nicht bereits mindestens ein Allel durch Insertion/Integration des Markergens inaktiviert ist/sind), inaktiviert. Ist also beispielsweise das Markergen in ein oder beide TDF-Allele des Subgenoms A insertiert, weisen die TDF-Allele /-Gene der beiden anderen ("übrigen") Subgenome B und D ebenfalls Mutationen auf, die zu ihrer Inaktivierung führen.

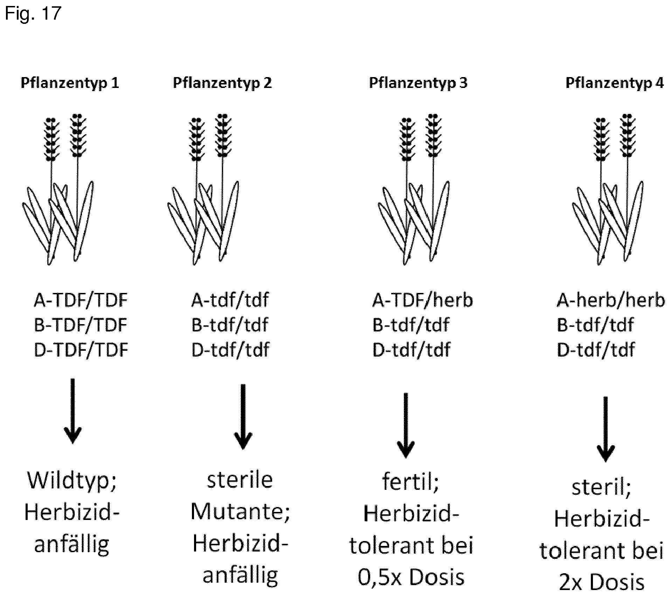

Eine derartige Pflanze kann beispielsweise hergestellt werden, indem zunächst ein Markergen in mindestens ein Allel des TDF-Gens eines Subgenoms (z.B. Subgenom A) einer Pflanze insertiert/integriert wird, wodurch dieses Allel inaktiviert wird. Anschließend wird die resultierende Pflanze mit einer Pflanze gekreuzt, in der die TDF-Allele in den beiden anderen Subgenomen (B und D), zum Beispiel durch kleinere Deletion(en), mutiert sind und das TDF-Gen dadurch inaktiviert ist. Das Ergebnis der Kreuzung sind zum einen Pflanzen, in denen das insertierte Markergen hemizygot vorliegt. Dann ist nur ein Allel beispielsweise des Subgenoms A inaktiviert, das andere Allel liegt als TDF-Wildtypallel vor (z.B. Pflanzentyp 3 gemäß Fig. 17). Zum anderen sind es Pflanzen, in denen das Markergen homozygot vorliegt. Dann sind beide Allele beispielsweise des Subgenoms A inaktiviert (z.B. Pflanzentyp 4 gemäß Fig. 17). Die Inaktivierung sämtlicher Allele des für das TDF-Protein codierenden Gens führt dazu, dass die Pflanze männlich steril ist. "Sämtliche Allele" bedeutet, dass alle sechs Allele des TDF-Gens in allen drei Subgenomen des Weizens (A, B, D) inaktiviert sind (z.B. Pflanzentyp 2 gemäß Fig. 17).

Der Vorteil der erfindungsgemäßen Pflanze liegt darin, dass die Insertion/Integration eines Markergens in das für die männliche Fertilität verantwortliche Gen des Weizengenoms eine Differenzierung männlich steriler und nicht-steriler (fertiler) Pflanzen anhand eines bestimmten Phänotyps erlaubt, der auf die Expression des Markergens zurückgeht. Der von dem Markergen vermittelte Phänotyp lässt also einen direkten Rückschluss auf die Sterilität bzw. Fertilität der Pflanze zu.

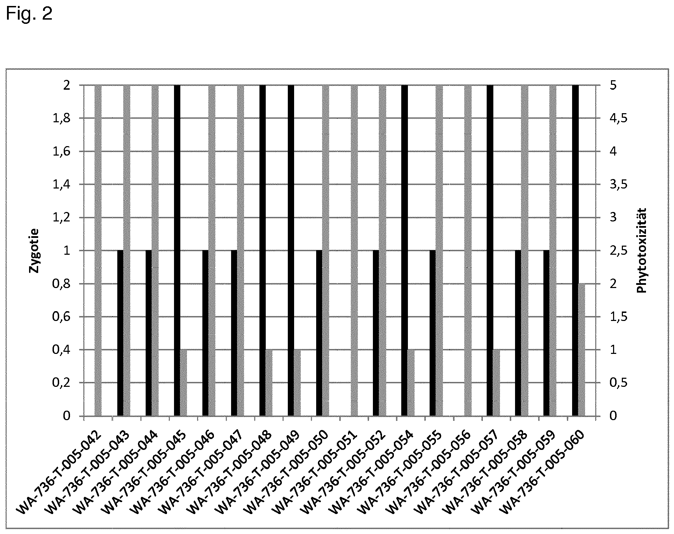

Wird beispielsweise ein Herbizidtoleranz-Gen als Markergen verwendet, kann die Selektion durch ein geeignetes Herbizid erfolgen. Pflanzen, bei denen das Markergen homozygot vorliegt, sind gegenüber hohen Dosen (z.B. der 2-fachen der vom Hersteller empfohlenen Aufwandsmenge) des Herbizids tolerant; sie sind ferner steril (Pflanzentyp 4 gemäß Fig. 17). Pflanzen dagegen, in denen das Markergen hemizygot vorliegt, sterben bei der Anwendung hoher Herbizidkonzentrationen; diese Pflanzen sind fertil (siehe Beispiel 1 und Figur 2; Pflanzentyp 3 gemäß Fig. 17).

Interessanterweise haben die Erfinder festgestellt, dass Pflanzen, in denen das Herbizidtoleranz-Gen hemizygot vorliegt, gegenüber geringen Dosen (z.B. der 0,5-fachen der vom Hersteller empfohlenen Menge) des Herbizids ebenfalls tolerant sind. Das Markergen wirkt also semidominant: der Phänotyp der Herbizidtoleranz ist im hemizygoten Zustand weniger stark ausgeprägt als im homozygoten (siehe Figur 17).

Alternativ zu der Integration eines Gens als Marker in den TDF-Locus könnten auch natürlich vorkommende (endogene) Gene als Marker dienen. Voraussetzung ist, dass solche potentiellen natürlichen Marker genetisch eng gekoppelt sind an das TDF-Gen. Das heißt, dass es sich um Gene handelt, die in direkter genomischer Umgebung des TDF-Gens vorkommen. Die Expression dieser endogenen Markergene muss einen phänotypischen Effekt auf die Pflanze haben. Gegebenenfalls können derartige endogenen Markergene genetisch modifiziert werden.

Die Begriffe "männliche Sterilität", "männlich steriler Phänotyp", "männlich steril" oder vergleichbare Termini bezeichnen die Eigenschaft einer Pflanze, nur noch weniger als 20%, vorzugsweise weniger als 15% oder 10%, besonders bevorzugt weniger als 5% und ganz besonders bevorzugt keine (0%) fertilen Nachkommen durch Kreuzung mit einer fertilen Pflanze derselben Gattung im Vergleich mit einer normal fertilen Pflanze zu erzeugen, wobei die männlich sterile Pflanze in der Kreuzung als Vater eingesetzt wird und die normal fertile Pflanze, welche als Mutter in der Kreuzung eingesetzt wird, eine Pflanze desselben Genotyps wie die männlich sterile Pflanze ist, welche jedoch die genetische Modifikation wie beispielsweise Geninaktivierung oder Mutation, die die männliche Sterilität verursacht, nicht aufweist.

Die männliche Sterilität lässt sich durch bekannte Verfahren überprüfen, beispielsweise durch den Anbau der Pflanze und morphologische Untersuchung der gebildeten Antheren auf makro- und mikroskopischer Ebene. Weiterhin können auch bekannte molekulare Analyseverfahren herangezogen werden, mit deren Hilfe für den Fall, dass die Inaktivierung aufgrund einer Mutation auf DNA-Ebene erfolgt, die molekulare Struktur dieser Mutation festgestellt werden kann. Führt die Mutation, beispielsweise eine Deletion oder Insertion von Nukleotiden, zu einer Leserasterverschiebung, kann der Fachmann Vorhersagen darüber treffen, ob eine männliche Sterilität in der Pflanze vorliegen wird. Im Fall einer Inaktivierung durch Inhibition des TDF-Gens/-Allels oder der TDF-Gene/-Allele über ein RNAi-vermitteltes Gene-Silencing oder einer Mutation/Deletion/Insertion im Genpromotor (zum Beispiel im Bereich des Minimalpromoters oder Transkriptionsstarts), sollte dies zu einer reduzierten Transkriptmenge führen. Hierbei sollten die Transkriptuntersuchungen dann vorzugsweise im von der Pflanze gebildeten Antherengewebe erfolgen, um verlässliche Vorhersagen über das Auftreten der männlichen Sterilität zu machen. Desweiteren kann der Fachmann auch bei der Einfügung von Deletionen oder Insertionen in die Nukleotidsequenz des TDF Gens, die nicht zur Leserasterverschiebung führen aber zu einer Deletion und/oder Insertion von Aminosäuren in das TDF-Protein eine Vorhersage treffen, ob dies zu einer Inaktivierung des funktionalen Proteins und damit zur Ausbildung einer männlichen Sterilität führt. Eine derartige Vorhersage kann z.B. durch eine Proteinstrukturvorhersage mittels geeigneter Software getroffen werden.

Ein "aktives Gen" bzw. "aktives Allel" kann ein TDF-Wildtyp-Gen/-Allel meinen, wie es zum Beispiel in einer männlich fertilen Pflanze vorliegt. Vorzugsweise umfasst das TDF-Gen der erfindungsgemäßen Pflanze im nicht-mutierten (Wildtyp) Zustand ein Nukleinsäuremolekül umfassend eine Nukleotidsequenz gemäß der SEQ ID NO: 1, 2 oder 3 oder eine Nukleotidsequenz, die zur SEQ ID NO: 1, 2, oder 3 homolog ist. Die cDNA des TDF-Wildtyp-Gens/-Allels entspricht vorzugsweise einem Nukleinsäuremolekül umfassend eine Nukleotidsequenz gemäß der SEQ ID NO: 7, 8 oder 9 oder eine Nukleotidsequenz, die zur SEQ ID NO: 7, 8 oder 9 homolog ist. Das "aktive Protein" kann ein TDF-Wildtyp-Protein sein. Das TDF-Wildtyp-Protein umfasst vorzugsweise die Aminosäuresequenz der SEQ ID NO: 10, 11 oder 12 oder eine Aminosäuresequenz, die zu mindestens 80%, bevorzugt zu mindestens 85% oder mindestens 90%, besonders bevorzugt zu mindestens 95%, mindestens 96%, mindestens 97%, mindestens 98% oder mindestens 99% identisch ist zu einer der SEQ ID NOs: 10, 11 oder 12.

Die Inaktivierung eines TDF-Gens bzw. eines TDF-Allels (zum Beispiel in den beiden übrigen Subgenomen) kann beispielsweise durch die Veränderung von codierenden oder regulatorischen (z.B. Promotor) TDF-Wildtyp-Sequenzen erfolgen, wobei die Veränderung die Funktion (Expression) des TDF-Gens verhindert oder signifikant reduziert. Es können auch gezielt eine oder mehrere Mutationen in die codierenden TDF-Sequenzen (Exons) oder die Spleißsignale eingefügt werden, die das Entstehen eines funktionellen TDF-Proteins verhindern, d.h. dass die Translation einer auf diese Art mutierten TDF-Sequenz zur Synthese eines nicht-funktionellen TDF-Proteins oder eines TDF-Proteins mit einer signifikant reduzierten Funktion führt.

Eine geeignete Mutation in einer codierenden Sequenz des TDF-Gens oder einem Spleißsignal umfasst zum Beispiel die Addition oder Deletion von einer oder mehreren Teilnukleotidsequenzen mit einer Länge von mindestens einem Nukleotid der genomischen TDF-Sequenz, vorausgesetzt diese Addition oder Deletion führt dazu, dass keine Funktion oder nur eine signifikant reduzierte Funktion des synthetisierten TDF-Proteins gegeben ist, d.h. dass eine Inaktivierung des TDF-Gens/-Allels bzw. TDF-Proteins vorliegt. Eine solche Addition oder Deletion ist dann eine besonders geeignete Mutation, wenn die Addition zur Insertion von mindestens einem Nukleotid in einem Exon führt und/oder zum Zerstören eines Spleißsignals führt, oder die Deletion zum Verlust eines Teils eines Exons und/oder zum Verlust eines Spleißsignals führt. Im Falle einer Addition mit Insertion in einem Exon oder einer Deletion mit Verlust eines Teils des Exons, ist vorzugsweise die Anzahl der addierten bzw. deletierten Nukleotide nicht durch drei teilbar, so dass die Addition bzw. Deletion zu einer Leserasterverschiebung führt. Eine Addition oder Deletion kann weiterhin im Zuge der Translation auch die Synthese zusätzlicher Aminosäuren bzw. weniger Aminosäuren zur Folge haben, was die Funktion des TDF-Proteins stören oder vollends zerstören kann, indem es Auswirkungen auf die sekundäre und tertiäre Proteinstruktur hat. Geeignete Mittel zur Mutation sind neben den bekannten Mutagenese-Techniken insbesondere die Technologien des "Genome Editing", wie beispielsweise Meganukleasen, TALENs, Zinkfingernukleasen oder ein CRISPR/Cas-System, welche Doppelstrangbrüche in der DNA induzieren können und dann das Hinzufügen oder den Verlust von Nukleotiden beispielsweise während eines Rekombinationsereignisses begünstigen.

Ferner können auch eine oder mehrere Punktmutationen in die codierende Sequenz eingefügt werden, zum Beispiel zum Einbringen von Stopp-Codons oder zur Substitution von Nukleotiden zwecks Veränderung der Aminosäuresequenz des codierten TDF-Proteins, beispielsweise durch Strahlung oder chemische Mutagenzien oder durch gerichtete Mutagenese, beispielsweise mittels TILLING. Teilweise können Punktmutationen auch durch Gene-Repair-OligoNucleotide (GRON)-Technik oder auch CRISPR/Cas erfolgen. Weiterhin können Insertionen, die zur Inaktivierung führen, auch durch Transposon-Mutagenese eingebracht werden. Die oben genannten Techniken des Genome Editings wie auch die Mutagenisierungs-Technologien sind auch geeignet, vorhandene Spleißsignale zu zerstören oder neue einzufügen.

Die Inaktivierung des TDF-Gens bzw. TDF-Allels kann durch eine oder mehrere der oben und auch weiter unten beschriebenen Techniken herbeigeführt werden; dem Fachmann sind jedoch noch weitere Verfahren zur Inaktivierung bekannt, welche erfindungsgemäß eingesetzt werden können. Somit sollen die genannten Techniken der Inaktivierung nicht als abschließend oder den Umfang der Erfindung beschränkend verstanden werden.

Die Entstehung von funktionellem TDF-Protein kann ferner posttranskriptionell verhindert oder signifikant reduziert werden, z.B. durch RNA-Inhibierung. Ein weiterer Weg zur Inaktivierung des TDF-Gens bzw. TDF-Allels ist, inaktive Genversionen in natürlichen Quellen mittels Eco-TILLING zu identifizieren und diese dann durch sexuelle Kreuzung, Protoplastenfusion oder ähnliche Verfahren und gleichzeitige Marker-gestützte Selektion in das Kerngenom von anderen Linien, vorzugweise Elite-Linien, zu überführen.

Das TDF-Gen der Pflanze der Gattung *Triticum* gemäß der Erfindung kann eine oder mehrere Mutation(en) enthalten. Vorzugsweise enthalten die TDF-Allele der übrigen Subgenome der Pflanze gemäß der Erfindung eine oder mehrere Mutation(en), die entweder die Expression des TDF-Gens reduziert (reduzieren) oder verhindert (verhindern), oder zur Synthese eines nichtfunktionellen TDF-Proteins oder eines TDF-Proteins mit reduzierter Funktionalität/Aktivität führt (führen).

In einer bevorzugten Ausführungsform liegen die Mutation(en) der Allele der anderen Subgenome in einem oder mehreren Exon(s), besonders bevorzugt in demselben Exon des TDF-Gens bzw. der TDF-Allele der Subgenome, vorzugsweise in Exon 2.

In einer ganz besonders bevorzugten Ausführungsform liegen die Insertion (Integration) des Markergens und/oder die Mutation(en) der Allele der anderen Subgenome im Exon 2 des TDF_Gens, bevorzugt in einer Region korrespondierend zu einem Sequenzabschnitt zwischen den Nukleotidpositionen 256 und 286 der SEQ ID NO: 3, beispielsweise (a) zwischen den Nukleotidpositionen 256 und 285 der SEQ ID NO: 3, (b) zwischen den Nukleotidpositionen 258 und 285 der SEQ ID NO: 3, (c) zwischen den Nukleotidpositionen 281 und 286 der SEQ ID NO: 3, oder (d) zwischen den Nukleotidpositionen 281 und 283 der SEQ ID NO: 3; oder (e) an der Nukleotidposition 282 der SEQ ID NO: 3. Besonders bevorzugt entsprechen die Mutationen mindestens einer der Mutationen in einer der Nukleotidsequenzen der SEQ ID NOs: 19 bis 25, welche mit Referenz zu SEQ ID NO: 3 wiedergegeben sind. Vorstehende Angaben zu Nukleotidpositionen bzw. Mutationen beziehen sich auf SEQ ID NO: 3 aus dem Subgenom D (die "Referenzsequenz"). In den TDF-Genen/-Allelen der anderen Subgenome können die Nukleotidpositionen aufgrund der Divergenz zwischen den Allelen unterschiedlich sein. Die für die Insertion/Integration des Markergens und Mutation(en) gemäß der Erfindung bevorzugten Regionen im TDF-Gen/-Allel sind nicht beschränkt auf die für die Referenzsequenz angegebenen Nukleotidpositionen, vielmehr können die Mutation(en) auch an den entsprechenden Positionen der Nukleotidsequenz anderer Allele liegen.

In einer bevorzugten Ausführungsform handelt es sich bei der(den) Gen-Mutation(en) der anderen Subgenome um Deletion(en), vorzugsweise um Deletion(en) von 1 bis 28 Nukleotiden. Besonders bevorzugt treten diese Deletion(en) im Exon 2 in der Region korrespondierend einem Sequenzabschnitt zwischen den Nukleotidpositionen 256 und 286 der SEQ ID NO: 3 (Referenzsequenz) auf.

Die Insertion (Integration) des Markergens oder die TDF-Gen Mutation(en) der anderen Subgenome in der Pflanze gemäß der Erfindung, die zur Inaktivierung des TDF-Gens führt (führen), kann (können) transgen oder nicht-transgen erzeugt werden.

Ferner betrifft die vorliegende Erfindung Teile und Samen einer Pflanze gemäß der Erfindung, in der sämtliche Allele des für TDF codierenden Gens inaktiviert sind.

Ein "Teil" einer Pflanze meint im Sinne der vorliegenden Erfindung ein pflanzliches Organ wie beispielsweise Blatt, Sprossachse, Stamm, Wurzel, vegetative Knospe, Meristem, Embryo, Anthere, Ovula oder Frucht; oder bezeichnet einen Zusammenschluss mehrerer Organe, z.B. eine Blüte oder ein Samen, oder einen Teil eines Organs, z.B. einen Querschnitt aus der Sprossachse; oder bezeichnet ein pflanzliches Gewebe wie Kallusgewebe, Speichergewebe, meristematisches Gewebe, Blattgewebe, Sprossgewebe, Wurzelgewebe, Pflanzentumorgewebe oder reproduktives Gewebe.

Unter einer "Pflanzenzelle" ist im Sinne der vorliegenden Erfindung beispielsweise eine isolierte Zelle mit einer Zellwand oder Aggregate davon oder Protoplasten zu verstehen.

Die vorliegende Erfindung betrifft auch Nachkommen der erfindungsgemäßen Pflanze der Gattung *Triticum* und Teile oder Samen davon, wobei ein Markergen in mindestens ein Allel des TDF-Gens eines Subgenoms der Pflanze bzw. Teils oder Samens insertiert ist und das Allel inaktiviert ist. Die Nachkommen der Pflanzen sind beispielsweise herstellbar durch Kreuzung einer erfindungsgemäßen Pflanze der Gattung *Triticum* (z.B. Pflanzentyp 4 gemäß Fig. 17) mit einer fertilen Pflanze derselben Gattung (z.B. Pflanzentyp 1 gemäß Fig. 17), wobei die erfindungsgemäße Pflanze in der Kreuzung als Mutter (maternale Komponente) eingesetzt wird. Die Nachkommenpflanzen oder ein Teil oder Samen davon können eine Hybridpflanze der Gattung *Triticum* bzw. ein Teil oder Samen davon sein. Vorzugsweise meint "Hybridpflanze" eine Pflanze der F1 Generation einer Kreuzung aus zwei nicht identischen Elternlinien.

Ferner betrifft die vorliegende Erfindung ein Verfahren zum Nachweis einer männlichen Sterilität in einer Pflanze der Gattung *Triticum* oder in Samen davon, umfassend die folgenden Schritte: (i) Herstellen einer (transgenen) Pflanze der Gattung *Triticum*, dadurch gekennzeichnet, dass ein Markergen in mindestens ein Allel des *tapetal development and function* (TDF) Gens eines Subgenoms der Pflanze insertiert ist und das Allel inaktiviert ist, und die Allele des TDF-Gens der übrigen beiden Subgenome ebenfalls inaktiviert sind (z.B. Pflanzentyp 3 oder 4 gemäß Fig. 17), wobei das Markergen ein Herbizidtoleranz-Gen ist, (ii) Behandeln der Pflanze oder ihrer Samen mit einem geeigneten Herbizid in einer Konzentration, die solche (transgenen) Pflanzen tötet, in denen das Herbizidtoleranz-Gen in nur einem Allel des einen Subgenoms vorliegt, und (iii) Identifizieren der Pflanzen, welche die Behandlung mit dem Herbizid überlebt haben, als männlich steril.

Ebenfalls von der Erfindung umfasst ist ein Verfahren zur Selektion einer männlich sterilen Pflanze der Gattung *Triticum*, umfassend die folgenden Schritte: (i) Herstellen einer Pflanze der Gattung *Triticum*, dadurch gekennzeichnet, dass ein Markergen in mindestens ein Allel des *tapetal development and function* (TDF) Gens eines Subgenoms der Pflanze inseriert ist und das Allel inaktiviert ist, wobei die Allele des TDF-Gens der übrigen beiden Subgenome ebenfalls inaktiviert sind und das Markergen ein Herbizidtoleranz-Gen ist, (ii) Behandeln der Pflanze oder ihrer Samen mit einem geeigneten Herbizid in einer Konzentration, die solche Pflanzen abtötet, in denen das Herbizidtoleranz-Gen in nur einem Allel des einen Subgenoms vorliegt, und (iii) Identifizieren der Pflanzen, welche die Behandlung mit dem Herbizid überlebt haben.

Die Erfindung betrifft weiterhin ein Verfahren zum Herstellen einer Pflanze der Gattung *Triticum*, bei dem ein Markergen in mindestens ein Allel des *tapetal development and function* (TDF) Gens eines Subgenoms der Pflanze insertiert wird und das Allel dadurch inaktiviert wird. Geeignete Verfahren, insbesondere zur gezielten Insertion/ Integration, sind dem Fachmann aus dem Stand der Technik hinreichend bekannt und werden ferner hierin beschrieben.

Beschrieben wird auch ein Nukleinsäuremolekül umfassend eine Nukleotidsequenz ausgewählt aus den folgenden: (a) einer Nukleotidsequenz der SEQ ID NO: 1, 2 oder 3 oder SEQ ID NO: 7, 8 oder 9; (b) einer Nukleotidsequenz, die für ein Protein enthaltend eine der Aminosäuresequenzen der SEQ ID NO: 10, 11 oder 12 oder eine Aminosäuresequenz, die eine Identität von mindestens 80 %, vorzugsweise mindestens 85%, mindestens 90% oder mindestens 95%, besonders bevorzugt mindestens 96%, mindestens 96%, mindestens 97%, mindestens 98% oder mindestens 99% zu einer der Aminosäuresequenzen der SEQ ID NO: 10, 11 oder 12 aufweist, codiert; (c) einer Nukleotidsequenz, die komplementär ist zu (a) oder (b); (d) einer Nukleotidsequenz, die homolog ist zu (a) oder (b) oder eine Identität von mindestens 70 % zu (a) oder (b) aufweist, wobei die Nukleotidsequenz für ein TDF-Protein kodiert; (e) einer Nukleotidsequenz, die unter stringenten Bedingungen mit (a) oder (b) hybridisiert; und (f) einer Nukleotidsequenz, die unter stringenten Bedingungen mit (c) hybridisiert, wobei die Nukleotidsequenz für ein TDF-Protein kodiert. Ein Nukleinsäuremolekül kann auch eine Teilsequenz (Fragment) der in (a) bis (f) genannten Nukleotidsequenzen umfassen. Das Fragment hat eine Länge von mindestens 17, 18, 19, 20, 25, 30, 50, 100, 200, 300, 400 oder 500 aufeinanderfolgenden Nukleotiden. Die Teilnukleotidsequenz liegt vorzugsweise in sense Orientierung, anti-sense (invers komplementäre) Orientierung oder komplementärer Orientierung zu derjenigen Nukleotidsequenz vor, aus welcher die Teilsequenz / das Fragment stammt. Fragmente können als Primer, Sonden, oder inhibitorische Sequenzen (zum Beispiel für einen RNAi-Ansatz) verwendet werden.

Unter "homologen Sequenzen" oder "Homologe" oder vergleichbaren Termini werden Nukleinsäuremoleküle verstanden, welche den gleichen phylogenetischen Ursprung haben. Vorzugsweise üben Proteine, die durch diese Nukleinsäuremoleküle codiert werden, die gleiche Funktion aus. Homologe Nukleinsäuremoleküle weisen mindestens 70%, vorzugsweise mindestens 75%, mindestens 80%, mindestens 85% oder mindestens 90%, besonders bevorzugt mindestens 95%, mindestens 96%, mindestens 97%, mindestens 98% oder mindestens 99% Sequenzidentität auf.

Unter "Hybridisieren" oder "Hybridisierung" wird ein Vorgang verstanden, bei dem sich ein einzelsträngiges Nukleinsäuremolekül an einen weitestgehend komplementären Nukleinsäurestrang anlagert, d.h. Basenpaarungen eingeht. Standardverfahren zur Hybridisierung sind beispielsweise in Sambrook et al. 2001 beschrieben. Vorzugsweise wird darunter verstanden, dass mindestens 60%, weiter bevorzugt mindestens 65%, 70%, 75%, 80% oder 85%, besonders bevorzugt 90%, 91 %, 92%, 93%, 94%, 95%, 96%, 97%, 98% oder 99% der Basen des Nukleinsäuremoleküls eine Basenpaarung mit dem weitestgehend komplementären Nukleinsäurestrang eingehen. Die Möglichkeit einer solchen Anlagerung hängt von der Stringenz der Hybridisierungsbedingungen ab. Der Begriff "Stringenz" bezieht sich auf die Hybridisierungsbedingungen. Hohe Stringenz ist dann gegeben, wenn eine Basenpaarung erschwert ist, niedrige Stringenz, wenn eine Basenpaarung erleichtert ist. Die Stringenz der Hybridisierungsbedingungen hängt beispielsweise von der Salzkonzentration bzw. Ionenstärke und der Temperatur ab. Generell kann die Stringenz durch eine Erhöhung der Temperatur und/oder eine Erniedrigung des Salzgehaltes erhöht werden. Unter "stringenten Hybridisierungsbedingungen" sind solche Bedingungen zu verstehen, bei denen eine Hybridisierung überwiegend nur zwischen homologen Nukleinsäuremolekülen stattfindet. Der Begriff "Hybridisierungsbedingungen" bezieht sich dabei nicht nur auf die bei der eigentlichen Anlagerung der Nukleinsäuren herrschenden Bedingungen, sondern auch auf die bei den anschließenden Waschschritten herrschenden Bedingungen. Stringente Hybridisierungsbedingungen sind beispielsweise Bedingungen, unter denen überwiegend nur solche Nukleinsäuremoleküle hybridisieren, die mindestens 70%, vorzugsweise mindestens 75%, mindestens 80%, mindestens 85% oder mindestens 90%, besonders bevorzugt mindestens 95%, mindestens 96%, mindestens 97%, mindestens 98% oder mindestens 99% Sequenzidentität aufweisen. Stringente Hybridisierungsbedingungen sind beispielsweise: Hybridisieren in 4 x SSC bei 65 °C und anschließendes mehrfaches Waschen in 0,1 x SSC bei 65 °C für insgesamt etwa 1 Stunde. Der hier verwendete Begriff "stringente Hybridisierungsbedingungen" kann auch bedeuten: Hybridisieren bei 68 °C in 0,25 M Natriumphosphat, pH 7,2, 7 % SDS, 1 mM EDTA und 1 % BSA für 16 Stunden und anschließendes zweimaliges Waschen mit 2 x SSC und 0,1 % SDS bei 68 °C. Bevorzugt findet eine Hybridisierung unter stringenten Bedingungen statt.

Die Nukleinsäuren können in einem Vektor enthalten sein. Bei dem Vektor kann es sich um ein Plasmid, ein Cosmid, einen Phagen oder einen Expressionsvektor, einen Transformationsvektor, Shuttle-Vektor oder Klonierungsvektor handeln. Der Vektor kann doppel- oder einzelsträngig, linear oder zirkulär sein und kann einen prokaryotischen oder eukaryotischen Wirt entweder durch Integration in dessen Genom oder extrachromosomal transformieren. Bevorzugt ist die Nukleinsäure in einem Vektor mit einer oder mehreren regulatorischen Sequenz(en), welche die Transkription und optional die Expression in einer prokaryotischen oder eukaryotischen Wirtszelle erlauben, operativ verknüpft. Eine regulatorische Sequenz, vorzugsweise DNA, kann homolog oder heterolog zu der Nukleinsäure sein. Beispielsweise steht die Nukleinsäure unter der Kontrolle eines geeigneten Promotors oder eines Terminators. Geeignete Promotoren können Promotoren sein, welche konstitutiv induziert werden (Bsp.: 35S-Promoter aus dem "Cauliflower mosaic virus" (Odell et al., 1985). Besonders geeignet sind solche Promotoren, welche gewebespezifisch sind (zum Beispiel pollenspezifische Promotoren (Chen et al., 2010; Zhao et al., 2006; oder Twell et al., 1991)), oder entwicklungsspezifisch sind (zum Beispiel blühspezifische Promotoren). Geeignete Promotoren können auch synthetische bzw. chimäre Promotoren sein, welche in der Natur nicht vorkommen, aus mehreren Elementen zusammengesetzt sind und einen Minimalpromotor beinhalten sowie stromaufwärts des Minimalpromotors mindestens ein cis-regulatorisches Element aufweisen, welches als Bindungsstelle für spezielle Transkriptionsfaktoren dient. Chimäre Promotoren können den gewünschten Spezifitäten nach konzipiert und durch unterschiedliche Faktoren induziert oder reprimiert werden. Beispiele für solche Promotoren finden sich in Gurr & Rushton, 2005 oder Venter, 2007. Ein geeigneter Terminator ist beispielsweise der nos-Terminator (Depicker et al., 1982).

Das beschriebene Nukleinsäuremolekül kann zum Nachweis verwendet werden, dass ein Allel des für TDF codierenden Gens inaktiviert ist, wobei das Nukleinsäuremolekül eine Teilsequenz (Fragment) einer Nukleotidsequenz umfasst, wobei die Nukleotidsequenz vorzugsweise ausgewählt ist aus (a) einer Nukleotidsequenz gemäß SEQ ID NO: 1, 2, 3, 4, 5, 6, 7, 8, oder 9; (b) einer Nukleotidsequenz, die komplementär ist zu einer Nukleotidsequenz gemäß SEQ ID NO: 1, 2, 3, 4, 5, 6, 7, 8, oder 9; oder (c) einer Nukleotidsequenz, die unter stringenten Bedingungen mit (a) oder (b) hybridisiert.

Die vorliegende Erfindung betrifft auch ein Verfahren zum Aufrechterhalten der männlichen Sterilität der erfindungsgemäßen Pflanze, wobei das Verfahren die folgenden Schritte umfasst: (i) Anbau von (transgenen) Pflanzen, in denen a) ein Markergen in ein Allel des *tapetal development and function* (TDF) Gens eines Subgenoms der Pflanze inseriert ist und das Allel inaktiviert ist und das andere Allel in dem Subgenom das aktive TDF-Allel ist, und b) die Allele des TDF-Gens der übrigen beiden Subgenome inaktiviert sind (z.B. Pflanzentyp 3 gemäß Fig. 17), wobei die Pflanzen sich selbst befruchten, ii) Selektieren derjenigen Pflanzen oder Samen aus der Nachkommengeneration, welche in denen a) ein Markergen in ein Allel des *tapetal development and function* (TDF) Gens eines Subgenoms der Pflanze inseriert ist und das Allel inaktiviert ist und das andere Allel in dem Subgenom das aktive TDF-Allel ist, und b) die Allele des TDF-Gens der übrigen beiden Subgenome inaktiviert sind (z.B. Pflanzentyp 3 gemäß Fig. 17). Die Selektion erfolgt bevorzugt auf Grundlage des Markergens (z.B. durch Herbizidbehandlung) und/oder auf Grundlage von molekularen Markern und/oder auf Grundlage der Bestimmung der Sterilität oder der Fertilität.

Ferner betrifft die Erfindung ein Verfahren zum Nachweis einer männlichen Sterilität in einer Pflanze der Gattung *Triticum* oder in einem Samen davon, umfassend die folgenden Schritte: (i) Isolieren von DNA aus der Pflanze, einem Teil davon oder dem Samen; (ii) Analysieren der DNA mit Hilfe eines Nukleinsäuremoleküls, welches eine Teilsequenz einer Nukleotidsequenz aufweist, ausgewählt aus (a) einer Nukleotidsequenz gemäß SEQ ID NO: 1, 2, 3, 4, 5, 6, 7, 8, oder 9, (b) einer Nukleotidsequenz, die komplementär ist zu einer Nukleotidsequenz gemäß SEQ ID NO: 1, 2, 3, 4, 5, 6, 7, 8, oder 9, oder (c) einer Nukleotidsequenz, die unter stringenten Bedingungen mit (a) oder (b) hybridisiert; und (iii) Identifizieren einer Pflanze, in welcher sämtliche Allele des für TDF codierenden Gens inaktiviert sind.

Die Erfindung wird durch die folgenden Figuren erläutert.
Figur 1: Transformationsvektor P6U-70 Subiintron-P450-wheat.
Figur 2: Abhängigkeit der Phytotoxizität, herbeigeführt durch Herbizidbehandlung, von dem in den Pflanzen vorliegenden Zygotiegrad. Ausgefüllt: Zygotie (0=azygot, 1=hemizygot, 2=homozygot); gestreift: Phytotoxizität (siehe auch Tabelle 3).
Figur 3: Nachweis der Menge des P450-Transkripts in Weizenpflanzen mittels qRT-PCR. Aufgetragen ist die durchschnittliche Transkriptmenge (+/- Standardabweichung) in hemizygoten und homozygoten Weizenpflanzen, angegeben als releative Expressionshöhe. Ein durchgeführter t-Test ergab, dass die Mittelwerte für hemizygote und homozygote Pflanzen signifikant (p < 0,05) unterschiedlich sind.

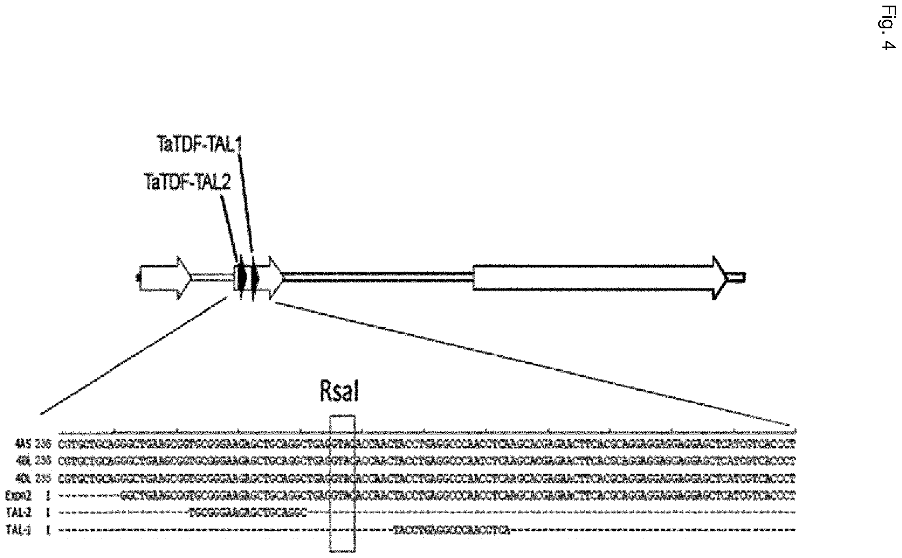

Figur 4: Oben: Vorhergesagte Genstruktur des TDF-Gens aus Weizen (TaTDF); unten: ein Ausschnitt des Gens aus Subgenom A ("4AS"; SEQ ID NO: 1, beginnend mit Nukleotidposition 236), Subgenom B ("4BL"; SEQ ID NO: 2, beginnend mit Nukleotidposition 236) und Subgenom D ("4DL"; SEQ ID NO: 3, beginnend mit Nukleotidposition 235) und Sequenz eines Teils des zweiten Exons ("Exon2") exemplarisch aus Subgenom A. Dargestellt sind außerdem die Bindungsstellen der beiden generierten TALEN-Arme ("TAL-2" bzw. "TaTDF-TAL2" und "TAL-1" bzw. "TaTDF-TAL1"), sowie die für die späteren Analysen verwendete Rsal-Schnittstelle.

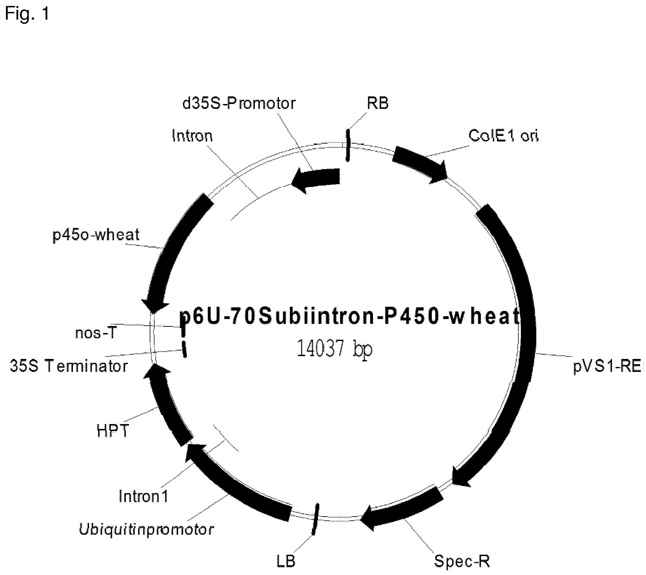

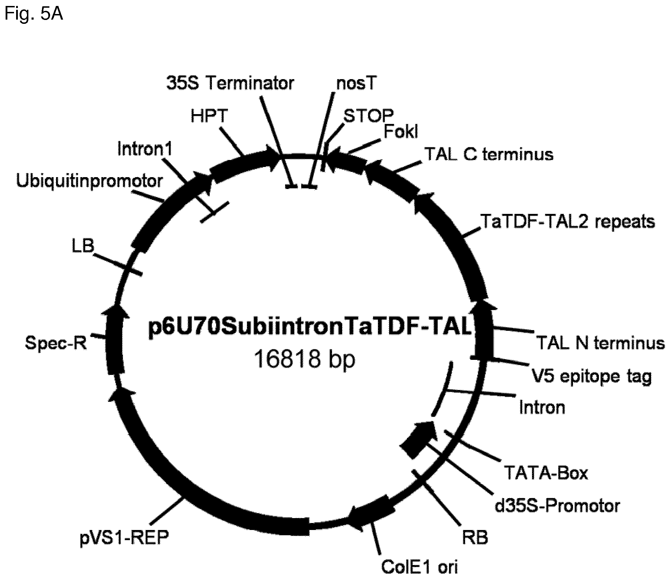

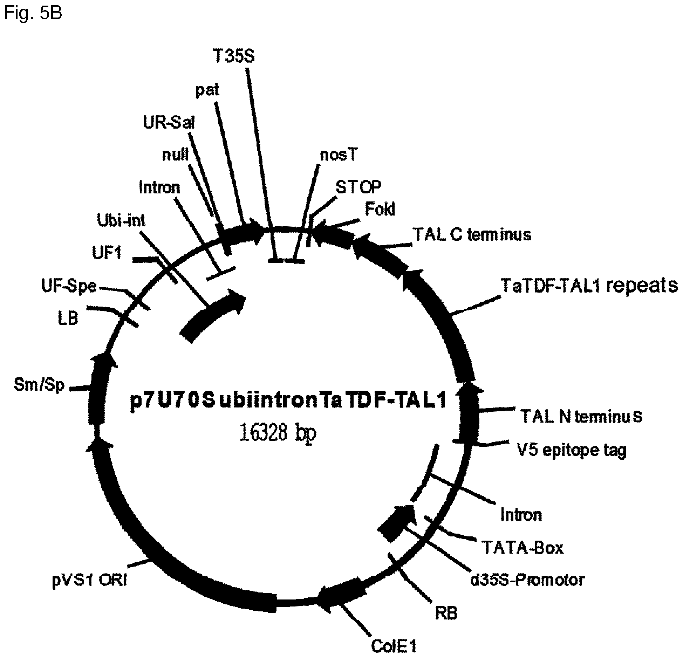

Figur 5A und 5B: Vektoren umfassend die TALEN-Konstrukte für die Transformation von Weizen zur gezielten Mutation der TaTDF Allele. Für die Transformation des TALEN-2 wurde das Konstrukt A, für die Transformation des TALEN-1 wurde das Konstrukt B verwendet. Vektorbestandteile: ColE1 ori: Replikationsursprung für die Amplifikation des Plasmids in E. coli; pVS1-REP: Replikationsursprung für die Amplifikation des Plasmids in *Agrobacterium tumefaciens* ;Spec-R: Spectinomycinresistenzgen zur bakteriellen Selektion; LB: Left border site der T-DNA; Ubiquitinpromotor: Promotor des Ubiquitin 1 Gens aus *Zea mays* inklusive des 1. Introns; Intron1: 1. Intron des Ubiquitin 1 Gens aus Zea mays; HPT: Hygromycinphosphotransferase Gen zur Selektion transgener Pflanzen auf Hygromycin; pat: Phosphinotricin-acetyltransferase Gen zur Selektion transgener Pflanzen auf Phosphinotricin; 35S Terminator: Terminationssequenz des 35S-Gens aus dem Blumenkohlmosaikvirus; nosT: Terminationssequenz des Nopalinsythasegens aus *Agrobacterium tumefaciens*; Fokl: Nukleasedomäne des Restriktionsenzyms Fokl aus *Flavobacterium okeanokoites;* TAL C terminus: C-Terminus des Transcription activation like effector (TALE) Proteins aus *Xanthomonas campestris*; TaTDF-TAL2 repeats: Aminosäuresequenz innerhalb des artifiziellen TALEN verantwortlich für die sequenzspezifische Bindung und die TAL-1 Sequenz (vergleiche Figur 1); TaTDF-TAL1 repeats: Aminosäuresequenz innerhalb des artifiziellen TALEN verantwortlich für die sequenzspezifische Bindung an die TAL-1 Sequenz (vergleiche Figur 1); TAL N terminus: N-Terminus des Transcription activation like effector (TALE) Proteins aus *Xanthomonas campestris*; d35S-Promotor: Promotor des 35S-Gens aus dem Blumenkohlmosaikvirus mit duplizierten Enhancerelement; RB: Right border site der T-DNA.

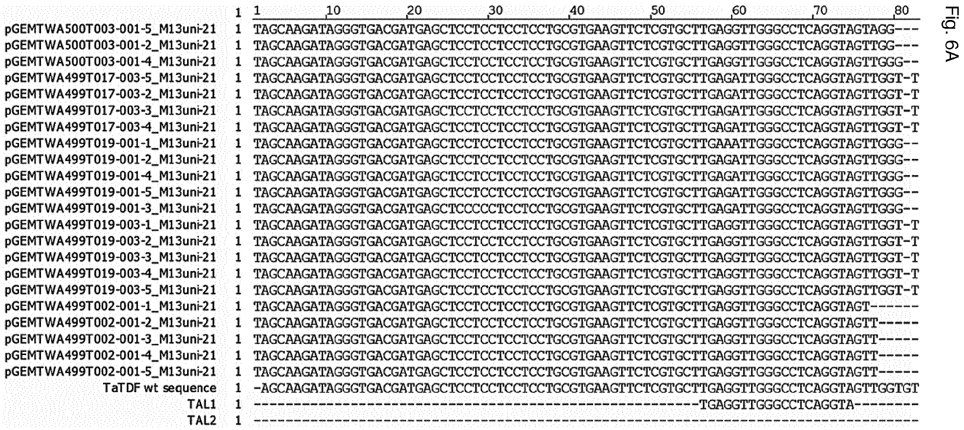

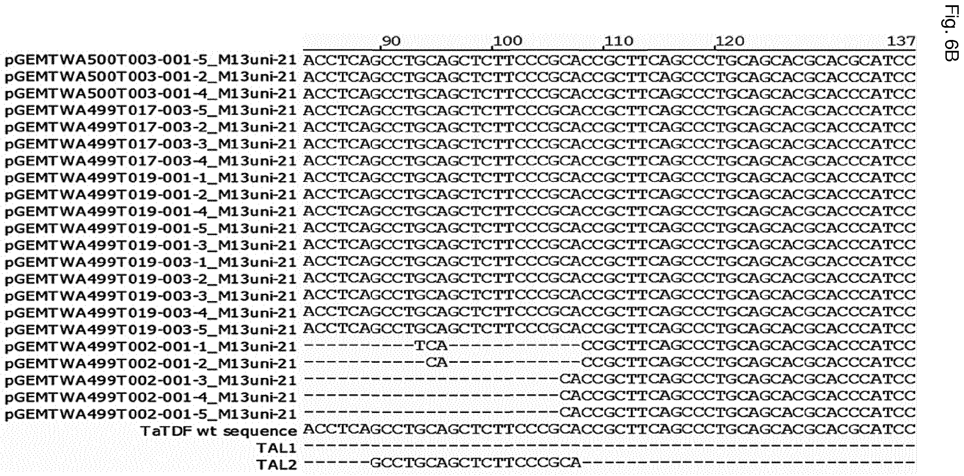

Figur 6A und 6B: Sequenzanalyse von amplifizierten, genomischen DNA-Fragmenten von transgenen Pflanzen der Transformationsexperimente WA499 und WA500 (Zeilen 1 bis 22; codogener Strang). Genomische DNA der Pflanzen wurde mit Rsal verdaut, eine PCR-Amplifikation des dargestellten Bereichs durchgeführt und die PCR-Fragmente wurden erneut mit Rsal verdaut. Unverdaute Fragmente wurden kloniert und sequenziert. Im Bereich der Bindungsstellen der beiden TALEN-Arme sind Deletionen zu erkennen, die zu einem Verlust der Rsal-Schnittstelle führen. Entsprechende Deletionen sind beispielhaft in Referenzsequenz SEQ ID NO: 3 übersetzt und in den Nukleotidsequenzen der SEQ ID NOs: 19-25 dargestellt.
Figur 7: Analyse von Einzelpflanzennachkommen der Linie WA500-T-003 mittels PCR Assay. Von allen Pflanzen wurde eine PCR-Amplifikation des TaTDF-Locus durchgeführt und das PCR Produkt anschließend mit Rsal verdaut. Unverdaute DNA zeigt die Präsenz von Mutationen an.

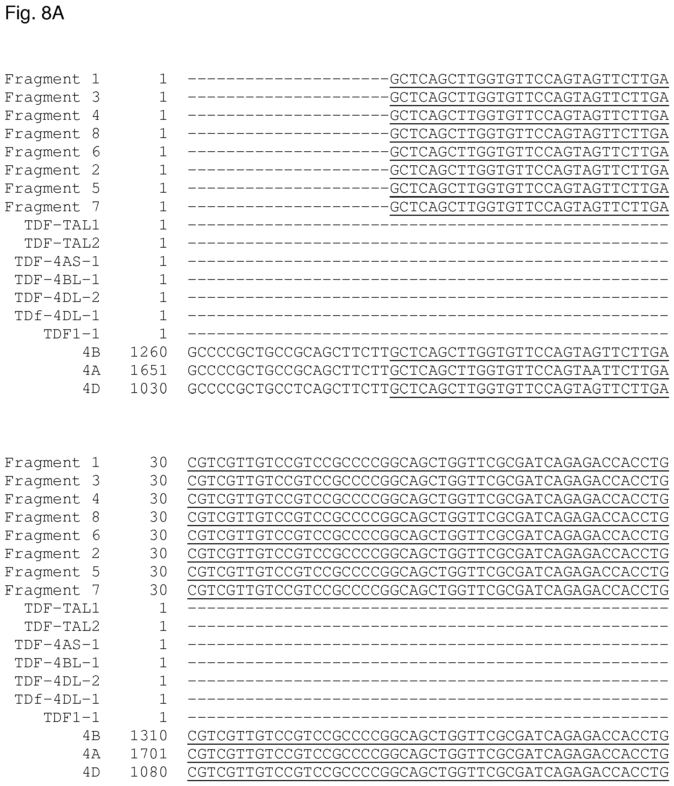

Figur 8A-J: Sequenzvergleich von amplifizierten und klonierten PCR-Fragmenten des Weizen TaTDF-Gens aus der Sorte Taifun. Sequenzübereinstimmungen sind durch Unterstreichung kenntlich gemacht; Bereiche, in denen sich die Sequenzen unterscheiden, weisen keine Unterstreichung auf. Die dargestellten Sequenzunterschiede lassen eine Zuordnung der jeweiligen Sequenz zu den einzelnen Weizensubgenomen zu. Dazu wurde die in den öffentlich verfügbaren Datenbanken vorhandenen Sequenzen aus dem A-, B- oder D-Subgenom des Weizens mit den amplifizierten Sequenzen verglichen (letzten drei Zeilen 4B (=Sugenom B), 4A (=Sugenom A) und 4D (=Sugenom D)). Zwischen den Sequenzen der Subgenome gibt es signifikante Unterschiede, die auch in den amplifizierten Sequenzen aus Taifun gefunden werden konnten. Wie aus dem Vergleich in Figur 5 zu sehen, sind die Fragmente 4 und 8 anhand der gefundenen Polymorphismen eindeutig als PCR Produkte aus dem Subgenom A anzusehen, wohingegen die übrigen Fragmente dem Subgenom D zuzuordnen sind. Aus dem Subgenom B wurde in dieser PCR somit kein Amplifikat erhalten. Die spezifischen Sequenzunterschiede zwischen den Subgenomen wurden dann für die Generierung von Subgenom-spezifischen Primern verwendet.

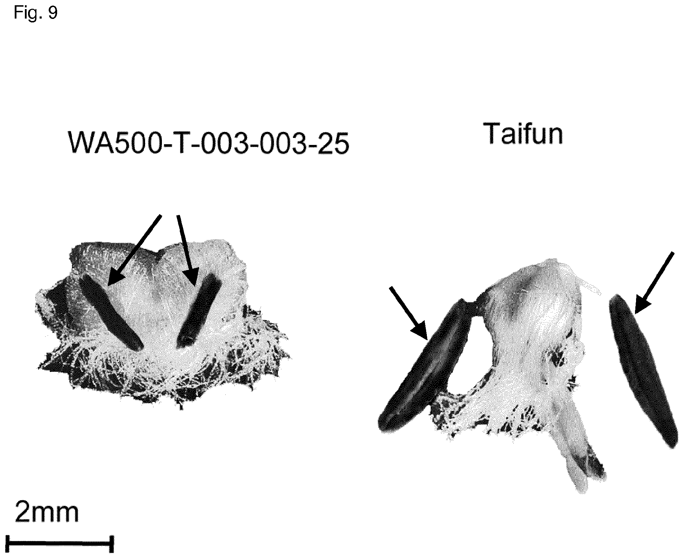

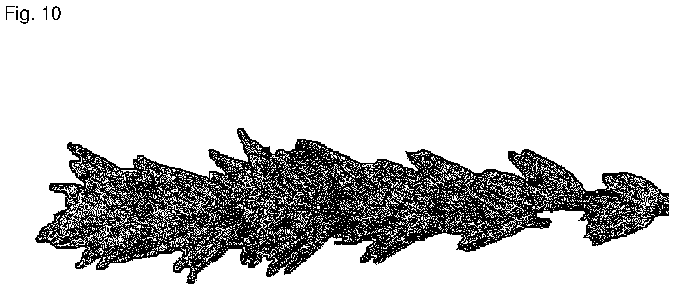

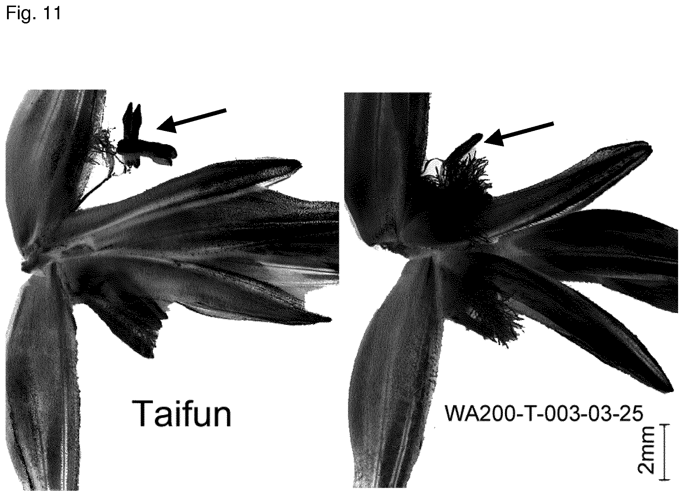

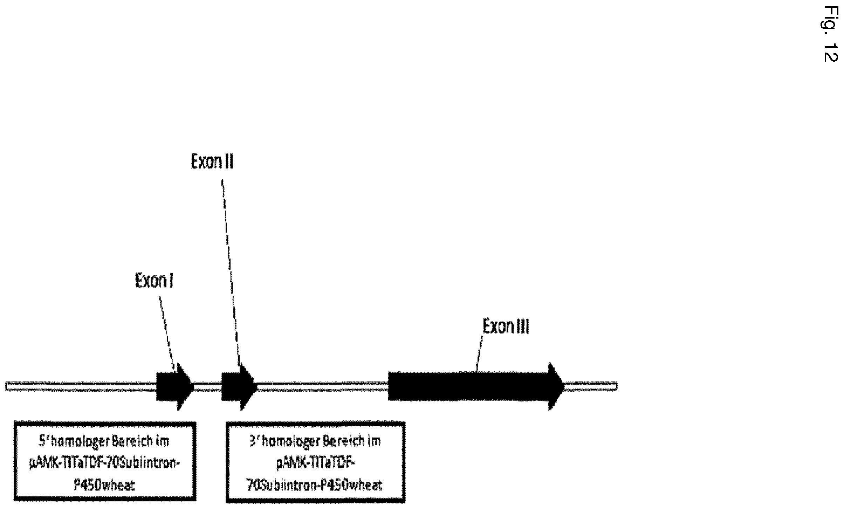

Figuren 9-11: Sterile Weizenpflanze WA500-T-003-03-25. Figur 9: isolierte Ovarien mit Antheren der sterilen Linie WA500-T-003-03-25 im Vergleich zur Wildtyppflanze Taifun; Figur 10: Ähre der Pflanze WA500-T-003-03-25. Es sind keine aus den Spelzen hervortretende Antheren zu sehen, die für das dargestellte Stadium typisch wären; Figur 11: Vergleich der Blüten der sterilen Weizenpflanze WA500-T-003-03-25 und der Wildtypflanze Taifun. (Pfeile zeigen Antheren). Figur 12: Genomische Organisation des TaTDF-Gens auf Weizen-Chromosom 4DL: Zielregion der gezielten Insertion mittels Gene-Editing Technologie.
Figur 13: Vektor pAMK-TITaTDF-70SubiintronP450wheat.
Figur 14: Fragment des Vektors pAMK-TITaTDF-70SubiintronP450wheat.
Figur 15: Vektor pDest_ubiCAS9_ChimeraTaU6-TaTDF.
Figur 16: Fragment des Vektors pDest_ubiCAS9_ChimeraTaU6-TaTDF.
Figur 17: Das Diagramm zeigt die verschiedenen Typen von genetischen Ausstattungen der Weizenpflanzen, welche zur Etablierung des erfindungsgemäßen Hybridsystems in Weizen notwendig sind. Pflanzentyp 1: unveränderter Wildtyp; eine solche Pflanze kann beispielsweise im Mischanbau als paternale Komponente (Pollenspender) eingesetzt werden; diese Pflanze ist männlich fertil, jedoch anfällig für Herbizidbehandlung. Pflanzentyp 2: alle TDF-Allele sind inaktiviert; diese Pflanze ist männlich steril und anfällig für Herbizidbehandlung; Pflanzentyp 3: ein TDF-Allel in einem Subgenom (z.B. Subgenom A) ist inaktiviert durch Insertion der P450-Gen-basierenden Herbizidtoleranz, das andere Allel in diesem Subgenom ist ein Wildtyp-Allel. Diese Pflanze ist männlich fertil und zeigt eine moderate Herbizidtoleranz (0,5-fache der vom Hersteller empfohlenen Menge des Herbizids Nicosulfuron); Pflanzen dieses Typs können zum Erhalt der maternalen Komponente des Hybridsystems eingesetzt werden. Pflanze 4: beide Allele des TDF-Gens in einem Subgenom (z.B. Subgenom A) sind inaktiviert durch Insertion der P450-Gen-basierenden Herbizidtoleranz; diese Pflanze ist männlich steril, weist zudem aber eine gute Herbizidtoleranz auf (2-fache der vom Hersteller empfohlenen Menge des Herbizids Nicosulfuron). Vorzugsweise wird eine Pflanze eines solchen Typs als maternale Komponente bei der Hybridisierung eingesetzt.
Figur 18: Hybridweizensystem: Flussdiagramm der wesentlichen Verfahrensschritte: der Herstellung von hybridem Saatgut von Weizen. Benannte Pflanzentypen verweisen auf die in Figur 17 beschriebenen Pflanzentypen.

Die folgenden Beispiele erläutern die Erfindung, schränken die Erfindung jedoch in keiner Weise ein.

### Beispiel 1: Herstellen einer transaenen Weizenpflanze mit Herbizidtoleranz

### 1.1. Einführen des Transgens P450 in die Weizenpflanze

Das Herbizidtoleranz-Gen P450 mit der in SEQ ID NO: 27 gezeigten Sequenz wurde mit Hilfe von Agrobacterium-vermittelter Transformation in eine Wildtyp-Weizenpflanze der Sorte Taifun eingeführt.

Dazu wurden Weizenpflanzen der Sorte Taifun im Gewächshaus angezogen. Die Anzuchtbedingungen waren 18°C am Tag und 16°C in der Nacht, wobei die Tageslänge 16 Std. betrug. Als Beleuchtungsquelle wurden Natriumlampen verwendet (Master SON-T Agro 400W). Die Embryonengröße in den sich entwickelnden Ähren wurde regelmäßig überprüft und Ähren, die Körner mit Embryonen enthielten, die ca. 1,5 - 2,5 mm Größe hatten, wurden geerntet und bis zur weiteren Verwendung in Wasser stehend bei 4 °C im Dunkeln gelagert.

Als Vorbereitung auf die Isolierung der unreifen Weizenembryonen wurden die Körner aus den Weizenähren isoliert und anschließend oberflächensterilisiert. Dazu wurden die Körner zunächst 45 Sekunden in 70% Ethanol inkubiert und anschließend 10 Minuten in 1% Natriumhypochloritlösung inkubiert. Nach der Sterilisation wurden die Körner durch mehrmaliges Waschen in sterilem Wasser von noch anhaftendem Natriumhypochlorit befreit. Die sterilisierten Körner wurden dann bis zur weiteren Verwendung bei 4°C im Dunkeln gelagert.

Die Anzucht von *Agrobacterium tumefaciens* zur Transformation erfolgte ausgehend von einer Glycerinkultur des *A. tumefaciens* Stammes AGL1, welcher das zu transformierende Genkonstrukt im Binärvektor p6U-70Subiintron-P450-wheat (Figur 1) trägt. Das Genkonstrukt enthält ein Codon-optimiertes P450-Gen mit der Sequenz der SEQ ID NO: 27.

Nach Ausstrich auf selektivem LB-Medium (mit 100 mg/L Rifampicin, 100 mg/L Carbenicillin, 50 mg/L Spectinomycin, 25 mg/L Streptinomycin) wurde mit einer Einzelkolonie eine 2 ml Flüssigkultur in MG/L-Medium (Wu et al., 2009) mit 100 mg/L Rifampicin, 100 mg/L Carbenicillin, 50 mg/L Spectinomycin, 25 mg/L Streptinomycin angeimpft und über Nacht bei 28°C und 200 rpm angezogen. 250 µl der Flüssigkultur wurden am nächsten Tag für das Animpfen von 50 ml frischem MG/L Medium (100 mg/L Rifampicin, 100 mg/L Carbenicillin, 50 mg/L Spectinomycin, 25 mg/L Streptinomycin) verwendet und die Kultur über Nacht bei 28°C und 200 rpm angezogen. Ein Aliquot der Über-Nacht-Kultur wurde anschließend abzentrifugiert (5 Minuten bei 4°C und 3500 x g), der Überstand verworfen und das Bakterienpellet im gleichen Volumen *Inf liquid medium* (Tabelle A) mit 100 µM Acetosyringon resuspendiert. Die so hergestellte Agrobakteriensuspension konnte für die Infektion der unreifen Embryonen verwendet werden.

Aus den sterilisierten Weizenkörnern wurden die unreifen Embryonen isoliert und im *Inf liquid medium* (Tabelle 1) gesammelt. Anschließend wurden die Embryonen einmal mit frischem *Inf liquid medium* gewaschen und dann durch Zentrifugation bei 15.000 rpm für 10 Minuten vorbehandelt. Zur Infektion mit den Agrobakterien wurde die vorbereitete Agrobakteriensuspension auf die Embryonen gegeben und die Embryonen wurden für 30 Sekunden in der Agrobakteriensuspension geschwenkt. Im Anschluss daran wurden die Embryonen für weitere 5 Minuten bei Raumtemperatur in der Agrobakteriensuspension inkubiert. Die unreifen Embryonen wurden dann auf *Co-Cul* Medium (Tabelle 1) gegeben und zwar mit der Scutellum-Seite nach oben. Die so behandelten Explantate wurden für zwei Tage bei 23°C im Dunkeln inkubiert.

Nach zwei Tagen Co-Kultur der unreifen Weizenembryonen mit den Agrobakterien wurde die Embryoachse mittels eines scharfen Skalpells von jedem Embryo entfernt und die verbleibenden Scutella wurden auf ein *resting medium* (Tabelle 1) gesetzt. Die Platten mit den Scutella wurden im Anschluss 5 Tage bei 25°C im Dunkeln inkubiert. Anschließend wurde der sich entwickelnde Kallus noch einmal bei 25°C im Dunkeln auf dem *resting medium* unter Zusatz des Antibiotikums Hygromycin (30 mg/L)(Tabelle 1) für 21 Tage subkultiviert.

Der induzierte Kallus wurde im Ganzen auf *LSZ medium* unter Zusatz des Antibiotikums Hygromycin (30 mg/L) (Tabelle 1) umgesetzt und für 14 Tage ins Licht gestellt. Sich bildende, grüne Sprosse wurden vom Kallus getrennt und auf *LSFmedium* unter Zusatz des Antibiotikums Hygromycin (15 mg/L) (Tabelle A) zur Bewurzelung umgesetzt. Dabei wurden die Sprosse, soweit dies bereits möglich war, voneinander getrennt um Einzelsprosse zu erhalten.

Nach ausreichendem Längenwachstum der Sprosse konnten diese Sprosse auf Anwesenheit des Transgens hin untersucht werden.

Dazu wurden Blattproben der *in vitro* Pflanzen abgenommen und mittels qPCR auf Anwesenheit und Kopienzahl des hpt-Gens untersucht. Bevorzugt wurden solche in vitro Sprosse in das Gewächshaus zur Saatgutproduktion überführt, die eine single copy-Integration des Transgens zeigten.

**Tabelle 1: Zusammensetzung der einsetzbaren Medien**

| *Inf liquid medium* | | *Co-Cul medium* | | *resting medium* | |
|---|---|---|---|---|---|
| 1/10 x | MS anorganische Salze | 1/10 x | MS anorganische Salze | 1x | MS anorganische Salze |
| 1X | MS vitamine | 1X | MS vitamine | 1X | MS vitamine |
| 10 g/L | Glucose | 10 g/L | Glucose | 40 g/L | Maltose |
| 0,5 g/L | MES | 0,5 g/L | MES | 0,5 g/L | Glutamin |
| | | 100 µM | Acetosyringon | 0,1 g/L | Caseinhydrolysat |
| | | 5 µM | Silbernitrat | 0,75 g/L | MgCL2 x 7H2O |
| | | 5 µM | Kupfersulfat | 1,95 g/L | MES |
| | | 8 g/L | Agarose | 100 mg/L | Ascorbinsäure |
| | | | | 150 mg/L | Timentin |
| | | | | 2,2 mg/L | Picloram |
| | | | | 0,5 mg/L | 2,4-D |
| | | | | 2 g/L | Gelrite |

| *LSZ medium* | | *LSF medium* | | | |
|---|---|---|---|---|---|
| 1x | LS anorganische Salze | 1x | LS anorganische Salze | | |
| 1X | LS vitamine (Ishida et al., 2007) | 1X | LS vitamine (Ishida et al., 2007) | | |
| 20 g/L | Sucrose | 15 g/L | Sucrose | | |
| 0,1 mM | Fe-EDTA | 0,1 mM | Fe-EDTA | | |
| 5 mg/L | Zeatin | 0,2 mg/L | Indolbuttersäure | | |
| 10 µM | Kupfersulfat | 10 µM | Kupfersulfat | | |
| 0,5 g/L | MES | 0,5 g/L | MES | | |
| 150 mg/L | Timentin | 150 mg/L | Timentin | | |
| 8 g/L | Aqar | 3 g/L | Gelrite | | |

### 1.2. Bestimmen der Herbizidtoleranz

Die transgenen *in vitro* Sprosse wurden zur Saatgutproduktion angezogen und geselbstet, um T1 Saatgut zu produzieren.

Gemäß den Mendelschen Regeln wird das eingebrachte Transgen im Verhältnis 1:2:1 vererbt, so dass man durch qPCR-Analyse Pflanzen in der T1 Population identifizieren kann, die azygot sind, also das Transgen nicht mehr tragen; die hemizygot sind, also nur eine Kopie des Transgens tragen; und Linien, die homozygot für das Transgen sind, also zwei Kopien des Transgens tragen.

Die T1-Generationen von insgesamt 20 transgenen Pflanzenlinien, hergestellt gemäß Beispiel 1, wurden mit dem Herbizid Nicosulfuron behandelt. Das Herbizid Nicosulfuron wird von Belchim Crop Protection unter dem Handelsnamen Motivell als Dispersion mit einem Wirkstoffgehalt von 60 g/L vertrieben. Die von dem Hersteller für die Landwirtschaft empfohlene Menge (1 x Aufwandsmenge) beträgt 0,75 Uha bei einer Applikationsmenge von 400 Uha. Somit wird in der Landwirtschaft eine ~ 0,20 % ige Lösung der Herbizidformulierung auf die zu behandelnde Fläche appliziert, was einer Wirkstoffkonzentration von 0,12 g/L entspricht.

**Tabelle 2: Übersicht der für P450-vermittelte Herbizidtoleranz einsetzbaren Herbizide. Die Aufwandsmenge basiert auf 400L Wasser pro ha. Bei einer 1x Aufwandmenge wird somit beispielsweise mit 0,12 g/L Nicosulfuronlösung gesprüht (60g/L * 0,20 / 100).**

| **Herbizid** | **Wirkstoff** | **1x** | **2x** | **4x** |
|---|---|---|---|---|
| Motivell | Nicosulfuron 60g/L | 0,20% | 0,40% | 0,80% |
| Callisto | Mesotrione 100g/L | 0,38% | 0,75% | 1,50% |
| U-46 Fluid | 2,4D 500g/L | 0,3% | 0,6% | 1,2% |

Die Herbizidanwendung erfolgte mit einer handelsüblichen Handspritze (http://www.hygi.de/gloria_druckspruehgeraet_hobby_100,pd,46929.html?mcid=5&gclid=CK3zh qqass8CFdXNGwodvUlBsQ). Die Ausbringung erfolgte drei Wochen nach Aussaat im Ein- bis Dreiblattstadium des Weizen (BBCH 11-13). Die Bonitur auf Phytotoxizität erfolgte 20 Tage nach Applikation des Herbizides.

Die Wirkung des Herbizids auf die Pflanzen wurde nach der Behandlung anhand des Schadensbildes der Pflanzen gemessen. Dabei wurde die in Tabelle 3 gezeigte Klassifizierung verwendet.

**Tabelle 3: Bonitur-Tabelle zur Bestimmung der Auswirkung einer Herbizidbehandlung an den behandelten Pflanzen (Schadensbild)**

| **Phytotoxizität:** | **Beschreibung / Schadensbild** |
|---|---|
| 1 | gesund, keine Effekte sichtbar |
| 2 | schwache Phytotoxizität; bis zu 25 % der Blattfläche zeigen Nekrosen oder Ausbleichung |
| 3 | mittlere Phytotoxizität; > 25 -50 % der Blattfläche zeigen Nekrosen oder Ausbleichung |
| 4 | hohe Phytotoxizität; > 50 % der Blattfläche zeigen Nekrosen oder Ausbleichung |
| 5 | Pflanze ist abgestorben |

Die Ergebnisse der Messung sind in FIG. 2 gezeigt. Danach überleben nur die Pflanzen, bei denen das Herbizidtoleranz-Gen homozygot vorliegt, die Behandlung mit der verwendeten hohen Herbiziddosis. Pflanzen, die nur eine Kopie des Herbizidtoleranz-Gens aufweisen (hemizygot) oder keine Kopie des Herbizidtoleranz-Gens aufweisen (azygot) dagegen überleben die Behandlung nicht.

Zum Zwecke des Nachweises des Transkripts des in das TDF-Gen inserierten Transgens, welches als Marker (z.B. P450-Gen) dient, finden sich im Stand der Technik zahlreiche bekannte Methoden: Zum Beispiel das Umschreiben einer von dem Transgen abgeleiteten RNA in cDNA und anschließende Polymerase Chain Reaction (RT-PCR; Sambrook et al., 2001), die Hybridisierung einer detektierbaren, einzelsträngigen Nukleinsäure, die komplementär zum eingebrachten Transgen ist, mit der RNA der transgenen Pflanze (Northern Blot, Sambrook et al., 2001) oder das Umschreiben einer von dem Transgen abgeleiteten RNA in cDNA und anschließender Sequenzierung des gesamten Pools an erhaltener cDNA. Das kodierte Peptid / Polypeptid / Protein kann beispielsweise mittels Immunodetektion über unterschiedliche Methoden wie Western Blot oder ELISA identifiziert werden. Ferner kann zum Selektieren einer phänotypischen Eigenschaft, welche durch das Transgen direkt oder indirekt vermittelt wird, nachgewiesen werden. Ein solcher phänotypischer Nachweis kann auch den Nachweis einer geänderten chemischen Zusammensetzung der Pflanzenzelle einschließen. Diese geänderte chemische Zusammensetzung kann dann mittels bekannter Methoden der chemischen Analyse nachgewiesen werden.

Beispielhaft erfolgte in der vorliegenden Erfindung der Nachweis des Transkripts und insbesondere die Quantifizierung des Transkripts über die qRT-PCR (quantitative Real-Time Polymerase Chain Reaction). Hierfür wurde aus ca. 20 mg Blattmaterial die gesamt RNA extrahiert. Anschließend wurde die mRNA unter Verwendung von kommerziell erhältlichen Reverse Transkriptase Kits (z.B. der RevertAid™ First Strand cDNA Sythesis Kit) in cDNA umgeschrieben. Über das in Tabelle 4 dargestellte Primer/Sondensystem wurde dann mittels qRT-PCR die Menge an Transkript bestimmt. Dazu wurde folgender PCR Ansatz zusammen pipettiert:

| | | **[µ]** |
|---|---|---|
| QuantiFAST Multiplex | | |
| PCR Kit | | 10 |
| TaEF1Bxxxf01 | | 0,9 |
| TaEF1Bxxxr01 | | 0,9 |
| p450xxxr01 | | 0,9 |
| p450xxxf01 | | 0,9 |
| TaEF1 BxxxMGB | Sonde | 0,5 |
| p450xxxMGB | Sonde | 0,5 |
| H2O | | 0,4 |
| cDNA | | 5 |

Die PCR wird in dem QuantStudio 6^{™} qPCR Gerät gemäß den Vorgaben des Herstellers durchgeführt und die relative Menge an P450 spezifischer mRNA wird anhand einer Normalisierung auf das endogene Kontrollgen TaEF1 durchgeführt.

**Tabelle 4: Quantitative RT-PCR mit Hilfe eines geeigneten Primer/Sondensystems**

| **Name** | **Oligo Sequenz** | **SEQ ID NO** | |
|---|---|---|---|
| p450xxxMGB | FAM-AGCATGGCAATGATGGA | 31 | FAM markierte Sonde |
| p450xxxr01 | GACGCCGGCGAGAAGAA | 32 | |
| p450xxxf01 | TCTGGCTCGGACTTTTGCA | 33 | |
| TaEF1 Bxxxmbg | TGACAAGGTTCCCTTCGT | 34 | VIC markierte Sonde |
| TaEF1Bxxxf01 | ACCTGAAGAAGGTCGGCTACAAC | 35 | |
| TaEF1Bxxxr01 | TCACCCTCAAACCCAGAGATG | 36 | |

### 1.3. Bestimmen des Expressionslevels des Herbizidtoleranz-Genes in der Pflanze

Die unterschiedliche Toleranz gegenüber hohen Dosen des Herbizids könnte auf Unterschiede in der Expressionshöhe des Toleranzgenes P450 zurückzuführen sein. Um diese Hypothese zu überprüfen, wurde in 36 Individuen der T1-Generation der gemäß Beispiel 1 hergestellten transgenen Pflanze mit Hilfe der unter 1.2. beschriebenden qRT-PCR die Menge des P450-Transkripts bestimmt.

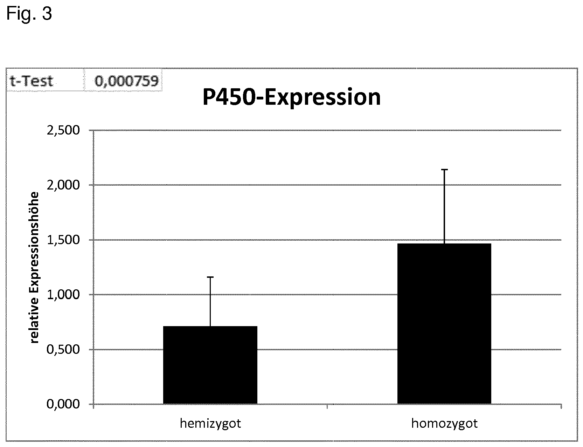

Die Ergebnisse sind in Figur 3 gezeigt. Demnach ist die Menge an Transkript des Herbizidtoleranz-Gens in hemizygoten Pflanzen etwa halb so hoch wie in Pflanzen, in denen das Herbizidtoleranz-Gen homozygot vorliegt. Dies ist durchaus bemerkenswert, da das Vorliegen von zwei Kopien eines Transgens nicht zwangsläufig zu einer Verdoppelung der Transkriptmenge führen muss.

### Beispiel 2: Herstellen einer männlich sterilen, transgenen Weizenpflanze

### 2.1. Identifizieren von Kandidatengenen in Weizen (Triticum aestivum)

Zur Identifizierung geeigneter Kandidaten-Gene wurden folgende Auswahlkriterien angelegt:
1) Gene codieren für Transkriptionsfaktoren, da diese eine Vielzahl von anderen Genen steuern, sollte eine Mutation bzw. Inaktivierung dieser Gene zu stärkeren Effekten führen, als die Mutation bzw. Inaktivierung eines Strukturgens;
2) Gene werden in anderen monocotyledonen Pflanzen wie beispielsweise Reis ausschließlich Antheren-spezifisch exprimiert

Anhand der Liste möglicher Kandidatengene wurden, sofern möglich, die jeweiligen Weizenhomologe identifiziert. Anschließend wurden die verfügbaren Expressionsdaten der Gene analysiert. Es wurde nun eine weitere Einschränkung vorgenommen auf solche Weizengene, die eine möglichst spezifische Expression in den Antheren hatten.

### 2.2. Identifizieren von zur gezielten Mutation geeigneten Bereichen innerhalb des Weizengenoms

Auf Basis der vorhandenen Genomdaten des Weizens wurden nun Genvorhersagemodelle für die identifizierten Gene erstellt. Ziel war es, Exonbereiche für eine gezielte Mutagenese zu nutzen, um eine Veränderung der Proteinstruktur oder einen vorzeitigen Abbruch der Translation zu erreichen. Die Mutagenese sollte dann die männliche Sterilität zur Folge haben. Erfolgreich gelang dies nur im Fall eines Kandidatengens, nämlich dem TaTDF-Gen, weshalb sich die nachfolgende Ausführungen ausschließlich auf die Arbeiten an und mit dem TaTDF-Gen beschränken.

Nach Vorlage der Exon/Intronstruktur konnten Bereiche in den Exons des TaTDF-Gens identifiziert werden, die in allen drei Subgenomen des Weizens identisch oder sehr ähnlich waren. Diese Bereiche wurden dann für die Entwicklung von TALENS genutzt (Figur 4).

### 2.3. Erzeugen von TALENs-Konstrukten und deren Verwendung zur Transformation von Weizen

Es wurden zweimal 19 bp bzw. 25 bp des genomischen Bereichs als Bindungsstelle für eine TAL-Nuklease ausgewählt, mit einem Abstand von 12-18 bp zwischen den beiden Bindungsstellen. Figur 4 zeigt die Exon/Intron Struktur des Gens TaTDF aus Weizen sowie die Bindungsstellen für die beiden TALEN-Arme TaTDF-TAL1 und TaTDF-TAL2, die mit einem Abstand von 14 bp auf dem codogenen bzw. dem nicht codogenen DNA-Strang binden.

Nach Erstellung der TALENs wurden diese mit dem starken, konstitutiven Promotor 70Subiintron fusioniert und entweder in den Binärvektor p7U oder p6U kloniert (Figur 5). Die Transformation der beiden TALEN-Arme in die Weizensorte Taifun erfolgte dann als Co-Transformation durch Mischen der beiden Agrobakterienstämme zu gleichen Teilen. Selektiert wurden die transgenen Pflanzen dann im Versuch WA499 mittels Hygromycin, so dass alle generierten transgenen Pflanzen das Konstrukt p6U70SubintronTaTDF-TAL2 enthielten, ein Teil der Pflanzen aber auch noch zusätzlich das Konstrukt p7U70SubiintronTaTDF-TAL1. Im ersten Transformationsversuch (WA499) konnten 9 transgene Weizenlinien identifiziert werden, in denen beide TALEN-Arme integriert waren. Im zweiten Transformationsversuch (WA500) wurde dann ebenfalls eine Co-Transformation durchgeführt. Hier wurde jedoch durch Zusatz von Phosphinotricin in das Regenerationsmedium auf Anwesenheit des *bar*-Gens selektiert. In diesem Versuch konnten 13 transgene Sprosse identifiziert werden, die beide TALEN-Arme enthielten.

### 2.4. Identifizieren von Deletionen in den transgenen Weizenpflanzen

Die transgenen Ausgangstransformanten aus Beispiel 2.3. wurden auf Deletionen im Bereich des Spacers zwischen den beiden TALEN-Armen untersucht.

Dazu wurde die Methode der Enrichment PCR angewandt (Curtin et al., 2011). Bei dieser Methode wird sich zu Nutze gemacht, dass durch die Wirkung der TALENS entweder Deletionen oder Insertionen im Bereich der Schnittstelle, also im Bereich des Spacers zwischen den Bindungsstellen der beiden TALEN-Arme, entstehen. Dies kann dazu führen, dass eine vorhandene Restriktionsschnittstelle im Bereich des Spacers mutagenisiert wird, und somit das Restriktionsenzym nicht mehr schneiden kann.

Für die Bindungsstelle des TALENs für das TaTDF-Gen wurde das Restriktionsenzym Rsal identifiziert, welches im Bereich des Spacers innerhalb der genomischen Weizen-DNA schneiden sollte (Figur 4).

Für die Enrichment-PCR wurde nun isolierte, genomische DNA der transgenen Weizenlinien mit dem Enzym Rsal verdaut. Mutagenisierte DNA sollte nun nicht verdaut werden, so dass bei einer anschließenden PCR über den zu mutagenisierenden Bereich des Gens nur solche DNA amplifiziert werden sollte, die nicht verdaut wurde, also durch die Wirkung des TALEN mutagenisiert wurde. Da ein Restriktionsverdau auf genomische DNA niemals vollständig ist, wird es jedoch auch immer zur Amplifikation der nicht mutagenisierten DNA kommen. In einem zweiten Schritt wurde daher das amplifizierte PCR-Produkt noch einmal mit dem Enzym Rsal verdaut. Sollten hier unverdaute Fragmente entstehen, liegt die Vermutung nahe, dass der amplifizierte Genlocus tatsächlich durch die Wirkung des TALEN mutagenisiert wurde. Um diese Vermutung zu bestätigen, wurden die generierten, unverdauten PCR-Produkte in den Klonierungsvektor pGEM-T kloniert und sequenziert.

Tatsächlich konnten in den generierten, transgenen Weizenlinien der Transformationen WA499 und WA500 zahlreiche Deletionen im Bereich der TALEN-Bindungsstelle detektiert werden. Diese reichten von einer Deletion von nur 1 bp bis zu 28 bp (Figur 6).

Des Weiteren ist zu beachten, dass für die Extraktion der DNA für die Enrichment PCR Blattmaterial verwendet wurde. Die TALENs sollten in allen Zellen aktiv sein. Somit könnte es sein, dass die detektierten Mutationen nur in dem analysierten Blattgewebe vorliegen, in anderen Teilen der Pflanze jedoch nicht. Dieser chimäre Charakter der Pflanze erfordert es, dass die transgenen Pflanzen in die nächste Generation überführt werden. Dann sollte die chimäre Natur der Pflanzen eliminiert sein, und die Mutation sollte in allen Zellen der Pflanze vorliegen und dadurch gemäß den Mendelschen Regeln vererbt werden.

### 2.5. Erzeugen und Analyse von Pflanzen der T1-Generation

Die transgenen Ausgangs-Weizenlinien, für die Mutationen im Bereich des TaTDF-Gens nachgewiesen werden konnten, wurden daher durch Selbstung in die nächste Generation überführt.

Die aus den Körnern der T0-Pflanzen hervorgegangenen Pflanzen (T1-Generation) wurden erneut mittels PCR auf Vorhandensein der Mutation getestet. Dabei wurde zunächst eine PCR auf die einzelnen Weizenpflanzen gemacht, um dann die PCR Produkte anschließend mittels Rsal-Verdau auf Mutagenese des TaTDF-Gens zu testen. Ein vorheriger Verdau der genomischen Weizen-DNA vor der PCR Reaktion war nun nicht mehr nötig, da davon ausgegangen werden konnte, dass eine evtl. vorhandene Mutation nun in allen Zellen der Pflanze vorliegt.

Es stellte sich mittels des PCR Assays heraus, dass nicht alle Pflanzen die in der T0-Generation gefundene Mutation weitervererbten.

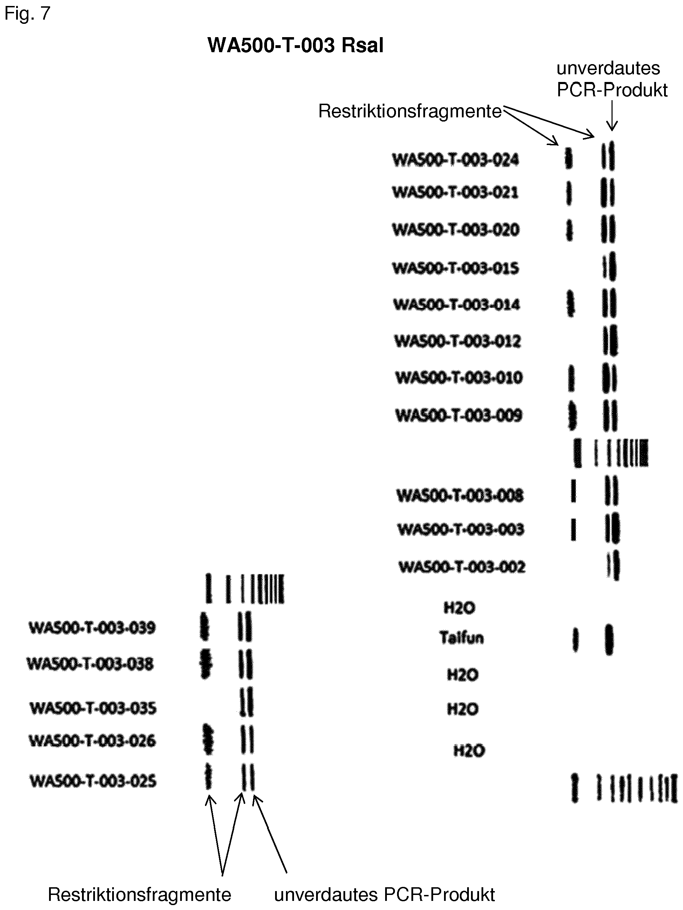

Besonderes Interesse weckte die Linie WA500-T-003, da hier in der T0-Pflanze in der Enrichment PCR nahezu die gesamte amplifizierte DNA nicht durch Rsal geschnitten werden konnte. In dem PCR-Assay auf die Selbstungsnachkommenschaft dieser Linie wurde daher erwartet, auch solche Pflanzen zu identifizieren, die nach Verdau der amplifizierten DNA nur unverdautes PCR Produkt nach Gelanalyse zeigen würden. Diese Pflanzen wären dann homozygot für die Mutation. Erstaunlicherweise war dies jedoch nicht der Fall, sondern es wurde in allen Pflanzen sowohl unverdaute als auch verdaute DNA Fragmente identifiziert (Figur 7). Überraschend war allerdings, dass das Verhältnis zwischen verdauten und unverdauten PCR Fragmenten unterschiedlich war. Dies ließ den Schluss zu, dass in der Linie WA500-T-003 zwar eine Mutation im TaTDF-Gen stattgefunden hatte, aber möglicherweise nicht auf allen Chromosomen bzw. nicht auf allen Chromosomen der drei Weizen(sub)genome.

### 2.6. Subgenom-spezifische Analyse der Pflanzen der T1-Generation

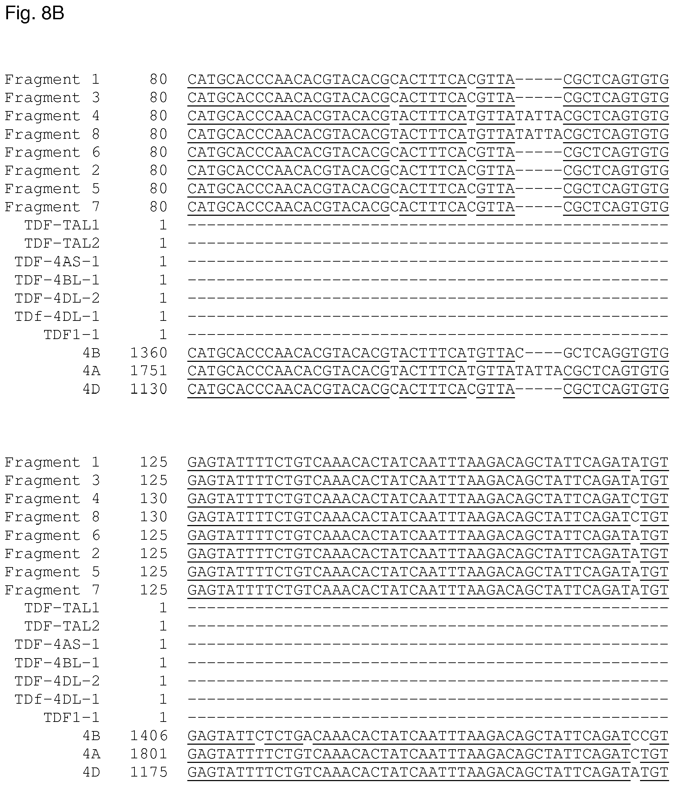

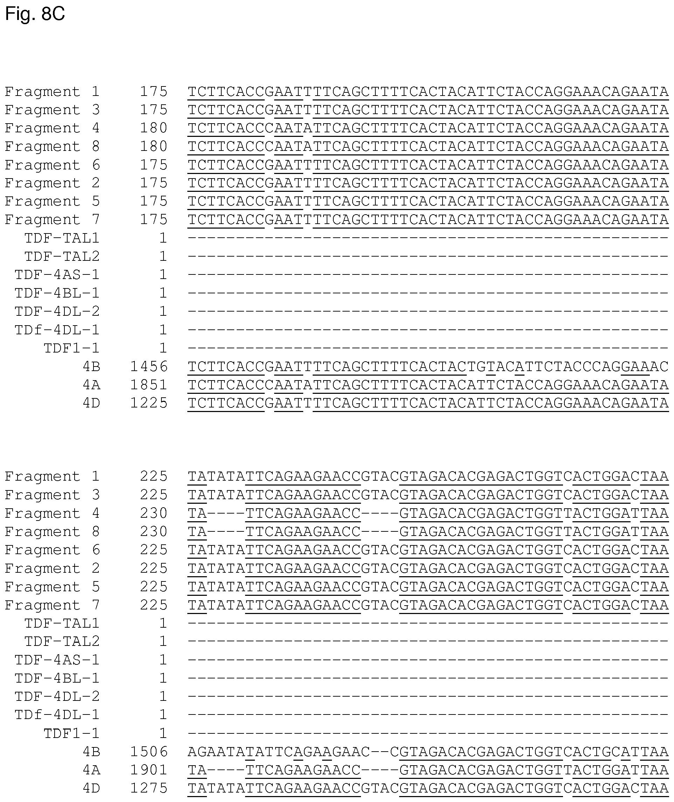

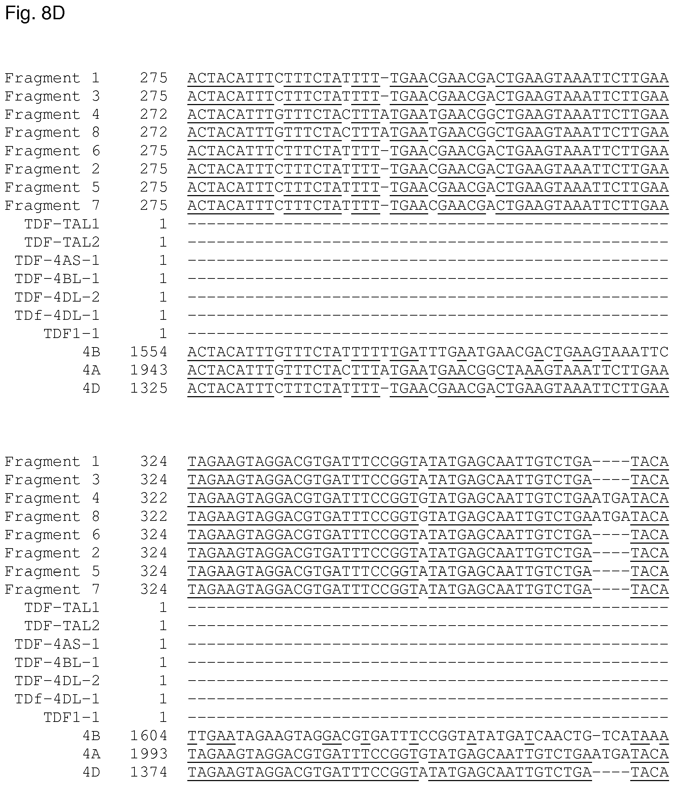

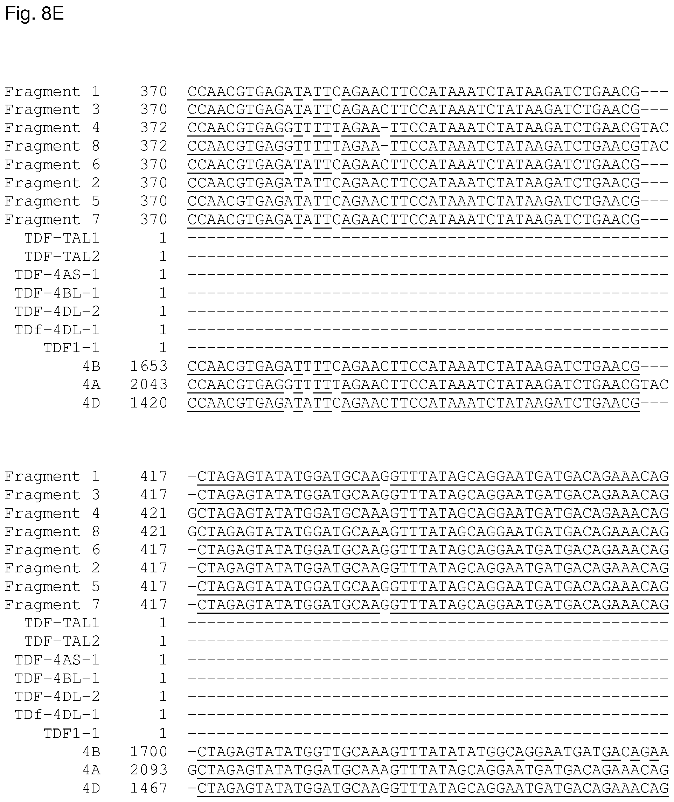

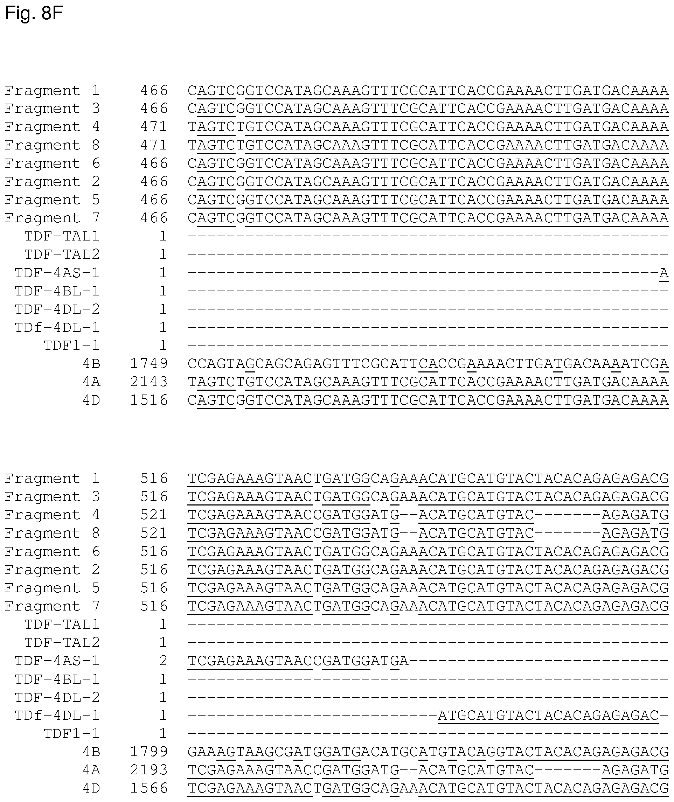

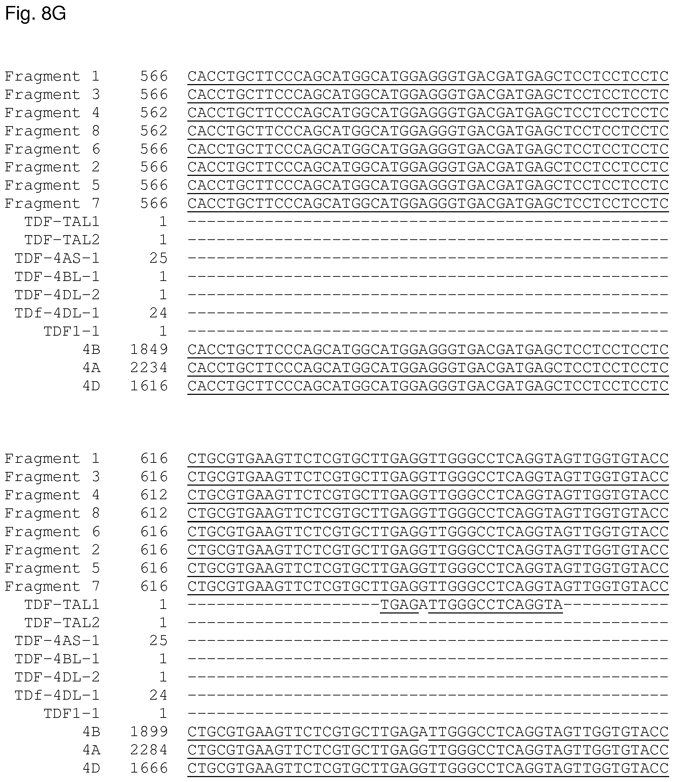

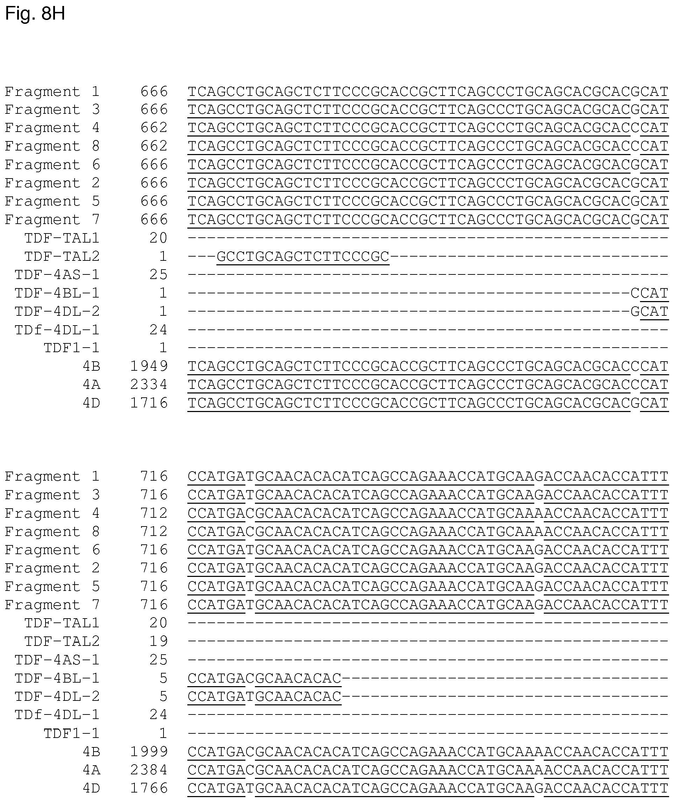

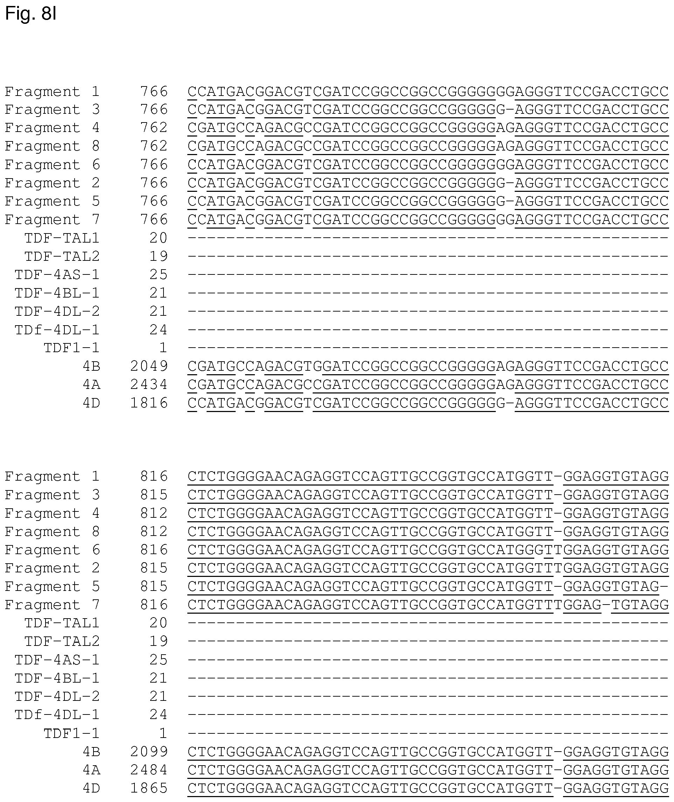

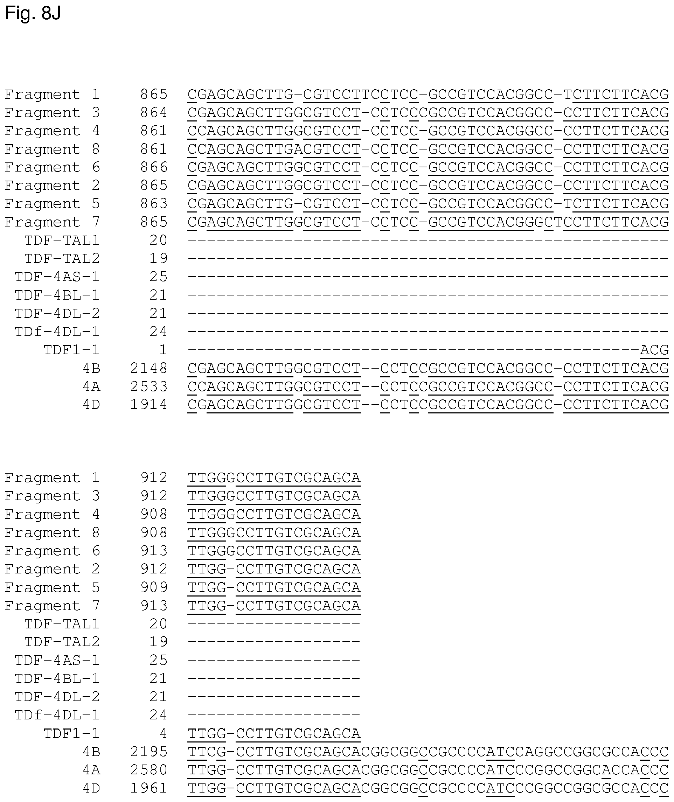

Durch Amplifikation von ca. 1 kb langen Fragmenten aus dem Genom der Weizensorte "Taifun" im Bereich des TaTDF-Gens und anschließender Sequenzierung von 8 klonierten PCR-Fragmenten, konnten die Sequenzen des TaTDF-Locus in den einzelnen Subgenomen identifiziert werden (Figur 8). Dementsprechend konnten Primersysteme entwickelt werden, die spezifisch den TaTDF-Locus nur eines Subgenoms amplifizieren (Tabelle 5).

**Tabelle 5: Primersysteme für die subgenomspezifische Amplifikation des Weizen TaTDF-Gens.**

| **Nachweis Subgenom** | **Primer** | **SEQ ID NO** | **Sequenz** | **Fragmentgröße** |
|---|---|---|---|---|
| **A** | TDF-4AS-1 | 13 | ATCGAGAAAGTAACCGATGGATGA | 406 bp |
| | TDF1-1 | 14 | TGCTGCGACAAGGCCAACGT | |
| **B** | TDF-4BL-1 | 15 | GTGTGTTGCGTCATGGATGG | 190 bp |
| | TDF-4DL-1 | 16 | ATGCATGTACTACACAGAGAGAC | |
| **D** | TDF-4DL-1 | 17 | ATGCATGTACTACACAGAGAGAC | 190 bp |
| | TDF-4DL-2 | 18 | GTGTGTTGCATCATGGATGC | |

Die entwickelten Primer wurden dann wieder für das PCR Assay eingesetzt, bei dem das amplifizierte Fragment des TaTDF-Gens anschließend mit Rsal verdaut wurde.

Dabei konnten für die Nachkommen der Linie WA500-T-003 Mutationen in allen drei Subgenomen festgestellt werden (Tabelle 6).

**Tabelle 6: Analyse der Mutationen im TaTDF-Locus von Nachkommen der Linie WA500-T-003 mittels Subgenom-spezifischer PCR. Unterstrichen markiert sind Pflanzen, die Mutationen in allen drei Subgenomen des TaTDF-Locus tragen (WA500-T-003-003, WA500-T-003-009, WA500-T-003-012, WA500-T-003-015, WA500-T-003-020, WA500-T-003-021, WA500-T-003-025, WA500-T-003-026 und WA500-T-003-039).**

| | A-Subgenom | | D-Subgenom | | B-Subgenom | |
|---|---|---|---|---|---|---|
| Name | hetero | homo | hetero | homo | hetero | homo |
| WA500-T-003-002 | - | + | - | + | - | - |
| WA500-T-003-003 | - | + | + | - | - | + |
| WA500-T-003-008 | - | + | + | - | - | - |
| WA500-T-003-009 | - | + | + | - | + | - |
| WA500-T-003-010 | - | + | - | - | - | + |
| WA500-T-003-012 | - | + | - | + | + | - |
| WA500-T-003-014 | - | + | + | - | - | - |
| WA500-T-003-015 | - | + | + | - | + | - |
| WA500-T-003-020 | - | + | + | - | + | - |
| WA500-T-003-021 | - | + | - | + | + | - |
| WA500-T-003-024 | - | + | - | - | - | + |
| WA500-T-003-025 | - | + | - | + | + | - |
| WA500-T-003-026 | - | + | + | - | + | - |
| WA500-T-003-035 | - | + | - | + | - | - |
| WA500-T-003-038 | - | + | + | - | - | - |
| WA500-T-003-039 | - | + | + | - | + | - |

PCR-Produkte des TaTDF-Locus spezifisch für das A-, B- oder D-Subgenom wurden generiert und anschließend mit Rsal verdaut. Bei Vorliegen von ausschließlich unverdauter DNA wurde davon ausgegangen, dass die Mutation homozygot vorliegt, bei Vorliegen von unverdauter und verdauter DNA wurde davon ausgegangen, dass die Mutation im heterozygoten Zustand vorliegt.

Erstaunlicherweise konnten dabei innerhalb der Nachkommenschaft der Linie WA500-T-003 Einzelpflanzen identifiziert werden, die in allen drei Genloci des TaTDF-Gens eine Mutation aufwiesen. Die Mutation im A-Subgenom lag bereits homozygot vor, so dass davon ausgegangen werden muss, dass bereits in der T0-Pflanzen beide Genloci des TaTDF-Gens auf dem A-Subgenom durch die Wirkung des TALEN mutagenisiert wurden.

Um in zukünftigen Analysen den Arbeitsaufwand zu reduzieren, wurden basierend auf den Primersystemen Markersysteme für die Kapillarelektrophorese entwickelt. Diese Markersysteme konnten eindeutig die Mutationen auf dem A-, B- und D-Subgenom nachweisen, sowie zwischen homozygoten und heterozygoten Pflanzen unterscheiden (Tabelle 7).

**Tabelle 7: Subgenom-spezifische Untersuchung des TaTDF-Locus mittels Kapillarelektrophorese. DNA der Nachkommen der Linie WA500-T-003 wurde für eine PCR des TaTDF-Locus mit fluoreszenzmarkierten, Subgenom-spezifischen Primern eingesetzt. Die PCR-Produkte wurden dann in der Kapillarelektrophorese aufgetrennt, um so Größenunterschiede von ≥ 1 bp zu detektieren. Als Kontrolle wurden DNAs von verschiedenen kommerziell erhältlichen fertilen Weizensorten eingesetzt (letzte 7 Zeilen). Im A-Subgenom konnte in den WA500-T-003-Nachkommen eine 2 bp Deletion festgestellt werden, die als 403 bp Fragment anstelle eines 405 bp Fragmentes in der Kapillarelektrophorese zu erkennen ist. Tritt nur das 403 bp Fragment auf, so liegt die Mutation homozygot vor; können sowohl das 403 bp als auch das 405 bp Fragment detektiert werden, so liegt die Mutation heterozygot vor; kann nur das 405 bp Fragment detektiert werden, so liegt die wt-Sequenz homozygot vor. Im D-Subgenom liegt eine Deletion von 3 bp vor, ebenso im B-Subgenom. Beim D-Subgenom wird im wt ein PCR Fragment von 190 bp in der Kapillarelektrophorese detektiert. Tritt die Mutation homozygot auf, so wird nur ein 187 bp Fragment detektiert, im heterozygoten Zustand wird sowohl ein 187 bp als auch ein 190 bp Fragment detektiert. Ähnlich verhält es sich für das B-Subgenom, nur das hier ein 189 bp Fragment im wt detektiert wird, wohingegen in homozygoten Mutanten ein 186 bp Fragment und in heterozygoten Mutanten ein 186 bp und ein 189 bp Fragment detektiert wird. Der Assay ließ ebenfalls eine Aussage darüber zu, ob die Deletion homozygot (einfach unterstrichen) oder heterozygot (doppelt unterstrichen) vorliegt.**

| **Name** | **A-Subgenom** | **D-Subgenom** | **B-Subgenom** |
|---|---|---|---|
| WA500-T-003-002 | 403 | 190 | 189 |
| WA500-T-003-003 | 403 | 187 | 186/189 |
| WA500-T-003-008 | 403 | 190 | 186/189 |
| WA500-T-003-009 | 403 | 187/190 | 186/189 |
| WA500-T-003-010 | 403 | 187 | 189 |
| WA500-T-003-012 | 403 | 187/190 | 186 |
| WA500-T-003-014 | 403 | 190 | 186/189 |
| WA500-T-003-015 | 403 | 187/190 | 186/189 |
| WA500-T-003-020 | 403 | 187/190 | 186/189 |
| WA500-T-003-021 | 403 | 187/190 | 186 |
| WA500-T-003-024 | 403 | 187 | 189 |
| WA500-T-003-025 | 403 | 187/190 | 186 |
| WA500-T-003-026 | 403 | 187/190 | 186/189 |
| WA500-T-003-035 | 403 | 190 | 186 |
| WA500-T-003-038 | 403 | 190 | 186/189 |
| WA500-T-003-039 | 403 | 187/190 | 186/189 |
| | | | |
| Avalon | 405 | 190 | 189 |
| Cadenza | 405 | 190 | 189 |
| Chinese Spring | 405 | 190 | 189 |
| Opus | 405 | 190 | 189 |
| Claire | 405 | 190 | 189 |
| Julius | 405 | 190 | 189 |

### 2.7. Erzeugen und Analyse von Pflanzen, welche die Mutation homozygot tragen

Auf Grundlage der Nachkommenschaftsanalyse der Linie WA500-T-003 wurden zwei Einzelpflanzen ausgewählt, WA500-T-003-003 bzw. WA500-T-003-021. WA500-T-003-003 war homozygot für das A- und D-Subgenom, und heterozygot für das B-Subgenom. WA500-T-003-021 hingegen war homozygot für das A-Subgenom und das B-Subgenom, aber nur heterozygot für das D-Subgenom.

Beide Linien wurden im Gewächshaus geselbstet, und die Nachkommen wurden mit Hilfe des Markersystems auf Anwesenheit der Mutationen in den einzelnen Subgenomen getestet.

Dabei konnten bei beiden Nachkommenschaften Pflanzen identifiziert werden, die für alle drei Subgenome die Mutationen im TaTDF-Genlocus homozygot tragen (Tabelle 8). Diese Pflanzen wurden im Gewächshaus zur Blüte gebracht. Dabei zeigte sich, dass keine der Pflanzen in der Lage war, funktionale Antheren zu bilden, die Pflanzen also männlich steril sind (Figuren 9-11).

**Tabelle 8: Subgenomspezifischer Nachweis von Deletionen im TaTDF-Locus von Nachkommen der Linien WA500-T-003-03 und WA500-T-003-21 mittels Kapillarelektrophoresemarker. Die Unterstreichung makiert Linien, welche für alle drei Deletionen homozygot sind.**

| Name | A-Subgenom | D-Subgenom | B-Subgenom |
|---|---|---|---|
| Taifun | 405 | 190 | 189 |
| WA500-T-003-003-01 | 403 | 187 | 189 |
| WA500-T-003-003-02 | 403 | 187 | 186/189 |
| WA500-T-003-003-03 | 403 | 187 | 189 |
| WA500-T-003-003-04 | 403 | 187 | 186 |
| WA500-T-003-003-05 | 403 | 187 | 186 |
| WA500-T-003-003-06 | 403 | 187 | 189 |
| WA500-T-003-003-08 | 403 | 187 | 186/189 |
| WA500-T-003-003-09 | 403 | 187 | 186/189 |
| WA500-T-003-003-10 | 403 | 187 | 186/189 |
| WA500-T-003-003-11 | 403 | 187 | 186 |
| WA500-T-003-003-13 | 403 | 187 | 186/189 |
| WA500-T-003-003-14 | 403 | 187 | 186 |
| WA500-T-003-003-15 | 403 | 187 | 189 |
| WA500-T-003-003-16 | 403 | 187 | 186/189 |
| WA500-T-003-003-17 | 403 | 187 | 186/189 |
| WA500-T-003-003-18 | 403 | 187 | 186 |
| WA500-T-003-003-19 | 403 | 187 | 186 |
| WA500-T-003-003-20 | 403 | 187 | 186 |
| WA500-T-003-003-21 | 403 | 187 | 186 |
| WA500-T-003-003-23 | 403 | 187 | 186/189 |
| WA500-T-003-003-24 | 403 | 187 | 186 |
| WA500-T-003-003-25 | 403 | 187 | 186 |
| WA500-T-003-021-01 | 403 | 187 | 186 |
| WA500-T-003-021-02 | 403 | 187 | 186 |
| WA500-T-003-021-03 | 403 | 187/190 | 186 |
| WA500-T-003-021-04 | 403 | 187/190 | 186 |
| WA500-T-003-021-05 | 403 | 187/190 | 186 |
| WA500-T-003-021-06 | 403 | 190 | 186 |
| WA500-T-003-021-07 | 403 | 187/190 | 186 |
| WA500-T-003-021-08 | 403 | 187 | 186 |
| WA500-T-003-021-09 | 403 | 190 | 186 |
| WA500-T-003-021-10 | 403 | 190 | 186 |
| WA500-T-003-021-11 | 403 | 187 | 186 |
| WA500-T-003-021-12 | 403 | 190 | 186 |
| WA500-T-003-021-13 | 403 | 187/190 | 186 |
| WA500-T-003-021-14 | 403 | 187/190 | 186 |
| WA500-T-003-021-15 | 403 | 187/190 | 186 |
| WA500-T-003-021-17 | 403 | 187/190 | 186 |
| WA500-T-003-021-18 | 403 | 187/190 | 186 |
| WA500-T-003-021-19 | 403 | 187/190 | 186 |
| WA500-T-003-021-20 | 403 | 187/190 | 186 |
| WA500-T-003-021-21 | 403 | 187 | 186 |
| WA500-T-003-021-22 | 403 | 190 | 186 |
| WA500-T-003-021-23 | 403 | 187 | 186 |
| WA500-T-003-021-24 | 403 | 187/190 | 186 |
| WA500-T-003-021-25 | 403 | 187 | 186 |

Die Ähren dieser Pflanzen (der Nachkommen von WA500-T-003-003 und WA500-T-003-021) wurden dann mit einer Tüte isoliert, um zu prüfen ob doch noch eine Selbstbefruchtung möglich war. In keiner der isolierten Weizenähren konnten Samen gefunden werden. Somit konnte eine 100%-ige männlichen Sterilität etabliert werden.

Die weiblichen Blütenorgane der Pflanzen hingegen sind vollkommen normal ausgebildet, was auf keine Störung der weiblichen Fertilität hindeutet. Auch sonst können an den Pflanzen keine anormalen Phänotypen beobachtet werden.

Um die weibliche Fertilität zu überprüfen, wurden Kreuzungen mit Pollen einer Winterweizensorte durchgeführt. In den Kreuzungsähren wurden Körner in normaler Anzahl gebildet, so dass eine 100%-ige weibliche Fertilität der Ähren nachgewiesen ist.

### Beispiel 3: Herstellen einer männlich sterilen, transgenen Weizenpflanze mit Herbizidtoleranz

### 3.1. Inaktivierung des Fertilitätsgens TDF durch Insertion eines Herbizidtoleranz-Genes

Die besonderen Eigenschaften der oben beschriebenen Herbizid-toleranten sowie der männlich-sterilen Weizenpflanzen können erfindungsgemäß in einer einzigen Pflanze gekoppelt werden.

Die in 2.2. identifizierten Exonbereiche können genutzt werden, um (anstelle kleinerer Deletionen) DNA-Insertionen durchzuführen. Zum Beispiel kann das in 1.1. beschriebene Herbizidtoleranz-Gen P450 (SEQ ID NO: 27) in Exon 2 des TaTDF-Locus insertiert werden. Dazu wird ein Genkonstrukt verwendet, welches die Herbizidtoleranzkassette enthält. Das P450-Transgen kann zum Beispiel durch CRISPR/Cas-vermittelte homologe Rekombination gezielt in das TaTDF-Gen eingefügt werden.

Die genomische Organisation des TaTDF-Gens auf Weizen-Chromosom 4DL ist in Figur 12 gezeigt.

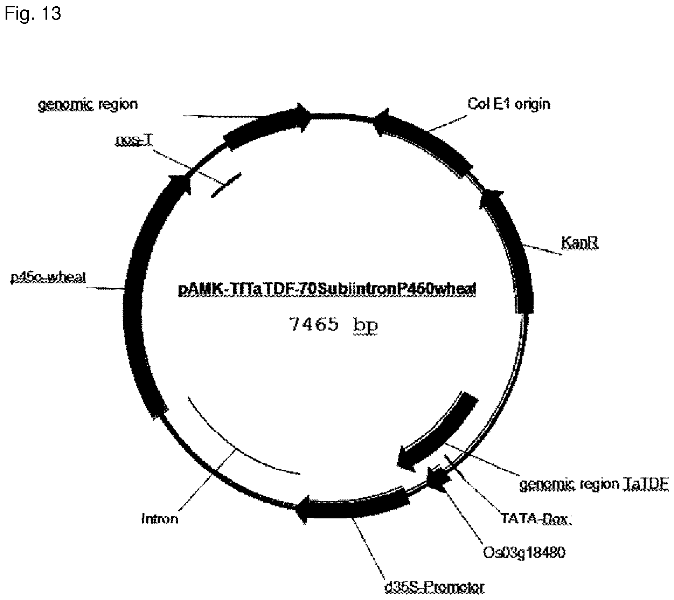

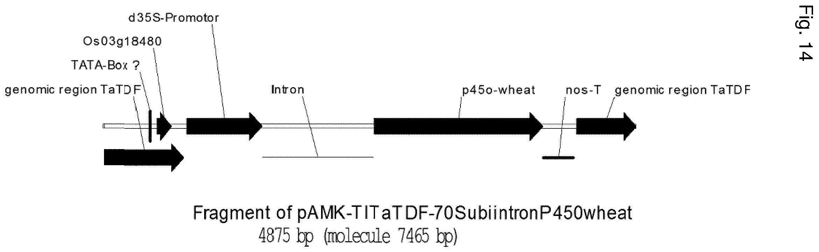

Um eine zielgerichtete Mutation des TaTDF-Gens durch Insertion einer Expressionkassette für das Herbizidtoleranz-Gen P450 zu erreichen, wurde das Plasmid pAMK-TITaTDF-70SubiintronP450wheat erstellt. Figur 13 zeigt das Plasmid pAMK-TITaTDF-70SubiintronP450wheat; Figur 14 zeigt ein Fragment des Plasmids mit der Herbizidtoleranzkassette sowie den für die gerichtete Integration in das Genom des Weizens notwendigen Sequenzabschnitten. Das dargestellte Fragment kann durch Restriktionsenzyme aus dem Plasmid isoliert werden und in andere Plasmid oder Bbinärvektoren umkloniert werden. Dies is insbesondere dann wichtig, wenn eine Transformation des Weizens mittels Agrobacterium tumefaciens vorgesehen ist. Die Nukleotidsequenz dieses Fragmentes ist in SEQ ID NO: 29 gezeigt.

Das Plasmid pAMK-TITaTDF-70SubiintronP450wheat enthält die identische Expressionskassette aus dem Binärvektor p6U-70Subiintron-p450wheat. Jedoch ist die Expressionskassette flankiert von homologer genomischer Sequenz aus dem auf Chromosom 4DL lokalisierten TaTDF-Gen. Der 5' stromaufwärts zu der Sequenz des Promoters d35S liegende genomische Bereich von 739 bp entspricht Teilen der Promotorsequenz des TaTDF-Gens, dem kompletten ersten Exon und Intron des TaTDF-Gens sowie den ersten 8 bp des zweiten Exons des TaTDF Gens. Die 3' stromabwärts liegende homologe Sequenz von 548 bp umfasst die letzten 75 bp des zweiten Exons des TaTDF-Gens sowie große Teile des zweiten Introns.

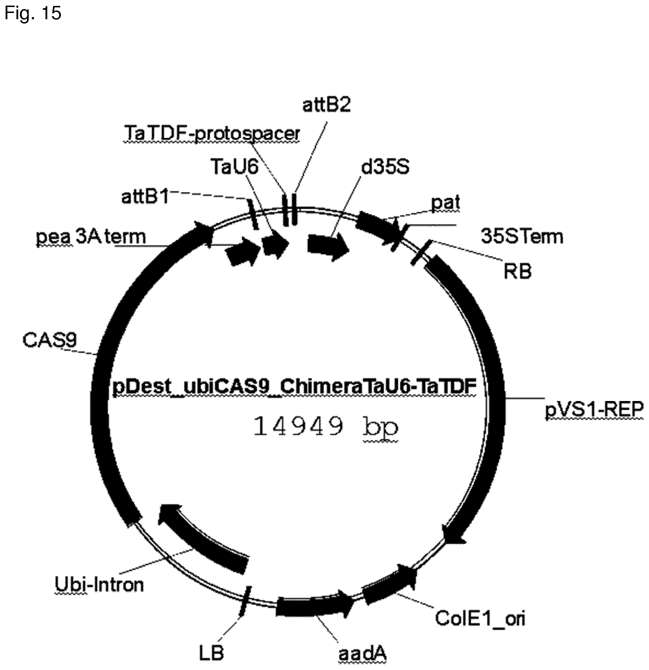

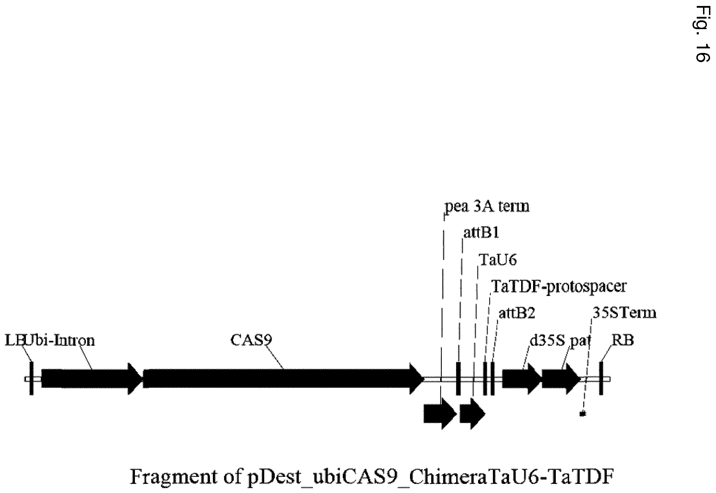

Die flankierenden, homologen Sequenzen sollen die gezielte Integration der transgenen Sequenz in das zweite Exon des TaTDF-Gens ermöglichen. Um dies zu erreichen, muss zunächst ein Doppelstrangbruch in den DNA Strang induziert werden, der für das TaTDF-Gen kodiert. Hierzu können artifizielle Nukleasen, wie z.B. Zink Finger Nukleasen, TALENS oder Meganucleasen verwendet werden. Eine weitere Möglichkeit bietet sich durch die Verwendung eines CRISPR/Cas-Systems. Ein funktionales Konstrukt für die Induktion von Doppelstrangbrüchen im zweiten Exon des TaTDF-Gens ist in Figur 15 dargestellt (pDest_ubiCAS9_ChimeraTaU6-TaTDF). Figur 16 zeigt ein Fragment des Vektors. Die Nukleotidsequenz dieses Fragmentes ist in SEQ ID NO: 30 gezeigt. Bei dem Konstrukt handelt es sich um einen Binärvektor der mittels Agrobakterium vermittelter oder biolistischer Transformation in das Weizengenom eingebracht werden kann. Durch die Expression der Cas9 unter der Kontrolle des starken Mais-Ubiquitinpromoters zusammen mit der sgRNA unter der Kontrolle des TaU6-Promoters kommt es zur Bindung der Cas9 Nuklease an das zweite Exon des TaTDF-Gens und nachfolgend zur Bildung eines Doppelstrangbruches. Dadurch werden endogene Reparaturmechanismen induziert, die bei Vorlage eines entsprechenden Reparaturtemplates zu einer Integration von DNA-Sequenzen in den anvisierten Bereich führen kann (Shukla et al., 2009).

Somit ist es notwendig, sowohl die Expressionskassette für das CRISPR/Cas-System aus dem Plasmid pDest_ubiCAS9_ChimeraTaU6-TaTDF als auch das Reparaturtemplate aus dem Plasmid pAMK-TITaTDF-70SubiintronP450wheat zusammen in eine Zelle des Weizen zu transformieren und aus dieser Zelle wieder eine ganze Pflanzen zu regenerieren.

Am effektivsten wird dies durch die Methode der klassischen Transformation von unreifen Weizenembryonen erreicht, wobei Agrobakterienstämme miteinander gemischt werden, die entweder das Plasmid pDest_ubiCAS9_ChimeraTaU6-TaTDF oder aber einen Binärvektor enthalten, indem das Reparaturtemplate aus dem Konstrukt pAMK-TITaTDF-70SubiintronP450wheat zwischen die Left Border- und Right Border-Sites kloniert wurde.

Eine weitere Transformationsmöglichkeit besteht, indem die aufgereinigten Plasmide pDest_ubiCAS9_ChimeraTaU6-TaTDF und pAMK-TITaTDF-70SubiintronP450wheat zusammen auf Goldpartikel präzipitiert werden und diese Goldpartikel für eine biolistische Transformation von unreifen Weizenembryonen verwendet werden.

Nach Transformation und Regeneration von Weizenpflanzen können diese mittels klassischer molekularbiologischer Methoden auf die gezielte Integration der Expressionskassette aus dem Plasmid pAMK-TITaTDF-70SubiintronP450wheat in das zweite Exon des TaTDF-Gens untersucht werden. Dazu bieten sich PCR-basierte Methoden an. Die erfolgreiche Insertion/Integration des P450 Transgens kann zum Beispiel durch ein PCR-Assay bestimmt werden, bei der ein Primer für die PCR Reaktion innerhalb der Expressionskassette für das Herbizidtoleranz-Gen bindet, die im Plasmid pAMK-TITaTDF-70SubiintronP450wheat beschrieben ist. Die Primerbindung muss jedoch außerhalb der für die gerichtete Integration notwendigen homologen Sequenzen aus dem TaTDF-Locus erfolgen. Als Gegenprimer muss dann eine Sequenz im Bereich des TaTDF-Locus genutzt werden, die außerhalb des homologen Bereiches des TaTDF-Gens bindet, welches für die gerichtete Integration verwendet wurde. Als Beispiel für solche Primersequenzen können die unten aufgeführten Primer nosTxxxf01 und TaTDF13 angesehen werden. Der Primer nosTxxxf01 bindet Im Bereich des nos-Terminators der Herbizidtoleranzkassette, wohingegen der Primer TaTDF13 im 3. Exon des TaTDF-Gens auf dem Chromosom 4DL bindet. Bei erfolgreicher, gerichteter Integration der Herbbizidtoleranzkassette in das TaTDF-Gen auf dem Chromosom 4DL würde eine PCR Reaktion mit den beiden Primern nosTxxxf01 und TaTDF13 ein PCR Fragment von 1135 bp ergeben.

| **Name** | **Oligo Sequenz** | **SEQ ID NO:** | **Länge** |
|---|---|---|---|
| nosTxxxf01 | GAATTTCCCCGATCGTTCAA | 37 | 20 |
| TaTDF13 | CTGTGTGGTATTTGACGTCGTCG | 38 | 23 |

Durch die Integration der Expressionskassette bestehend aus dem konstitutiven Promoter d35S zusammen mit dem ersten Intron des Mais-Polyubiquitingens (Christensen et al., 1992), der codierenden Sequenz des P450-gens aus *Cynodon dactylon* sowie der Terminationssequenz des Nopalinsynthasegens aus *Agrobakterium tumefaciens* werden zwei neue Eigenschaften in dem transgenen Weizen eingefügt. Zum einen wird, wie in Figur 2 gezeigt, eine Toleranz gegenüber dem Herbizid Nicosulfuron erreicht. Zum anderen wird durch die Einfügung von zusätzlicher DNA-Sequenz in die codierende Sequenz des TaTDF-Gens dieses mutiert und ist nicht mehr funktional. Im homozygoten Zustand führt dies dazu, dass kein funktionales TaTDF-Protein gebildet werden kann. Das TaTDF-Protein ist jedoch essentiell für die männliche Fertilität der Weizenpflanze, so dass als zweite Eigenschaft durch die gezielte Integration der Herbizidtoleranzkassette eine männliche Sterilität erzeugt wird.

Die Insertion des Herbizidtoleranz-Gens kann in einem beliebigen Subgenom des Weizen erfolgen. Pflanzen, bei denen die TDF-Gene sämtlicher Subgenome (A, B, D) inaktiviert sind, sind männlich steril.

### 3.2. Herstellen einer männlich sterilen Pflanze mit Herbizidtoleranz

Pflanzen, in denen sämtliche TDF-Gene/-Alle inaktiviert sind und bei denen mindestens ein TDF-Allel durch gezielte Insertion eines Herbizidtoleranz-Gens wie in 3.1. beschrieben mutiert wurde, sind also sowohl männlich steril, als auch herbizidtolerant. Dabei sind Herbizid-Toleranz und Sterilität gekoppelt. Wie in Beispiel 1.2. gezeigt, sind nur solche Pflanzen gegenüber einer hohen Dosis (2-fache Konzentration, ausgehend von der vom Hersteller empfohlenen Dosis) tolerant, welche das Herbizidtoleranz-Gen homozygot tragen. Liegt das Herbizidtoleranz-Gen hemizygot vor, überlebt die transgene Pflanze die Behandlung mit der hohen Dosis Herbizid nicht, ist aber gegenüber einer niedrigen Dosis des Herbizids (0,5-fache Konzentration, ausgehend von der vom Hersteller empfohlenen Dosis) tolerant (siehe Figur 17).

Pflanzen, in denen die TDF-Gene der übrigen Sugenome mutiert sind (derjenigen Subgenome, in denen nicht gezielt ein Herbizidtoleranz-Gen eingefügt wurde), können zum Beispiel erzeugt werden, indem die gemäß Beispiel 3.1. hergestellte Pflanze mit männlich sterilen Pflanzen gekreuzt wird, deren Herstellung in Beispiel 2.7. beschrieben wird.

### 3.3. Herstellen von Hybridsamen

Zur Herstellung von Hybridsamen werden zunächst die gemäß Beispiel 3.2. hergestellten Pflanzen mit hohen Dosen des Herbizids Nicosulfuron behandelt (zum Beispiel 2-fache Aufwandsmenge), um Pflanzen zu selektieren, in denen das Herbizidresistenz-Gen homozygot vorliegt. Die selektierten Pflanzen sind gleichzeitig herbizidtolerant und männlich steril (weiblich) (s. Figur 15). Alternativ kann bereits der Samen der gemäß Beispiel 3.2. hergestellten Pflanzen mit hohen Dosen des Herbizids Nicosulfuron behandelt werden; dies führt dazu, dass nur männlich sterile Pflanzen erfolgreich keimen und wachsen.

Als Samen bzw. Pflanzen zur Kreuzung mit den männlich sterilen (weiblichen) Samen bzw. Pflanzen kommen jegliche, männlich fertile (männliche) Weizenlinien in Frage. Um die Bestäubung zu vereinfachen, können Samen des weiblichen und männlichen Pools vor dem Aussäen gemischt werden.

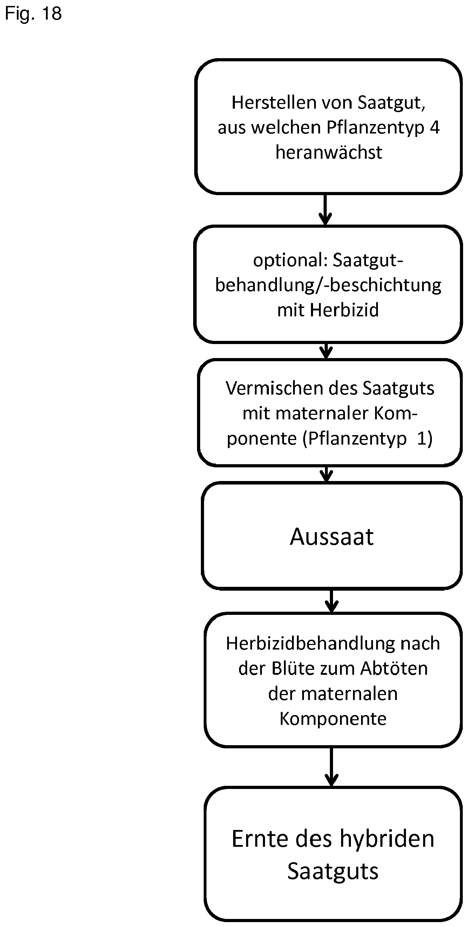

Nachdem die ausgesäten Pflanzen geblüht haben, werden sie erneut mit Herbizid besprüht, um Pflanzen der männlichen Linie (paternale Komponente) abzutöten. Damit wird vermieden, dass Samen der männlichen Pflanzenlinien geerntet werden, denn diese enthalten kein Hybridsaatgut. Nur herbizidtolerante, weibliche Pflanzen überleben, und deren Samen werden schließlich geerntet (siehe Figur 18).

### Literaturnachweis:

Abdel-Aal, E-SM, und P. Hucl. 1999 "A rapid method for quantifying total anthocyanins in blue aleurone and purple pericarp wheats." Cereal chemistry 76.3: 350-354.
Burešvá, V., Kopecký, D., Bartos, J., Martinek, P., Watanabe, N., Vyhnänek, T., & Doležel, J. (2015). Variation in genome composition of blue-aleurone wheat. Theoretical and Applied Genetics, 128(2), 273-282.
Chen Let al. (2010). Isolation and heterologous transformation analysis of a pollen-specific promoter from wheat (Triticum aestivum L.). Molecular biology reports, 37(2), 737-744.
Christensen AH., Sharrock RA and Quail PH, "Maize polyubiquitin genes: structure, thermal perturbation of expression and transcript splicing, and promoter activity following transfer to protoplasts by electroporation." Plant molecular biology 18.4 (1992): 675-689.
Curtin SJ, Zhang F, Sander JD, Haun WJ, Starker C, Baltes NJ, Reyon D, Dahlborg EJ, Goodwin MJ, Coffman AP, Dobbs D, Joung JK, Voytas DF, Stupar RM. (2011), "Targeted mutagenesis of duplicated genes in soybean with zinc-finger nucleases"; Plant Physiol. 156(2):466-473
Depicker A, Stachel S, Dhaese P, Zambryski P, & Goodman HM (1981). Nopaline synthase: transcript mapping and DNA sequence. Journal of molecular and applied genetics, 1(6), 561-573.
Ellis, M., Spielmeyer, W., Gale, K., Rebetzke, G., & Richards, R. (2002). " Perfect" markers for the Rht-B1b and Rht-D1 b dwarfing genes in wheat. Theoretical and Applied Genetics, 105(6-7), 1038-1042.
Fujita M, Horiuchi Y, Ueda Y, Mizuta Y, Kubo T, Yano K, Yamaki S, Tsuda K, Nagata T, Niihama M, Kato H, Kikuchi S, Hamada K, Mochizuki T, Ishimizu T, Iwai H, Tsutsumi N, Kurata N.(2010) "Rice expression atlas in reproductive development"; Plant Cell Physiol. 51(12): 2060-2081
Gurr SJ, & Rushton PJ (2005). Engineering plants with increased disease resistance: what are we going to express? TRENDS in Biotechnology, 23(6), 275-282.
Jimenez F, et al. 2016 "Physiological, biochemical and molecular characterization of an induced mutation conferring imidazolinone resistance in wheat." Physiologia plantarum*.*
Kempe K and Gils M (2011), "Pollination control technologies for hybrid breeding"; Mol. Breeding 27, 417-437
Odell JT, Nagy F, & Chua NH (1985). Identification of DNA sequences required for activity of the cauliflower mosaic virus 35S promoter. Nature 313 (1985): 810-2.
Sambrook J, & Russell DW (2001). Molecular cloning: a laboratory manual (3-volume set) (Vol. 999). Cold Spring Harbor, New York:: Cold spring harbor laboratory press.
Shukla, Vipula K., et al. (2009) "Precise genome modification in the crop species Zea mays using zinc-finger nucleases." Nature 459.7245: 437-441.
Twell D, Yamaguchi J, Wing RA, Ushiba J, & McCormick S (1991). Promoter analysis of genes that are coordinately expressed during pollen development reveals pollen-specific enhancer sequences and shared regulatory elements. Genes & development, 5(3), 496-507.
Venter M (2007). Synthetic promoters: genetic control through cis engineering. Trends in plant science, 12(3), 118-124.
Whitford R, Fleury D, Reif JC, Garcia M, Okada T, Korzun V, Langridge P.(2013), "Hybrid breeding in wheat: technologies to improve hybrid wheat seed production"; J Exp Bot. 64(18):5411-5428
Wilson, ZA and Zhang, D (2009), "From Arabidopsis to rice: pathways in pollen development"; J Exp Bot. 60(5):1479-1492
Zhao Y, Zhao Q, Ao G, & Yu J (2006). Characterization and functional analysis of a pollen-specific gene st901 in Solanum tuberosum. Planta, 224(2), 405-412.
Zhou KJ, Wang SH, Feng YQ, Liu ZX, Wang GX. (2006), "The 4E-ms system of producing hybrid wheat"; Crop Science 46, 250-255

## Patentansprüche

1. Pflanze der Gattung *Triticum,* **dadurch gekennzeichnet, dass** ein Markergen in mindestens ein Allel des *tapetal development and function* (TDF) Gens eines Subgenoms der Pflanze insertiert ist und das Allel inaktiviert ist.

2. Pflanze nach Anspruch 1, **dadurch gekennzeichnet, dass** die Expression des Markergens zu einem unterschiedlichen Phänotyp führt, abhängig davon, ob das Markergen in dem Subgenom homozygot oder hemizygot vorliegt.

3. Pflanze nach Anspruch 1 oder 2, **dadurch gekennzeichnet, dass** das Markergen ausgewählt ist aus einem Herbizidtoleranz-Gen, einem Farbmarker-Gen und einem phänotypischen Markergen.

4. Pflanze nach einem der Ansprüche 1-3, **dadurch gekennzeichnet, dass** die Allele des TDF-Gens der übrigen beiden Subgenome inaktiviert sind.

5. Pflanze nach einem der vorangehenden Ansprüche, **dadurch gekennzeichnet, dass** das für *tapetal development and function* (TDF) codierende Gen eine Nukleotidsequenz umfasst, ausgewählt aus den folgenden:
(a) Nukleotidsequenz der SEQ ID NO:1, 2 oder 3 oder SEQ ID NO: 7, 8 oder 9;
(b) Nukleotidsequenz, die für ein Protein enthaltend eine der Aminosäuresequenzen der SEQ ID NO: 10, 11 oder 12 oder eine Aminosäuresequenz, die eine Identität von mindestens 80 % zu einer der Aminosäuresequenzen der SEQ ID NO: 10, 11 oder 12 aufweist, codiert;
(c) Nukleotidsequenz, die komplementär ist zu (a) oder (b);
(d) Nukleotidsequenz, die homolog ist zu (a) oder (b), wobei die Nukleotidsequenz für ein TDF-Protein codiert;
(e) Nukleotidsequenz, die unter stringenten Bedingungen mit (a) oder (b) hybridisiert; und
(f) Nukleotidsequenz, die unter stringenten Bedingungen mit (c) hybridisiert, wobei die Nukleotidsequenz für ein TDF-Protein codiert.

6. Pflanze nach einem der vorangehenden Ansprüche, **dadurch gekennzeichnet, dass** die Insertion des Markergens in Exon 2 des TDF-Gens liegt.

7. Pflanze nach einem der Ansprüche 4-6, **dadurch gekennzeichnet, dass** die inaktivierten TDF-Allele der beiden übrigen Subgenome jeweils eine oder mehrere Mutation(en) enthalten, welche vorzugsweise transgen oder nicht-transgen erzeugt wurden.

8. Pflanze nach Anspruch 7, **dadurch gekennzeichnet, dass** die Mutationen in den Allelen der übrigen Subgenome in demselben Exon des TDF-Gens liegen, vorzugsweise in Exon 2.

9. Pflanze nach Anspruch 7 oder 8, **dadurch gekennzeichnet, dass** die Mutationen im Exon 2 in einer Region zwischen den Nukleotidpositionen 256 und 286 der SEQ ID NO: 3 (Referenzsequenz) liegen, bevorzugt:
(a) zwischen den Nukleotidpositionen 256 und 285 der SEQ ID NO: 3,
(b) zwischen den Nukleotidpositionen 258 und 285 der SEQ ID NO: 3,
(c) zwischen den Nukleotidpositionen 281 und 286 der SEQ ID NO: 3,
(d) zwischen den Nukleotidpositionen 281 und 283 der SEQ ID NO: 3; oder
(e) an der Nukleotidposition 282 der SEQ ID NO: 3.

10. Pflanze nach einem der Ansprüche 7 bis 9, **dadurch gekennzeichnet, dass** die Mutationen Deletionen sind, vorzugsweise Deletionen von 1 bis 28 Nukleotiden.

11. Teil oder Samen einer Pflanze nach einem der Ansprüche 1 bis 10.

12. Nachkommen einer Pflanze nach einem der Ansprüche 1 bis 10, **dadurch gekennzeichnet, dass** ein Markergen in mindestens ein Allel des TDF-Gens eines Subgenoms der Pflanze insertiert ist und das Allel inaktiviert ist.

13. Nachkommenpflanze gemäß Anspruch 12, oder ein Teil oder Samen davon, wobei die Nachkommenpflanze eine Hybridpflanze der Gattung *Triticum* ist.

14. Verfahren zur Selektion einer männlich sterilen Pflanze der Gattung *Triticum,* umfassend die folgenden Schritte:
(i) Herstellen einer Pflanze nach Anspruch 4, wobei das Markergen ein Herbizidtoleranz-Gen ist;
(ii) Behandeln der Pflanze oder ihrer Samen mit einem geeigneten Herbizid in einer Konzentration, welche solche Pflanzen tötet, in denen das Herbizidtoleranz-Gen in nur einem Allel des einen Subgenoms vorliegt; und
(iii) Identifizieren der Pflanzen, welche die Behandlung mit dem Herbizid überlebt haben.

15. Verfahren zum Herstellen einer Pflanze der Gattung *Triticum,* **dadurch gekennzeichnet, dass** ein Markergen in mindestens ein Allel des TDF-Gens eines Subgenoms der Pflanze inseriert wird und das Allel dadurch inaktiviert wird.


SEQUENCE LISTING

<110>  KWS SAAT AG

<120>  Pflanze der Gattung Triticum, in der das TDF-Gen durch ein Markergen
inaktiviert ist

<130>  KWS0257EP

<140> EP not yet assigned
<141> yyyy-mm-dd

<160>  38

<170>  PatentIn version 3.5

<210>  1
<211>  2611
<212>  DNA
<213>  Triticum aestivum

<400>  1
atggggcggc cgccgtgctg cgacaaggcc aacgtgaaga aggggccgtg gacggcggag      60

gaggacgcca agctgctggc ctacacctcc aaccatggca ccggcaactg gacctctgtt     120

ccccagaggg caggtcggaa ccctctcccc cggccggccg gatcggcgtc tggcatcgaa     180

atggtgttgg ttttgcatgg tttctggctg atgtgtgttg cgtcatggat gggtgcgtgc     240

tgcagggctg aagcggtgcg ggaagagctg caggctgagg tacaccaact acctgaggcc     300

caacctcaag cacgagaact tcacgcagga ggaggaggag ctcatcgtca ccctccatgc     360

catgctggga agcaggtgca tctctgtaca tgcatgtcat ccatcggtta ctttctcgat     420

tttgtcatca agttttcggt gaatgcgaaa ctttgctatg gacagactac tgtttctgtc     480

atcattcctg ctataaactt tgcatccata tactctagcg tacgttcaga tcttatagat     540

ttatggaagt tctaaaaacc tcacgttggt gtatcattca gacaattgct catacaccgg     600

aaatcacgtc ctacttctat tcaagaattt actttagccg ttcattcata aagtagaaac     660

aaatgtagtt taatccagta accagtctcg tgtctacggt tcttctgaat atattctgtt     720

tcctggtaga atgtagtgaa aagctgaata ttgggtgaag aacagatctg aatagctgtc     780

ttaaattgat agtgtttgac agaaaatact ccacactgag cgtaatataa catgaaagta     840

cgtgtacgtg ttgggtgcat gcaggtggtc tctgatcgcg aaccagctgc cggggcggac     900

ggacaacgac gtcaagaatt actggaacac caagctgagc aagaagctgc ggcagcgggg     960

catcgacccc atcacccatc gccccatcgc cgacctcatg cagagcatcg gcaccctccc    1020

catccgcccg ccgcccagcg ccgcgggtgc ctcctcgtcc tcctacatcc ccgtgaaccc    1080

agcggcggcg ccggggctcc agccgctgca cgacgacgtc aaataccacg cagtcctgaa    1140

ccaccaccag cagcaggtca tcacgctcct cgaccccgac gcgccagggg cggcggcgtc    1200

cccggaccac cagctcaagt ggagcgactt ccttgccgac gacgccgccg ccttcgaggc    1260

ggcgccgcag gtggttcttg gtcagtacca ggaggccgcg gtggctggtg gcggagcagc 1320

gtatggagac actgatagta ttgcagccga tggtgtcggc gggggcgggg aggatagcgc 1380

agcgtcagcg ttcatcgacg cgatgctgga cagcgacaaa aagatgggcg tggaccagct 1440

catcgccgac ctgctcgccg acccggcata ctactacggc ggaggctctt cctcttcgac 1500

gtcggagctg gggcggggcg gttgatcatt tatttaacta ctagaggtta catatcttac 1560

attgggacgg tatttaagca tatttagggc atcacatttt cgtttgaact ttttttttgc 1620

gtgaaaactt tcgatctatt tatcaactgt caaggtagta caaagaacac tagaagtaaa 1680

atttacatcc aggtccgtag accacctagc gacgactaca atcactagag cgagccgaag 1740

gcgcgccgcc gtcatcgccc ctccctcgtc ggagccgggc aaacattgtt gtagtagaca 1800

gtcgggaagt cgtcgtgcta ccagcgcacc agaacaacaa ccgccgccga tgaagagaag 1860

catagaccgg aaggatccaa cctgcagaca ctcacacata gacgaacaaa gaccagatcc 1920

agttggatcc accaaagaca aacatcgacc ggatcccacg agatccgccg gagacaaacc 1980

tccacacgcc tttcgatgat gcttgaaaca tcaccgggac aggggctagg cggggaggac 2040

cttattccat cttcaagaag acgccgccgc ctcatcttct tgagcatggc acaaacccta 2100

acaaaactca gaaaagcatc taaaaacgaa gccctcccgc cggcaagggc cgggatccac 2160

cgtgcctcca tgatccgagg accataaaag ataaggcgga ccagcggcgc cgacgagagg 2220

caaggaaacc ctagatagga gttcgggcgt gggcttctgt tttcacctcc gatttagttg 2280

cctcataaca aattggttta gcacgtagtt acattttcgt tttaacttgt tattggtatt 2340

ctattcccac cccgatcaat gatgagctga ttaaaagtgc gtttttgctt tttaagaaat 2400

ctagtcaata tgtatgtgta atgcttaaat taaaagtata ttaagtgcat gcggatatta 2460

agtatgatat tatttttgtg ttattatgtg attagtatta ttttttagca tgaaattaac 2520

tgcacgctaa acatgttgag cgctcgacat tgaagcagtc taggtcgttg gattgacatg 2580

atttgatggg cgagattaat tgaatttgcc t 2611

<210> 2
<211> 2226
<212> DNA
<213> Triticum aestivum


<220>
<221> misc_feature
<222> (1227)..(1229)
<223> n is a, c, g, or t

<400> 2
atggggcggc cgccgtgctg cgacaaggcg aacgtgaaga aggggccgtg gacggcggag 60

gaggacgcca agctgctcgc ctacacctcc aaccatggca ccggcaactg gacctctgtt 120

ccccagaggg caggtcggaa ccctctcccc cggccggccg gatccacgtc tggcatcgaa 180

atggtgttgg ttttgcatgg tttctggctg atgtgtgttg cgtcatggat gggtgcgtgc 240

tgcagggctg aagcggtgcg ggaagagctg caggctgagg tacaccaact acctgaggcc 300

caatctcaag cacgagaact tcacgcagga ggaggaggag ctcatcgtca ccctccatgc 360

catgctggga agcaggtgcg tctctctgtg tagtacctgt acatgcatgt catccatcgc 420

ttactttctc gattttgtca tcaagttttc ggtgaatgcg aaactctgct gctactggtt 480

ctgtcatcat tcctgccata tataaacttt gcaaccatat actctagcgt tcagatctta 540

tagatttatg gaagttctga aaatctcacg ttggtttatg acagttgatc atataccgga 600

aatcacgtcc tacttctatt caagaattta cttcagtcgt tcattcaaat caaaaaatag 660

aaacaaatgt agtttaatgc agtgaccagt ctcgtgtcta cggttcttct gaatatattc 720

tgtttcctgg gtagaatgta cagtagtgaa aagctgaaaa ttcggtgaag aacggatctg 780

aatagctgtc ttaaattgat agtgtttgtc agagaatact ccacacctga gcgtaacatg 840

aaagtacgtg tacgtgttgg gtgcatgcag gtggtctctg atcgcgaacc agctgccggg 900

gcggacggac aacgacgtca agaactactg gaacaccaag ctgagcaaga agctgcggca 960

gcggggcatc gaccccatca cccaccgccc catcgccgac ctcatgcaga gcatcggcac 1020

cctcgccatc cgcccgccac cgagcgccgc gggtgcctcc tcctcctcct acctccccgt 1080

gaacccagcg gcggcgccgg ggctccagcc gctgcacgac gacgtcaaat accacgcagt 1140

cctgaaccag cagcagcagc aggtcatcac gctcctcgac gccgacgcgc caggggcggc 1200

ggcgtccccg gaccacccgc tcaagtnnng ccgacgacgc cgccgccttc gaggcggcgc 1260

cgcaggtcgt tcttggtcag taccaggatg ccgcggtcgc tggtggcgga gcacacgcgt 1320

atggcgacac tgacagtact gcagccgatg gtgtcggcgt gggcggggag gatagcgcag 1380

cgtcagcgtt catcgacgcg atgctggaca gcgacaagaa gatgggcgtg gaccagctca 1440

tcgccgacct gctcgccgac ccggcatact actacggcgg gggctcttcc tcttcgacgt 1500

cggatctggg gtggggctgt tgatcattta tttaactaga ggttacatat cttacattgg 1560

gacggcattt aaacatattt agggcatcac attttcgttt gaacttgtta ttggtactcc 1620

atgaaccccg atcgataatg agctgattaa gcgtgcgttt ttgtttttaa aaaatgatta 1680

agcgtgagct gatttgatgt atttgtggca atcgaacggg tagatggtgt aggatttact 1740

ttttgataag ttcctttggc cggctgatcc ttccagcatt tacacggcga agtcggttta 1800

catgaggttg tgtatggtat gggtcttgag cgatctccca cagacgtgtg catttggagg 1860

agttaggcat ctttgaagtg caaggtcttt gcgtggttga caaccaaata tcgcgtgtgg 1920

acgtccgacc aacgtgtgac acgggctgca ggacgagccg tcaccctgct acctttgcct 1980

atagaaaaaa agacaatgtg gatcacattt tcggacattg tgtctatacg agggaagttt 2040

cgcatgggtg ttgtgagagc aactctagca gaccccgtat ccatccccac ccggaaaata 2100

acccccaaaa tgcgggtagg ggcggaaaaa cctgcccgat cagaccccgc atatcgcccc 2160

ggccctcaat tttttttaag gggcgcggca aaatctcggc cccaacccgc gaatacacgg 2220

gtttcc 2226

<210> 3
<211> 1992
<212> DNA
<213> Triticum aestivum

<400> 3
atggggcggc cgccgtgctg cgacaaggcc aacgtgaaga aggggccgtg gacggcggag 60

gaggacgcca agctgctcgc ctacacctcc aaccatggca ccggcaactg gacctctgtt 120

ccccagaggg caggtcggaa ccctcccccc ggccggccgg atcgacgtcc gtcatggaaa 180

tggtgttggt cttgcatggt ttctggctga tgtgtgttgc atcatggatg cgtgcgtgct 240

gcagggctga agcggtgcgg gaagagctgc aggctgaggt acaccaacta cctgaggccc 300

aacctcaagc acgagaactt cacgcaggag gaggaggagc tcatcgtcac cctccatgcc 360

atgctgggaa gcaggtgcgt ctctctgtgt agtacatgca tgtttctgcc atcagttact 420

ttctcgattt tgtcatcaag ttttcggtga atgcgaaact ttgctatgga ccgactgctg 480

tttctgtcat cattcctgct ataaaccttg catccatata ctctagcgtt cagatcttat 540

agatttatgg aagttctgaa tatctcacgt tggtgtatca gacaattgct catataccgg 600

aaatcacgtc ctacttctat tcaagaattt acttcagtcg ttcgttcaaa aatagaaaga 660

aatgtagttt agtccagtga ccagtctcgt gtctacgtac ggttcttctg aatatatata 720

ttctgtttcc tggtagaatg tagtgaaaag ctgaaaattc ggtgaagaac atatctgaat 780

agctgtctta aattgatagt gtttgacaga aaatactcca cactgagcgt aacgtgaaag 840

tgcgtgtacg tgttgggtgc atgcaggtgg tctctgatcg cgaaccagct gccggggcgg 900

acggacaacg acgtcaagaa ctactggaac accaagctga gcaagaagct gaggcagcgg 960

ggcatcgacc ccatcaccca ccgccccatc gccgatctca tgcagagcat cggcaccctc 1020

gccatccgcc cgccaccgag cgccgcgggt gcctcctcct cctcctacct ccccgtgaac 1080

ccaccggcgg cgccggggct ccagccgctg cacgacgacg tcaaatacca cacagtcctg 1140

aaccagcagc agcagcaggt catcacgctc ctcgaccccg acgcgccagg ggcggcggcg 1200

tccccggagc accagctcaa gtggagcgac ttcctcgcgg acgacgccgc ggccctcgag 1260

gcggcgccgc aggtcgttct tggtcagtac caggaggccg cggtcgctgg tggcggagca 1320

cacgcgtatg gcgacactga cagtactgca gccgatggtg tcggcggggg cggggacgat 1380

agcgcagcgt cagcgttcat cgacgcgatg ctggacagcg acaagaagat gggcgtggac 1440

cagctcatcg ccgacctgct cgccgacccg gcatactact acggcggagg ctcttcctct 1500

tcgacgtcgg agctggggtg gggctgttga tcatttattt aactagaggt tacatatctt 1560

tacattggga cggcatttaa gcatatttag ggcatcacat tttcgtttga acttgttatt 1620

ggtattcgct gaaccccgat cgatgatgag ctattaagcg tgcgtttttg tttttttaag 1680

aaaatgatta agcgtgagct gatttgatgt ctttgtggca aacgaacggg tagatggtgt 1740

aggagtactt tttcgagaat gctaatttcc agtcggcagg aaatctgcaa cacatccgca 1800

cactagcgaa ccacctcacc tacgttcttt tttttgtaaa aatgaaagca aataggcttt 1860

ttaacctgtc tccttttcta cgtttgcaca aaagtgaatg caatgtccga tcttttccac 1920

acttaagtag attatttttc caactcgtct cgagttgatg acaccatggt aatttttgcg 1980

cggggcgggg gg 1992

<210> 4
<211> 1686
<212> DNA
<213> Triticum aestivum

<220>
<221> misc_feature
<222> (431)..(531)
<223> n is a, c, g, or t

<220>
<221> misc_feature
<222> (666)..(756)
<223> n is a, c, g, or t

<400> 4
taagtatttc ccttagtttt tgagaaccaa ggtatcaatc tagtaggagg aaatacgcaa 60

gtccctaata cctacacaaa caaacaagaa cctcgcaacc aacgcgataa aggggttgtc 120

aatcccttca cggtcactca cgaaaacgag atctgataga gataataaga taaatatttt 180

tggtattttt attgtataga ttggaaaata aagattgcaa aataaagaac gagagaaata 240

gcaagttgat aggaaaataa tatgatggaa gatagacccg ggagccatag gtttcaccag 300

tggcttctct atagaaaatt ctacggtggg tgaacaaatt actgtcgagc aattgataga 360

agttatgaga atatctaggc atgatcatgt atataggcat cacgtccgtg acaagtagat 420

cgaaacgatt nnnnnnnnnn nnnnnnnnnn nnnnnnnnnn nnnnnnnnnn nnnnnnnnnn 480

nnnnnnnnnn nnnnnnnnnn nnnnnnnnnn nnnnnnnnnn nnnnnnnnnn ntttccttta 540

cacgaagctt tttacctgac gaaccataag aacaagtagt acaaactgtt aacaccacac 600

cagaaatgct cggtgctttt tctctgaaac caacacgaga cgctgaaaaa ggcattcaaa 660

aaaaannnnn nnnnnnnnnn nnnnnnnnnn nnnnnnnnnn nnnnnnnnnn nnnnnnnnnn 720

nnnnnnnnnn nnnnnnnnnn nnnnnnnnnn nnnnnngatt tgatatttct atctgtttat 780

tttcctggat tgaacttcgt ttcttttttc tcttcgcgaa tggcttactc tactttctgt 840

tctcctccaa atattgatca tctacacact ttcctttaca cgaagctttt tacctgacga 900

accataagaa caagtagtac aaactgttaa cgccacacca gaaatgctcg gtgcttcttc 960

tctgaaacca acacgagacg ctgaaaaagg cattaaaaaa aacacgagac gctgaaaaca 1020

gcatgcattt tctccttgta ataccacaac tgttaaggat cacatgtgcg gcgccaacac 1080

aactgtcaag acgatcgtac aaacgcgatg agcagaggcg cctgtgatat gatcgcatcg 1140

ccagctccca ggtgaaaacc gattccgcgg cgctgatgtc gtgaaaatca tcatgtccaa 1200

ctcgtgaaaa tatttgaaac agcccccggc ccctccaccc tcacacacgg cgtttgatta 1260

gattagatcg gattgtatta acccaccaac ccgcatctgc atacatattt gatccctccc 1320

tgattaagcg gttgatttgc cggagggaaa cagttgcagg cgtgccataa actagggtta 1380

aagaagcgtt tccatcggat cgtcactaac ccatgccatg ccatcactag ttaaatgagg 1440

agggggaggg tcatgcttcc gtcgcgtgcc cctccaccat ctggacatgt tgttgccgtg 1500

cagtgcccct ctctctctcg ccggagcgcc ccgcccccta tatgtacgcc ggtgccaccc 1560

tgcagtgtga tctcgtcggg gggcaccgga ggttggtgcg gtgctggggt tctcgagcta 1620

tattatctat caccgtttgg ctgcttgttg tagaagggga gggggaggag ggtggtgccg 1680

gccggg 1686

<210> 5
<211> 1712
<212> DNA
<213> Triticum aestivum

<400> 5
gccgccgccg ccgccgttag tagcacccga catggccacg ccgatcttgg acgccatgct 60

gctgagaaac ccgccctcct cagccaccgc cacctctgcc ttcccctcca ccgcacgacc 120

agcgccggcc tcctgctctt cctctcgtcc gcgcttcttc ccctgcgcct cctgctccct 180

cctctccgcc tcgcccaccg aatcctccat cacgaaaaag tgcgtgtgct actgctgcag 240

gaaaggcagg aacagatcag gtaatatctt ccaggagccg tgccgcagga gggatatata 300

ggcggctggc gggcacgcgt cctgcggagg tgcccaccat ggcggcccct cgcgtgcttg 360

gcctggctcc gcgtagcctg gcgcgcggtg attggctgga cgttgccgcg atatttttaa 420

ggtcgctttg tggattgcgc ttttgttttt ccgcttctcg attttgtgca cgcaaaactt 480

ccgggcgggg cccggaggca gcgacgtgcc cacggcgcgg tggccagtgg atcgcagggg 540

ttgtctgtgg ggatgcccgg gacggcgagt gccgaaacta tactttactc catgggaatt 600

gagcgtcggg gtcggtcggt cttcacgtgt cccgggaatg ggaagtgcaa ctctgtgagc 660

aacgggcggg tgggggtgct gcatctgagg ctcgtaatgc tggtgccatg tctatccaca 720

cgagagaagc tgcagcgtac agtgtgaagc ctgataggtt ggttgcgatg cttagcgttg 780

gaagagaaac tactactagt accgtacggg tctacgttag acacaaaaat cagagtggcg 840

cagcggaagc gtggtgggcc cataacccac aggtcccagg atcgaaacct ggctctgata 900

tgattatttt tttgaatttt tcttgattag atttgatact tctatctgtt tcttttccct 960

ggattgagct tcgttttttt ccctctctgc ccgaatggct tgctcggtgc ttcttcttcc 1020

ttcttctcta aaaccaacac gagacgctga gacagcatgc atgcattttc tcctcgtaat 1080

actacaaact gtcaaggatc acatgtgcgg cgccaacaca actgtcaaga cgatcgtaca 1140

aacgcgatga gcagaggcgc ctgcgatatg atcgcatcgc cagctcccag gtgaaaaccg 1200

attccgcggc gctgatgtcg tgaaaatcat catgtacaac tcgtgaaaat atttgaaaca 1260

gcccccggcc ctccaccctc acacacagcg tttgattaga ttagattaga ttaacccacc 1320

aacccgcatc tgaatatttg atccctccct gattaagctg ttgatttgcc ggagggaaac 1380

agttgcaggc gtgccataaa ctagggttaa agaagcgttt ccatcggatc gtcactaacc 1440

catgccatca ctagttaaat gaggaggggg gaggtcatgc ttccgtcgcg tgcccctcca 1500

ccatctggac atgttgttgc cgtgccccca tctctctcgc cggagcgccc cggcccctta 1560

tatgtacacc ggtgccaccc tgcagtgtgc gatctcgtcg gggggcaccg gaggttggtg 1620

cagtgctggt tctcgagcta tacatatata tatatctatc accgtttggc tgcttgtaga 1680

aggggagggg aaggagggtg gcgccggcct gg 1712

<210> 6
<211> 2743
<212> DNA
<213> Triticum aestivum

<400> 6
tgcttcagag aagtcgcttt aacatatgtg tcttgtatct gtgtctgttg tccattgttt 60

ggaatggttt cagaggactc tatttgactt tttacttgtg cttacagttt gcataccaca 120

gcagtgactt caaacctgtc cggtcttgta gcctagtagg ctgacaaagt aataatagca 180

ttattatatt tcctcctttc cggtttagta ggctcatctc attttcttgg taatccttct 240

tttctaaaaa aatataattt cttggttttt ccatattata attcttattt cacttctttc 300

caccacatgt tcacgttcca aggagtagca actataataa gcgaggagga ccagttctca 360

gtcaatgcgg ttacaggcca gaacacttat caaggtgcaa acatgtgtta caacatcagc 420

atcaaggaag ggggcacgct gggcgatgta aaactacacc caggaataac aagtgccaaa 480

tctatctact gctagtctag tcgtcgatga tggagatgag cagggaggcc tcctcagcat 540

cgggcccccg agtgtctgca gtgaggcaaa aaaaacagag aagaacaaat cctgtcagct 600

gaaacaatcg aggcgacaca cgaggatatg gtggaactga aaatagcaga ttggcagtgc 660

acctgatgtg gggaggttgg acatgatctg cttaacgatc ccaccgccgt tcggctcgtc 720

gcctcccatc tcctccttgt cctcaccctc acctttactg tgatcaacat cttccccgct 780

gccgccgtga gtcccattgc cggacatggc catgccaatc ttggaagcca tggcgcttag 840

tatcccggcc tgctcgccgc cttcgtcctc gtctttccct ccccttctct cttcctctgt 900

tccacggaag actgtcaatt acagtaaagt ttcagagagt gggcgacaaa gattaaaagc 960

gcagtaactt acctgtgacc ggtggggaag gagacgagct ggacaggagc ttgtggaaga 1020

tgccattgcc ggccgccttc tcctcgtcgc cgtcagagac agcaagcgcg ctcccgttgc 1080

cgttgccatt gccttcgccg gtgtcctcag cgccgccgct gccgttagta gcacccgaca 1140

tggccacgcc gatcttggac gccatgctgc tgagaaaccc accctcctca gccaccgcca 1200

cctccgcctt cccctccacc acacgaccag cgccggcctc ctgctcttcc tctcgcccgc 1260

gcttcttccc ctgcgccatg gtctcctcct gctccctcct ctccgcctcg cccaccgagt 1320

cctccatcac gaacgaacta agctcaccaa agtgtgtgcg ctaccactgc aggaaaggca 1380

ggaacagatc agatagtact agtattatct tccaggagcc gtgccgcagg agggatatat 1440

aggcggctgg cggacacgcg tcctgtggag atggccacca tggcggcccc tcgcgtgctt 1500

ggcctcgatc cgcgtagcct ggcgcgcggt gattggccgg ccgttgccgc gatattttaa 1560

ggtcgctttg tgggctgcgc ttttgttttt ccgcttctcg attttgtgca cgtagaattt 1620

ccggccgggg cccggaggca gcgacgtgcc cacggcgcgg tggccggtgg atcgcagggg 1680

ttgtcggtgg gaatgcctgg gacggagagc gtccaaactg tagtagtagt actttactcc 1740

atgggaactg ggcgtcgggg tcggtcggtc ttcacgtgtc ccggggatgg gaagtgcaac 1800

tctgtgtgct gcatctgatg ctcgtattgc tggtgccatg tgtatccaca ggagagatgc 1860

tgctttggga gtctagagtg tgtgtcaagc ggcattgctc ggtgctcagc gttggaagca 1920

taagacaagt accgcacgag agatgctgtt tcttttcctg gatttaactt cgtatcttcc 1980

caagtggctt actgactcag tagtacaaaa cgttacacgc caaaccacaa aatgctcggt 2040

gcttcttctc ccttcttctc tgaaaccaac acgagacgct gagacagcat gcattttctc 2100

ctcgtaatac tgcaactgtc aagcatcaca tctccggcgc caacgcaact gtcaatacga 2160

tcgtacaaaa gcgatgagca gaggcgcctg cgatatgatc gcatcgccag ctcccaggtg 2220

aaaaccgatt ccgcggcgct gatgtcgtga aaatcatcat gtccaactcg tgaaaatatt 2280

tgaaacagcc cccggcccct ccaccctcac acacagcgtt tgattagatt agatcggatt 2340

aacccaccaa cccgcatctg aatatttgat ccctccctga ttaagcggtt gatttgccgg 2400

agggaaacag ttgcaggcgt gccataaact agggttaaag aagcgtttcc atcggatcgt 2460

cactaaccca tgcatgccat cacttgttaa atgaggaggg gggaggtcat gcttccgtcg 2520

cgtgcccctc caccatctgg acatgttgtt gccgtgcagt gcccctctct ctctcgccgg 2580

agcgccccgg cccctatatg tacaccggtg ccaccctgca gtgcgatctc gtcggggggc 2640

accgcaccgg aggttggtgc agtgcggggg ttctcgagct atatatctat caccgtttgg 2700

ctgcttgtag aaggggaggg gaaggagggt ggcgccggcc ggg 2743

<210> 7
<211> 921
<212> DNA
<213> artificial

<220>
<223> Subgenome A

<400> 7
atggggcggc cgccgtgctg cgacaaggcc aacgtgaaga aggggccgtg gacggcggag 60

gaggacgcca agctgctggc ctacacctcc aaccatggca ccggcaactg gacctctgtt 120

ccccagaggg cagggctgaa gcggtgcggg aagagctgca ggctgaggta caccaactac 180

ctgaggccca acctcaagca cgagaacttc acgcaggagg aggaggagct catcgtcacc 240

ctccatgcca tgctgggaag caggtggtct ctgatcgcga accagctgcc ggggcggacg 300

gacaacgacg tcaagaatta ctggaacacc aagctgagca agaagctgcg gcagcggggc 360

atcgacccca tcacccatcg ccccatcgcc gacctcatgc agagcatcgg caccctcccc 420

atccgcccgc cgcccagcgc cgcgggtgcc tcctcgtcct cctacatccc cgtgaaccca 480

gcggcggcgc cggggctcca gccgctgcac gacgacgtca aataccacgc agtcctgaac 540

caccaccagc agcaggtcat cacgctcctc gaccccgacg cgccaggggc ggcggcgtcc 600

ccggaccacc agctcaagtg gagcgacttc cttgccgacg acgccgccgc cttcgaggcg 660

gcgccgcagg tggttcttgg tcagtaccag gaggccgcgg tggctggtgg cggagcagcg 720

tatggagaca ctgatagtat tgcagccgat ggtgtcggcg ggggcgggga ggatagcgca 780

gcgtcagcgt tcatcgacgc gatgctggac agcgacaaaa agatgggcgt ggaccagctc 840

atcgccgacc tgctcgccga cccggcatac tactacggcg gaggctcttc ctcttcgacg 900

tcggagctgg ggcggggcgg t 921

<210> 8
<211> 912
<212> DNA
<213> artificial

<220>
<223> Subgenome B

<400> 8
atggggcggc cgccgtgctg cgacaaggcg aacgtgaaga aggggccgtg gacggcggag 60

gaggacgcca agctgctcgc ctacacctcc aaccatggca ccggcaactg gacctctgtt 120

ccccagaggg cagggctgaa gcggtgcggg aagagctgca ggctgaggta caccaactac 180

ctgaggccca atctcaagca cgagaacttc acgcaggagg aggaggagct catcgtcacc 240

ctccatgcca tgctgggaag caggtggtct ctgatcgcga accagctgcc ggggcggacg 300

gacaacgacg tcaagaacta ctggaacacc aagctgagca agaagctgcg gcagcggggc 360

atcgacccca tcacccaccg ccccatcgcc gacctcatgc agagcatcgg caccctcgcc 420

atccgcccgc caccgagcgc cgcgggtgcc tcctcctcct cctacctccc cgtgaaccca 480

gcggcggcgc cggggctcca gccgctgcac gacgacgtca aataccacgc agtcctgaac 540

cagcagcagc agcaggtcat cacgctcctc gacgccgacg cgccaggggc ggcggcgtcc 600

ccggaccacc cgctcaagtg ggccgacgac gccgccgcct tcgaggcggc gccgcaggtc 660

gttcttggtc agtaccagga tgccgcggtc gctggtggcg gagcacacgc gtatggcgac 720

actgacagta ctgcagccga tggtgtcggc gtgggcgggg aggatagcgc agcgtcagcg 780

ttcatcgacg cgatgctgga cagcgacaag aagatgggcg tggaccagct catcgccgac 840

ctgctcgccg acccggcata ctactacggc gggggctctt cctcttcgac gtcggatctg 900

gggtggggct gt 912

<210> 9
<211> 924
<212> DNA
<213> artificial

<220>
<223> Subgenome D

<400> 9
atggggcggc cgccgtgctg cgacaaggcc aacgtgaaga aggggccgtg gacggcggag 60

gaggacgcca agctgctcgc ctacacctcc aaccatggca ccggcaactg gacctctgtt 120

ccccagaggg cagggctgaa gcggtgcggg aagagctgca ggctgaggta caccaactac 180

ctgaggccca acctcaagca cgagaacttc acgcaggagg aggaggagct catcgtcacc 240

ctccatgcca tgctgggaag caggtggtct ctgatcgcga accagctgcc ggggcggacg 300

gacaacgacg tcaagaacta ctggaacacc aagctgagca agaagctgag gcagcggggc 360

atcgacccca tcacccaccg ccccatcgcc gatctcatgc agagcatcgg caccctcgcc 420

atccgcccgc caccgagcgc cgcgggtgcc tcctcctcct cctacctccc cgtgaaccca 480

ccggcggcgc cggggctcca gccgctgcac gacgacgtca aataccacac agtcctgaac 540

cagcagcagc agcaggtcat cacgctcctc gaccccgacg cgccaggggc ggcggcgtcc 600

ccggagcacc agctcaagtg gagcgacttc ctcgcggacg acgccgcggc cctcgaggcg 660

gcgccgcagg tcgttcttgg tcagtaccag gaggccgcgg tcgctggtgg cggagcacac 720

gcgtatggcg acactgacag tactgcagcc gatggtgtcg gcgggggcgg ggacgatagc 780

gcagcgtcag cgttcatcga cgcgatgctg gacagcgaca agaagatggg cgtggaccag 840

ctcatcgccg acctgctcgc cgacccggca tactactacg gcggaggctc ttcctcttcg 900

acgtcggagc tggggtgggg ctgt 924

<210> 10
<211> 307
<212> PRT
<213> Triticum aestivum

<400> 10

Met Gly Arg Pro Pro Cys Cys Asp Lys Ala Asn Val Lys Lys Gly Pro
1               5                   10                  15

Trp Thr Ala Glu Glu Asp Ala Lys Leu Leu Ala Tyr Thr Ser Asn His
            20                  25                  30

Gly Thr Gly Asn Trp Thr Ser Val Pro Gln Arg Ala Gly Leu Lys Arg
        35                  40                  45

Cys Gly Lys Ser Cys Arg Leu Arg Tyr Thr Asn Tyr Leu Arg Pro Asn
    50                  55                  60

Leu Lys His Glu Asn Phe Thr Gln Glu Glu Glu Glu Leu Ile Val Thr
65                  70                  75                  80

Leu His Ala Met Leu Gly Ser Arg Trp Ser Leu Ile Ala Asn Gln Leu
                85                  90                  95

Pro Gly Arg Thr Asp Asn Asp Val Lys Asn Tyr Trp Asn Thr Lys Leu
            100                 105                 110

Ser Lys Lys Leu Arg Gln Arg Gly Ile Asp Pro Ile Thr His Arg Pro
        115                 120                 125

Ile Ala Asp Leu Met Gln Ser Ile Gly Thr Leu Pro Ile Arg Pro Pro
    130                 135                 140

Pro Ser Ala Ala Gly Ala Ser Ser Ser Ser Tyr Ile Pro Val Asn Pro
145                 150                 155                 160

Ala Ala Ala Pro Gly Leu Gln Pro Leu His Asp Asp Val Lys Tyr His
                165                 170                 175

Ala Val Leu Asn His His Gln Gln Gln Val Ile Thr Leu Leu Asp Pro

180                 185                 190

Asp Ala Pro Gly Ala Ala Ala Ser Pro Asp His Gln Leu Lys Trp Ser
        195                 200                 205

Asp Phe Leu Ala Asp Asp Ala Ala Ala Phe Glu Ala Ala Pro Gln Val
    210                 215                 220

Val Leu Gly Gln Tyr Gln Glu Ala Ala Val Ala Gly Gly Gly Ala Ala
225                 230                 235                 240

Tyr Gly Asp Thr Asp Ser Ile Ala Ala Asp Gly Val Gly Gly Gly Gly
                245                 250                 255

Glu Asp Ser Ala Ala Ser Ala Phe Ile Asp Ala Met Leu Asp Ser Asp
            260                 265                 270

Lys Lys Met Gly Val Asp Gln Leu Ile Ala Asp Leu Leu Ala Asp Pro
        275                 280                 285

Ala Tyr Tyr Tyr Gly Gly Gly Ser Ser Ser Ser Thr Ser Glu Leu Gly
    290                 295                 300

Arg Gly Gly
305

<210>  11
<211>  304
<212>  PRT
<213>  Triticum aestivum

<400>  11

Met Gly Arg Pro Pro Cys Cys Asp Lys Ala Asn Val Lys Lys Gly Pro
1               5                   10                  15

Trp Thr Ala Glu Glu Asp Ala Lys Leu Leu Ala Tyr Thr Ser Asn His
            20                  25                  30

Gly Thr Gly Asn Trp Thr Ser Val Pro Gln Arg Ala Gly Leu Lys Arg
        35                  40                  45

Cys Gly Lys Ser Cys Arg Leu Arg Tyr Thr Asn Tyr Leu Arg Pro Asn
    50                  55                  60

Leu Lys His Glu Asn Phe Thr Gln Glu Glu Glu Glu Leu Ile Val Thr
65                  70                  75                  80

Leu His Ala Met Leu Gly Ser Arg Trp Ser Leu Ile Ala Asn Gln Leu

85                  90                  95

Pro Gly Arg Thr Asp Asn Asp Val Lys Asn Tyr Trp Asn Thr Lys Leu
            100                 105                 110

Ser Lys Lys Leu Arg Gln Arg Gly Ile Asp Pro Ile Thr His Arg Pro
        115                 120                 125

Ile Ala Asp Leu Met Gln Ser Ile Gly Thr Leu Ala Ile Arg Pro Pro
    130                 135                 140

Pro Ser Ala Ala Gly Ala Ser Ser Ser Ser Tyr Leu Pro Val Asn Pro
145                 150                 155                 160

Ala Ala Ala Pro Gly Leu Gln Pro Leu His Asp Asp Val Lys Tyr His
                165                 170                 175

Ala Val Leu Asn Gln Gln Gln Gln Gln Val Ile Thr Leu Leu Asp Ala
            180                 185                 190

Asp Ala Pro Gly Ala Ala Ala Ser Pro Asp His Pro Leu Lys Trp Ala
        195                 200                 205

Asp Asp Ala Ala Ala Phe Glu Ala Ala Pro Gln Val Val Leu Gly Gln
    210                 215                 220

Tyr Gln Asp Ala Ala Val Ala Gly Gly Gly Ala His Ala Tyr Gly Asp
225                 230                 235                 240

Thr Asp Ser Thr Ala Ala Asp Gly Val Gly Val Gly Gly Glu Asp Ser
                245                 250                 255

Ala Ala Ser Ala Phe Ile Asp Ala Met Leu Asp Ser Asp Lys Lys Met
            260                 265                 270

Gly Val Asp Gln Leu Ile Ala Asp Leu Leu Ala Asp Pro Ala Tyr Tyr
        275                 280                 285

Tyr Gly Gly Gly Ser Ser Ser Ser Thr Ser Asp Leu Gly Trp Gly Cys
    290                 295                 300

<210>  12
<211>  308
<212>  PRT
<213>  Triticum aestivum

<400>  12

Met Gly Arg Pro Pro Cys Cys Asp Lys Ala Asn Val Lys Lys Gly Pro

1                   5                   10                  15

Trp Thr Ala Glu Glu Asp Ala Lys Leu Leu Ala Tyr Thr Ser Asn His
            20                  25                  30

Gly Thr Gly Asn Trp Thr Ser Val Pro Gln Arg Ala Gly Leu Lys Arg
        35                  40                  45

Cys Gly Lys Ser Cys Arg Leu Arg Tyr Thr Asn Tyr Leu Arg Pro Asn
    50                  55                  60

Leu Lys His Glu Asn Phe Thr Gln Glu Glu Glu Glu Leu Ile Val Thr
65                  70                  75                  80

Leu His Ala Met Leu Gly Ser Arg Trp Ser Leu Ile Ala Asn Gln Leu
                85                  90                  95

Pro Gly Arg Thr Asp Asn Asp Val Lys Asn Tyr Trp Asn Thr Lys Leu
            100                 105                 110

Ser Lys Lys Leu Arg Gln Arg Gly Ile Asp Pro Ile Thr His Arg Pro
        115                 120                 125

Ile Ala Asp Leu Met Gln Ser Ile Gly Thr Leu Ala Ile Arg Pro Pro
    130                 135                 140

Pro Ser Ala Ala Gly Ala Ser Ser Ser Ser Tyr Leu Pro Val Asn Pro
145                 150                 155                 160

Pro Ala Ala Pro Gly Leu Gln Pro Leu His Asp Asp Val Lys Tyr His
                165                 170                 175

Thr Val Leu Asn Gln Gln Gln Gln Gln Val Ile Thr Leu Leu Asp Pro
            180                 185                 190

Asp Ala Pro Gly Ala Ala Ala Ser Pro Glu His Gln Leu Lys Trp Ser
        195                 200                 205

Asp Phe Leu Ala Asp Asp Ala Ala Ala Leu Glu Ala Ala Pro Gln Val
    210                 215                 220

Val Leu Gly Gln Tyr Gln Glu Ala Ala Val Ala Gly Gly Gly Ala His
225                 230                 235                 240

Ala Tyr Gly Asp Thr Asp Ser Thr Ala Ala Asp Gly Val Gly Gly Gly
                245                 250                 255

Gly Asp Asp Ser Ala Ala Ser Ala Phe Ile Asp Ala Met Leu Asp Ser
            260                 265                 270

Asp Lys Lys Met Gly Val Asp Gln Leu Ile Ala Asp Leu Leu Ala Asp
        275                 280                 285

Pro Ala Tyr Tyr Tyr Gly Gly Gly Ser Ser Ser Ser Thr Ser Glu Leu
    290                 295                 300

Gly Trp Gly Cys
305

<210>  13
<211>  24
<212>  DNA
<213>  artificial

<220>
<223>  Primer 1 TDF-4AS-1 / Subgenome A

<400>  13
atcgagaaag taaccgatgg atga                                              24

<210>  14
<211>  20
<212>  DNA
<213>  artificial

<220>
<223>  Primer 2 TDF1-1 /  Subgenome A

<400>  14
tgctgcgaca aggccaacgt                                                   20

<210>  15
<211>  20
<212>  DNA
<213>  artificial

<220>
<223>  Primer 1 TDF-4BL-1 / Subgenome B

<400>  15
gtgtgttgcg tcatggatgg                                                   20

<210>  16
<211>  23
<212>  DNA
<213>  artificial

<220>
<223>  Primer 2 TDF-4DL-1 /  Subgenome B

<400>  16
atgcatgtac tacacagaga gac                                               23

<210>  17
<211>  23
<212>  DNA
<213>  artificial

<220>
<223>  Primer 1 TDF-4DL-1 / Subgenome D

<400>  17
atgcatgtac tacacagaga gac                                              23


<210>  18
<211>  20
<212>  DNA
<213>  artificial

<220>
<223>  Primer 2 TDF-4DL-2 /  Subgenome B

<400>  18
gtgtgttgca tcatggatgc                                                  20


<210>  19
<211>  1989
<212>  DNA
<213>  artificial

<220>
<223>  mutated SEQ ID NO: 3 (reference) pGEMTWA500T003-001-5_M13uni-21

<400>  19
atggggcggc cgccgtgctg cgacaaggcc aacgtgaaga aggggccgtg gacggcggag      60

gaggacgcca agctgctcgc ctacacctcc aaccatggca ccggcaactg gacctctgtt     120

ccccagaggg caggtcggaa ccctcccccc ggccggccgg atcgacgtcc gtcatggaaa     180

tggtgttggt cttgcatggt ttctggctga tgtgtgttgc atcatggatg cgtgcgtgct     240

gcagggctga agcggtgcgg gaagagctgc aggctgaggt cctactacct gaggcccaac     300

ctcaagcacg agaacttcac gcaggaggag gaggagctca tcgtcaccct ccatgccatg     360

ctgggaagca ggtgcgtctc tctgtgtagt acatgcatgt ttctgccatc agttactttc     420

tcgattttgt catcaagttt tcggtgaatg cgaaactttg ctatggaccg actgctgttt     480

ctgtcatcat tcctgctata aaccttgcat ccatatactc tagcgttcag atcttataga     540

tttatggaag ttctgaatat ctcacgttgg tgtatcagac aattgctcat ataccggaaa     600

tcacgtccta cttctattca agaatttact tcagtcgttc gttcaaaaat agaaagaaat     660

gtagtttagt ccagtgacca gtctcgtgtc tacgtacggt tcttctgaat atatatattc     720

tgtttcctgg tagaatgtag tgaaaagctg aaaattcggt gaagaacata tctgaatagc     780

tgtcttaaat tgatagtgtt tgacagaaaa tactccacac tgagcgtaac gtgaaagtgc     840

gtgtacgtgt tgggtgcatg caggtggtct ctgatcgcga accagctgcc ggggcggacg     900

gacaacgacg tcaagaacta ctggaacacc aagctgagca agaagctgag gcagcggggc 960

atcgacccca tcacccaccg ccccatcgcc gatctcatgc agagcatcgg caccctcgcc 1020

atccgcccgc caccgagcgc cgcgggtgcc tcctcctcct cctacctccc cgtgaaccca 1080

ccggcggcgc cggggctcca gccgctgcac gacgacgtca aataccacac agtcctgaac 1140

cagcagcagc agcaggtcat cacgctcctc gaccccgacg cgccaggggc ggcggcgtcc 1200

ccggagcacc agctcaagtg gagcgacttc ctcgcggacg acgccgcggc cctcgaggcg 1260

gcgccgcagg tcgttcttgg tcagtaccag gaggccgcgg tcgctggtgg cggagcacac 1320

gcgtatggcg acactgacag tactgcagcc gatggtgtcg gcgggggcgg ggacgatagc 1380

gcagcgtcag cgttcatcga cgcgatgctg gacagcgaca agaagatggg cgtggaccag 1440

ctcatcgccg acctgctcgc cgacccggca tactactacg gcggaggctc ttcctcttcg 1500

acgtcggagc tggggtgggg ctgttgatca tttatttaac tagaggttac atatctttac 1560

attgggacgg catttaagca tatttagggc atcacatttt cgtttgaact tgttattggt 1620

attcgctgaa ccccgatcga tgatgagcta ttaagcgtgc gtttttgttt ttttaagaaa 1680

atgattaagc gtgagctgat ttgatgtctt tgtggcaaac gaacgggtag atggtgtagg 1740

agtacttttt cgagaatgct aatttccagt cggcaggaaa tctgcaacac atccgcacac 1800

tagcgaacca cctcacctac gttctttttt ttgtaaaaat gaaagcaaat aggcttttta 1860

acctgtctcc ttttctacgt ttgcacaaaa gtgaatgcaa tgtccgatct tttccacact 1920

taagtagatt atttttccaa ctcgtctcga gttgatgaca ccatggtaat ttttgcgcgg 1980

ggcgggggg 1989

<210> 20
<211> 1989
<212> DNA
<213> artificial

<220>
<223> mutated SEQ ID NO: 3 (reference) pGEMTWA500T003-001-2_M13uni-21

<400> 20
atggggcggc cgccgtgctg cgacaaggcc aacgtgaaga aggggccgtg gacggcggag 60

gaggacgcca agctgctcgc ctacacctcc aaccatggca ccggcaactg gacctctgtt 120

ccccagaggg caggtcggaa ccctcccccc ggccggccgg atcgacgtcc gtcatggaaa 180

tggtgttggt cttgcatggt ttctggctga tgtgtgttgc atcatggatg cgtgcgtgct 240

gcagggctga agcggtgcgg gaagagctgc aggctgaggt ccaactacct gaggcccaac 300

ctcaagcacg agaacttcac gcaggaggag gaggagctca tcgtcaccct ccatgccatg 360

ctgggaagca ggtgcgtctc tctgtgtagt acatgcatgt ttctgccatc agttactttc 420

tcgattttgt catcaagttt tcggtgaatg cgaaactttg ctatggaccg actgctgttt 480

ctgtcatcat tcctgctata aaccttgcat ccatatactc tagcgttcag atcttataga 540

tttatggaag ttctgaatat ctcacgttgg tgtatcagac aattgctcat ataccggaaa 600

tcacgtccta cttctattca agaatttact tcagtcgttc gttcaaaaat agaaagaaat 660

gtagtttagt ccagtgacca gtctcgtgtc tacgtacggt tcttctgaat atatatattc 720

tgtttcctgg tagaatgtag tgaaaagctg aaaattcggt gaagaacata tctgaatagc 780

tgtcttaaat tgatagtgtt tgacagaaaa tactccacac tgagcgtaac gtgaaagtgc 840

gtgtacgtgt tgggtgcatg caggtggtct ctgatcgcga accagctgcc ggggcggacg 900

gacaacgacg tcaagaacta ctggaacacc aagctgagca agaagctgag gcagcggggc 960

atcgacccca tcacccaccg ccccatcgcc gatctcatgc agagcatcgg caccctcgcc 1020

atccgcccgc caccgagcgc cgcgggtgcc tcctcctcct cctacctccc cgtgaaccca 1080

ccggcggcgc cggggctcca gccgctgcac gacgacgtca aataccacac agtcctgaac 1140

cagcagcagc agcaggtcat cacgctcctc gaccccgacg cgccaggggc ggcggcgtcc 1200

ccggagcacc agctcaagtg gagcgacttc ctcgcggacg acgccgcggc cctcgaggcg 1260

gcgccgcagg tcgttcttgg tcagtaccag gaggccgcgg tcgctggtgg cggagcacac 1320

gcgtatggcg acactgacag tactgcagcc gatggtgtcg gcgggggcgg ggacgatagc 1380

gcagcgtcag cgttcatcga cgcgatgctg gacagcgaca agaagatggg cgtggaccag 1440

ctcatcgccg acctgctcgc cgacccggca tactactacg gcggaggctc ttcctcttcg 1500

acgtcggagc tggggtgggg ctgttgatca tttatttaac tagaggttac atatctttac 1560

attgggacgg catttaagca tatttagggc atcacatttt cgtttgaact tgttattggt 1620

attcgctgaa ccccgatcga tgatgagcta ttaagcgtgc gtttttgttt ttttaagaaa 1680

atgattaagc gtgagctgat ttgatgtctt tgtggcaaac gaacgggtag atggtgtagg 1740

agtacttttt cgagaatgct aatttccagt cggcaggaaa tctgcaacac atccgcacac 1800

tagcgaacca cctcacctac gttctttttt ttgtaaaaat gaaagcaaat aggcttttta 1860

acctgtctcc ttttctacgt ttgcacaaaa gtgaatgcaa tgtccgatct tttccacact 1920

taagtagatt atttttccaa ctcgtctcga gttgatgaca ccatggtaat ttttgcgcgg 1980

ggcgggggg 1989

<210> 21
<211> 1990
<212> DNA
<213> artificial

<220>
<223> mutated SEQ ID NO: 3 (reference) pGEMTWA500T003-001-4_M13uni-21,
pGEMTWA499T019-001-1_M13uni-21, pGEMTWA499T019-001-2_M13uni-21,

pGEMTWA499T019-001-4_M13uni-21, pGEMTWA499T019-001-5_M13uni-21,
or pGEMTWA499T019-001-3_M13uni-21

<400> 21
atggggcggc cgccgtgctg cgacaaggcc aacgtgaaga aggggccgtg gacggcggag 60

gaggacgcca agctgctcgc ctacacctcc aaccatggca ccggcaactg gacctctgtt 120

ccccagaggg caggtcggaa ccctcccccc ggccggccgg atcgacgtcc gtcatggaaa 180

tggtgttggt cttgcatggt ttctggctga tgtgtgttgc atcatggatg cgtgcgtgct 240

gcagggctga agcggtgcgg gaagagctgc aggctgaggt cccaactacc tgaggcccaa 300

cctcaagcac gagaacttca cgcaggagga ggaggagctc atcgtcaccc tccatgccat 360

gctgggaagc aggtgcgtct ctctgtgtag tacatgcatg tttctgccat cagttacttt 420

ctcgattttg tcatcaagtt ttcggtgaat gcgaaacttt gctatggacc gactgctgtt 480

tctgtcatca ttcctgctat aaaccttgca tccatatact ctagcgttca gatcttatag 540

atttatggaa gttctgaata tctcacgttg gtgtatcaga caattgctca tataccggaa 600

atcacgtcct acttctattc aagaatttac ttcagtcgtt cgttcaaaaa tagaaagaaa 660

tgtagtttag tccagtgacc agtctcgtgt ctacgtacgg ttcttctgaa tatatatatt 720

ctgtttcctg gtagaatgta gtgaaaagct gaaaattcgg tgaagaacat atctgaatag 780

ctgtcttaaa ttgatagtgt ttgacagaaa atactccaca ctgagcgtaa cgtgaaagtg 840

cgtgtacgtg ttgggtgcat gcaggtggtc tctgatcgcg aaccagctgc cggggcggac 900

ggacaacgac gtcaagaact actggaacac caagctgagc aagaagctga ggcagcgggg 960

catcgacccc atcacccacc gccccatcgc cgatctcatg cagagcatcg gcaccctcgc 1020

catccgcccg ccaccgagcg ccgcgggtgc ctcctcctcc tcctacctcc ccgtgaaccc 1080

accggcggcg ccggggctcc agccgctgca cgacgacgtc aaataccaca cagtcctgaa 1140

ccagcagcag cagcaggtca tcacgctcct cgaccccgac gcgccagggg cggcggcgtc 1200

cccggagcac cagctcaagt ggagcgactt cctcgcggac gacgccgcgg ccctcgaggc 1260

ggcgccgcag gtcgttcttg gtcagtacca ggaggccgcg gtcgctggtg gcggagcaca 1320

cgcgtatggc gacactgaca gtactgcagc cgatggtgtc ggcgggggcg gggacgatag 1380

cgcagcgtca gcgttcatcg acgcgatgct ggacagcgac aagaagatgg gcgtggacca 1440

gctcatcgcc gacctgctcg ccgacccggc atactactac ggcggaggct cttcctcttc 1500

gacgtcggag ctggggtggg gctgttgatc atttatttaa ctagaggtta catatcttta 1560

cattgggacg gcatttaagc atatttaggg catcacattt tcgtttgaac ttgttattgg 1620

tattcgctga accccgatcg atgatgagct attaagcgtg cgtttttgtt tttttaagaa 1680

aatgattaag cgtgagctga tttgatgtct ttgtggcaaa cgaacgggta gatggtgtag 1740

gagtactttt tcgagaatgc taatttccag tcggcaggaa atctgcaaca catccgcaca 1800

ctagcgaacc acctcaccta cgttcttttt tttgtaaaaa tgaaagcaaa taggcttttt 1860

aacctgtctc cttttctacg tttgcacaaa agtgaatgca atgtccgatc ttttccacac 1920

ttaagtagat tatttttcca actcgtctcg agttgatgac accatggtaa tttttgcgcg 1980

gggcgggggg 1990

<210> 22
<211> 1991
<212> DNA
<213> artificial

<220>
<223> mutated SEQ ID NO: 3 (reference)

<400> 22
atggggcggc cgccgtgctg cgacaaggcc aacgtgaaga aggggccgtg gacggcggag 60

gaggacgcca agctgctcgc ctacacctcc aaccatggca ccggcaactg gacctctgtt 120

ccccagaggg caggtcggaa ccctcccccc ggccggccgg atcgacgtcc gtcatggaaa 180

tggtgttggt cttgcatggt ttctggctga tgtgtgttgc atcatggatg cgtgcgtgct 240

gcagggctga agcggtgcgg gaagagctgc aggctgaggt aaccaactac ctgaggccca 300

acctcaagca cgagaacttc acgcaggagg aggaggagct catcgtcacc ctccatgcca 360

tgctgggaag caggtgcgtc tctctgtgta gtacatgcat gtttctgcca tcagttactt 420

tctcgatttt gtcatcaagt tttcggtgaa tgcgaaactt tgctatggac cgactgctgt 480

ttctgtcatc attcctgcta taaaccttgc atccatatac tctagcgttc agatcttata 540

gatttatgga agttctgaat atctcacgtt ggtgtatcag acaattgctc atataccgga 600

aatcacgtcc tacttctatt caagaattta cttcagtcgt tcgttcaaaa atagaaagaa 660

atgtagttta gtccagtgac cagtctcgtg tctacgtacg gttcttctga atatatatat 720

tctgtttcct ggtagaatgt agtgaaaagc tgaaaattcg gtgaagaaca tatctgaata 780

gctgtcttaa attgatagtg tttgacagaa aatactccac actgagcgta acgtgaaagt 840

gcgtgtacgt gttgggtgca tgcaggtggt ctctgatcgc gaaccagctg ccggggcgga 900

cggacaacga cgtcaagaac tactggaaca ccaagctgag caagaagctg aggcagcggg 960

gcatcgaccc catcacccac cgccccatcg ccgatctcat gcagagcatc ggcaccctcg 1020

ccatccgccc gccaccgagc gccgcgggtg cctcctcctc ctcctacctc cccgtgaacc 1080

caccggcggc gccggggctc cagccgctgc acgacgacgt caaataccac acagtcctga 1140

accagcagca gcagcaggtc atcacgctcc tcgaccccga cgcgccaggg gcggcggcgt 1200

ccccggagca ccagctcaag tggagcgact tcctcgcgga cgacgccgcg gccctcgagg 1260

cggcgccgca ggtcgttctt ggtcagtacc aggaggccgc ggtcgctggt ggcggagcac 1320

acgcgtatgg cgacactgac agtactgcag ccgatggtgt cggcgggggc ggggacgata 1380

gcgcagcgtc agcgttcatc gacgcgatgc tggacagcga caagaagatg ggcgtggacc 1440

agctcatcgc cgacctgctc gccgacccgg catactacta cggcggaggc tcttcctctt 1500

cgacgtcgga gctggggtgg ggctgttgat catttattta actagaggtt acatatcttt 1560

acattgggac ggcatttaag catatttagg gcatcacatt ttcgtttgaa cttgttattg 1620

gtattcgctg aaccccgatc gatgatgagc tattaagcgt gcgtttttgt ttttttaaga 1680

aaatgattaa gcgtgagctg atttgatgtc tttgtggcaa acgaacgggt agatggtgta 1740

ggagtacttt ttcgagaatg ctaatttcca gtcggcagga aatctgcaac acatccgcac 1800

actagcgaac cacctcacct acgttctttt ttttgtaaaa atgaaagcaa ataggctttt 1860

taacctgtct ccttttctac gtttgcacaa aagtgaatgc aatgtccgat cttttccaca 1920

cttaagtaga ttatttttcc aactcgtctc gagttgatga caccatggta atttttgcgc 1980

ggggcggggg g 1991

<210> 23
<211> 1964
<212> DNA
<213> artificial

<220>
<223> mutated SEQ ID NO: 3 (reference) pGEMTWA499T002-001-1_M13uni-21

<400> 23
atggggcggc cgccgtgctg cgacaaggcc aacgtgaaga aggggccgtg gacggcggag 60

gaggacgcca agctgctcgc ctacacctcc aaccatggca ccggcaactg gacctctgtt 120

ccccagaggg caggtcggaa ccctcccccc ggccggccgg atcgacgtcc gtcatggaaa 180

tggtgttggt cttgcatggt ttctggctga tgtgtgttgc atcatggatg cgtgcgtgct 240

gcagggctga agcggtgaac tacctgaggc ccaacctcaa gcacgagaac ttcacgcagg 300

aggaggagga gctcatcgtc accctccatg ccatgctggg aagcaggtgc gtctctctgt 360

gtagtacatg catgtttctg ccatcagtta ctttctcgat tttgtcatca agttttcggt 420

gaatgcgaaa ctttgctatg gaccgactgc tgtttctgtc atcattcctg ctataaacct 480

tgcatccata tactctagcg ttcagatctt atagatttat ggaagttctg aatatctcac 540

gttggtgtat cagacaattg ctcatatacc ggaaatcacg tcctacttct attcaagaat 600

ttacttcagt cgttcgttca aaaatagaaa gaaatgtagt ttagtccagt gaccagtctc 660

gtgtctacgt acggttcttc tgaatatata tattctgttt cctggtagaa tgtagtgaaa 720

agctgaaaat tcggtgaaga acatatctga atagctgtct taaattgata gtgtttgaca 780

gaaaatactc cacactgagc gtaacgtgaa agtgcgtgta cgtgttgggt gcatgcaggt 840

ggtctctgat cgcgaaccag ctgccggggc ggacggacaa cgacgtcaag aactactgga 900

acaccaagct gagcaagaag ctgaggcagc ggggcatcga ccccatcacc caccgcccca 960

tcgccgatct catgcagagc atcggcaccc tcgccatccg cccgccaccg agcgccgcgg 1020

gtgcctcctc ctcctcctac ctccccgtga acccaccggc ggcgccgggg ctccagccgc 1080

tgcacgacga cgtcaaatac cacacagtcc tgaaccagca gcagcagcag gtcatcacgc 1140

tcctcgaccc cgacgcgcca ggggcggcgg cgtccccgga gcaccagctc aagtggagcg 1200

acttcctcgc ggacgacgcc gcggccctcg aggcggcgcc gcaggtcgtt cttggtcagt 1260

accaggaggc cgcggtcgct ggtggcggag cacacgcgta tggcgacact gacagtactg 1320

cagccgatgg tgtcggcggg ggcggggacg atagcgcagc gtcagcgttc atcgacgcga 1380

tgctggacag cgacaagaag atgggcgtgg accagctcat cgccgacctg ctcgccgacc 1440

cggcatacta ctacggcgga ggctcttcct cttcgacgtc ggagctgggg tggggctgtt 1500

gatcatttat ttaactagag gttacatatc tttacattgg gacggcattt aagcatattt 1560

agggcatcac attttcgttt gaacttgtta ttggtattcg ctgaaccccg atcgatgatg 1620

agctattaag cgtgcgtttt tgttttttta agaaaatgat taagcgtgag ctgatttgat 1680

gtctttgtgg caaacgaacg ggtagatggt gtaggagtac tttttcgaga atgctaattt 1740

ccagtcggca ggaaatctgc aacacatccg cacactagcg aaccacctca cctacgttct 1800

tttttttgta aaaatgaaag caaataggct ttttaacctg tctccttttc tacgtttgca 1860

caaaagtgaa tgcaatgtcc gatcttttcc acacttaagt agattatttt tccaactcgt 1920

ctcgagttga tgacaccatg gtaatttttg cgcggggcgg gggg 1964

<210> 24
<211> 1964
<212> DNA
<213> artificial

<220>
<223> mutated SEQ ID NO: 3 (reference) pGEMTW A499T002-001-2_M13uni-21

<400> 24
atggggcggc cgccgtgctg cgacaaggcc aacgtgaaga aggggccgtg gacggcggag 60

gaggacgcca agctgctcgc ctacacctcc aaccatggca ccggcaactg gacctctgtt 120

ccccagaggg caggtcggaa ccctcccccc ggccggccgg atcgacgtcc gtcatggaaa 180

tggtgttggt cttgcatggt ttctggctga tgtgtgttgc atcatggatg cgtgcgtgct 240

gcagggctga agcggtgaac tacctgaggc ccaacctcaa gcacgagaac ttcacgcagg 300

aggaggagga gctcatcgtc accctccatg ccatgctggg aagcaggtgc gtctctctgt 360

gtagtacatg catgtttctg ccatcagtta ctttctcgat tttgtcatca agttttcggt 420

gaatgcgaaa ctttgctatg gaccgactgc tgtttctgtc atcattcctg ctataaacct 480

tgcatccata tactctagcg ttcagatctt atagatttat ggaagttctg aatatctcac 540

gttggtgtat cagacaattg ctcatatacc ggaaatcacg tcctacttct attcaagaat 600

ttacttcagt cgttcgttca aaaatagaaa gaaatgtagt ttagtccagt gaccagtctc 660

gtgtctacgt acggttcttc tgaatatata tattctgttt cctggtagaa tgtagtgaaa 720

agctgaaaat tcggtgaaga acatatctga atagctgtct taaattgata gtgtttgaca 780

gaaaatactc cacactgagc gtaacgtgaa agtgcgtgta cgtgttgggt gcatgcaggt 840

ggtctctgat cgcgaaccag ctgccggggc ggacggacaa cgacgtcaag aactactgga 900

acaccaagct gagcaagaag ctgaggcagc ggggcatcga ccccatcacc caccgcccca 960

tcgccgatct catgcagagc atcggcaccc tcgccatccg cccgccaccg agcgccgcgg 1020

gtgcctcctc ctcctcctac ctccccgtga acccaccggc ggcgccgggg ctccagccgc 1080

tgcacgacga cgtcaaatac cacacagtcc tgaaccagca gcagcagcag gtcatcacgc 1140

tcctcgaccc cgacgcgcca ggggcggcgg cgtccccgga gcaccagctc aagtggagcg 1200

acttcctcgc ggacgacgcc gcggccctcg aggcggcgcc gcaggtcgtt cttggtcagt 1260

accaggaggc cgcggtcgct ggtggcggag cacacgcgta tggcgacact gacagtactg 1320

cagccgatgg tgtcggcggg ggcggggacg atagcgcagc gtcagcgttc atcgacgcga 1380

tgctggacag cgacaagaag atgggcgtgg accagctcat cgccgacctg ctcgccgacc 1440

cggcatacta ctacggcgga ggctcttcct cttcgacgtc ggagctgggg tggggctgtt 1500

gatcatttat ttaactagag gttacatatc tttacattgg gacggcattt aagcatattt 1560

agggcatcac attttcgttt gaacttgtta ttggtattcg ctgaaccccg atcgatgatg 1620

agctattaag cgtgcgtttt tgttttttta agaaaatgat taagcgtgag ctgatttgat 1680

gtctttgtgg caaacgaacg ggtagatggt gtaggagtac tttttcgaga atgctaattt 1740

ccagtcggca ggaaatctgc aacacatccg cacactagcg aaccacctca cctacgttct 1800

tttttttgta aaaatgaaag caaataggct ttttaacctg tctccttttc tacgtttgca 1860

caaaagtgaa tgcaatgtcc gatcttttcc acacttaagt agattatttt tccaactcgt 1920

ctcgagttga tgacaccatg gtaatttttg cgcggggcgg gggg 1964

<210> 25
<211> 1964
<212> DNA
<213> artificial

<220>
<223> mutated SEQ ID NO: 3 (reference) pGEMTWA499T002-001-3_M13uni-21,
pGEMTWA499T002-0014_M13uni-21, or pGEMTWA499T002-001-5_M13uni-21

<400> 25
atggggcggc cgccgtgctg cgacaaggcc aacgtgaaga aggggccgtg gacggcggag 60

gaggacgcca agctgctcgc ctacacctcc aaccatggca ccggcaactg gacctctgtt 120

ccccagaggg caggtcggaa ccctcccccc ggccggccgg atcgacgtcc gtcatggaaa 180

tggtgttggt cttgcatggt ttctggctga tgtgtgttgc atcatggatg cgtgcgtgct 240

gcagggctga agcggtgaac tacctgaggc ccaacctcaa gcacgagaac ttcacgcagg 300

aggaggagga gctcatcgtc accctccatg ccatgctggg aagcaggtgc gtctctctgt 360

gtagtacatg catgtttctg ccatcagtta ctttctcgat tttgtcatca agttttcggt 420

gaatgcgaaa ctttgctatg gaccgactgc tgtttctgtc atcattcctg ctataaacct 480

tgcatccata tactctagcg ttcagatctt atagatttat ggaagttctg aatatctcac 540

gttggtgtat cagacaattg ctcatatacc ggaaatcacg tcctacttct attcaagaat 600

ttacttcagt cgttcgttca aaaatagaaa gaaatgtagt ttagtccagt gaccagtctc 660

gtgtctacgt acggttcttc tgaatatata tattctgttt cctggtagaa tgtagtgaaa 720

agctgaaaat tcggtgaaga acatatctga atagctgtct taaattgata gtgtttgaca 780

gaaaatactc cacactgagc gtaacgtgaa agtgcgtgta cgtgttgggt gcatgcaggt 840

ggtctctgat cgcgaaccag ctgccggggc ggacggacaa cgacgtcaag aactactgga 900

acaccaagct gagcaagaag ctgaggcagc ggggcatcga ccccatcacc caccgcccca 960

tcgccgatct catgcagagc atcggcaccc tcgccatccg cccgccaccg agcgccgcgg 1020

gtgcctcctc ctcctcctac ctccccgtga acccaccggc ggcgccgggg ctccagccgc 1080

tgcacgacga cgtcaaatac cacacagtcc tgaaccagca gcagcagcag gtcatcacgc 1140

tcctcgaccc cgacgcgcca ggggcggcgg cgtccccgga gcaccagctc aagtggagcg 1200

acttcctcgc ggacgacgcc gcggccctcg aggcggcgcc gcaggtcgtt cttggtcagt 1260

accaggaggc cgcggtcgct ggtggcggag cacacgcgta tggcgacact gacagtactg 1320

cagccgatgg tgtcggcggg ggcggggacg atagcgcagc gtcagcgttc atcgacgcga 1380

tgctggacag cgacaagaag atgggcgtgg accagctcat cgccgacctg ctcgccgacc 1440

cggcatacta ctacggcgga ggctcttcct cttcgacgtc ggagctgggg tggggctgtt 1500

gatcatttat ttaactagag gttacatatc tttacattgg gacggcattt aagcatattt 1560

agggcatcac attttcgttt gaacttgtta ttggtattcg ctgaaccccg atcgatgatg 1620

agctattaag cgtgcgtttt tgttttttta agaaaatgat taagcgtgag ctgatttgat 1680

gtctttgtgg caaacgaacg ggtagatggt gtaggagtac tttttcgaga atgctaattt 1740

ccagtcggca ggaaatctgc aacacatccg cacactagcg aaccacctca cctacgttct 1800

tttttttgta aaaatgaaag caaataggct ttttaacctg tctccttttc tacgtttgca 1860

caaaagtgaa tgcaatgtcc gatcttttcc acacttaagt agattatttt tccaactcgt 1920

ctcgagttga tgacaccatg gtaatttttg cgcggggcgg gggg 1964

<210>  26
<211>  1554
<212>  DNA
<213>  Cynodon dactylon

<220>
<223>  P450 Gen

<400>  26
atggataagg cctacgtggc cctcctctcc ttcgcctccc tcttcttgct ccactacctc      60

gtttcccgcc gcaatggcac cgggaagggc agcaaggcca agggcgcgct gccgccaagc     120

cctccatccg ttccgttcct gggccacctc caccttgtca agacgccatt ccacgctgcg     180

ctggcacgcc tcgcggactg ccacggcccg gtcttctccc tgcggatggg agcccgcccc     240

gcagttgtgg tgtcctcgcc ggagcacgcc aaggagtgct tcacggagca cgacgtggcc     300

ttcgccaacc ggccgcgctt tccctcgcag cagctcgcct ccttcaacgg tgccgcgctg     360

ggttccgcca gctacggccc gtactggcgc aacctccgcc gcgtcgccac cgtccacctc     420

ctgtccgcgc accgcgtcgc gtgcatgacg gggactatcg cggccgaggt gcgggccatg     480

gtgcgacgga tgaaccgcgc cgcgcaggtg gcatcaggcg gcgcggcgcg catcgagctc     540

aagcggaggc tatttgaggt ctcgctcagc gtgcttatgg agaccatcgc gcggaccaag     600

acgtcacgta cggaggcgga cgacgacacg gacatgtcgc ctgaggcccg ggagttcaag     660

cagatcgtgg atgagctcct gcctcacctc ggcacggcta acttgtggga ctacatgccg     720

gtgttgcggt ggttcgacgt gttcggcgtg aggaagaaga tcgtgtccgc ggtgaggaga     780

agggacgcgt tcctgcggca tcttgtcgac gcagagagga cgaggctgga cgacggcaac     840

gatgcgggcg agaagaagag catcattgct atgctgctca ctctgcagaa gtcagagccg     900

gacgtctact cggataccat gatcatggct ctatgtggga acttgtttgg ggccggcaca     960

gagaccacgt cgacgaccac cgaatgggcc atgtctctcc tcctcaacca cccggagaag    1020

ctcaggaagg cgcaggctga gatcgatgct gtcgtgggca catcccgcct tcttaccgcc    1080

gacgacatgc ctcgtctcac ctacctccgc tgcatcatcg acgagaccat gcgcctgtac    1140

ccggccgcac cacttctgct gccacacgag tcctcgacac actgcaaggt cggcggctac    1200

gacgtgcccg ccggcacgat gctgctcgtc aacgtgtacg ccatccacag ggaccccgcg    1260

gtgtgggacg ggccgaccga gttcgtgccg gagcggttcg aggatggcaa ggcagaaggc    1320

cggctgctga tgccgttcgg gatgggacgg cgcaagtgtc ccggcgagac gctcgcgctg    1380

cggacgatcg ggctggtgct cggcacgctg atccagtgtt tcgactggga ccgggttgat    1440

ggtcttgagg tcgacatgac tgaaagtggt gggctcacga tccccagggc tgtcccgttg    1500

gaggccatgt gcaggcctcg tgcgacgatg cgtgaggttt tgcaggagct ctga          1554

<210>  27
<211>  1554
<212>  DNA
<213>  Artificial Sequence

<220>
<223>  Codon-optimierte Version des P450 Gens

<400>  27
atggacaagg cctacgtcgc cctgctgagc ttcgcttctc tgttcctgct gcactacctg      60

gtgtctaggc gcaacggcac cggcaagggc agcaaggcca agggcgccct gccaccaagc     120

cctccaagcg tgccattcct gggccatctg cacctggtca agaccccatt ccatgccgct     180

ctggctaggc tggccgactg ccacggccca gtgttcagcc tgaggatggg cgccaggcca     240

gctgtggtgg tgagcagccc agagcacgcc aaggagtgct tcactgagca cgatgtggcc     300

ttcgccaaca ggccaaggtt cccatctcag cagctcgcct cattcaacgg cgctgctctt     360

ggctctgcta gctacggccc atactggcgc aatcttagaa gggtggccac cgtgcatctg     420

ctgtctgctc acagagtggc ttgcatgacc ggcactatcg ctgctgaggt gagggctatg     480

gtgaggcgca tgaacagggc tgctcaagtt gcctctggcg gcgctgctag gattgagctt     540

aagaggcgcc tgttcgaggt gagcctgagc gtgctgatgg agactatcgc caggaccaag     600

accagcagga ccgaggctga cgacgacacc gatatgagcc cagaggctcg cgagttcaag     660

cagatcgtgg atgagctgct gccacacctg ggcactgcta acctgtggga ctacatgcca     720

gtgctgcgct ggttcgatgt gttcggcgtg cgcaagaaga tcgtcagcgc cgtcaggcgc     780

agggatgctt tcctgaggca tctggtggat gccgagagga ccaggctgga cgatggcaac     840

gacgccggcg agaagaagtc catcattgcc atgctgctga ccctgcaaaa gtccgagcca     900

gacgtgtaca gcgacacgat gatcatggcc ctgtgcggca acctgttcgg cgctggcacc     960

gagactacct ctaccactac tgagtgggcc atgagcctgc tgctgaacca cccagagaag    1020

ctgaggaagg cccaggctga gatcgatgct gtggtgggca cttctaggct gctgaccgct    1080

gacgatatgc cgaggctgac ttacctgcgc tgcatcatcg acgagactat gaggctgtac    1140

ccagccgctc cactgctgct cccacatgag tcatctaccc actgcaaggt gggcggctac    1200

gatgttccag ctggcaccat gctgctggtg aacgtgtacg ctatccacag ggacccagct    1260

gtgtgggatg gcccaactga gttcgtgcca gagagattcg aggacggcaa ggctgagggc    1320

aggctgctga tgccattcgg catgggcagg cgcaagtgcc caggcgagac tcttgctctt    1380

aggaccatcg gcctggtgct gggcactctg atccagtgct tcgactggga tagggtggac    1440

ggccttgagg tggacatgac cgagtctggc ggcctgacta tcccaagggc tgttccactt    1500

gaggccatgt gcaggccaag ggctactatg cgcgaggtgc tgcaagagct gtga          1554

<210>  28
<211>  517
<212>  PRT
<213>  Artificial Sequence

<220>
<223>  AA-Sequenz der Codon-optimierten Version des P450 Gens

<400>  28

Met Asp Lys Ala Tyr Val Ala Leu Leu Ser Phe Ala Ser Leu Phe Leu
1               5                   10                  15

Leu His Tyr Leu Val Ser Arg Arg Asn Gly Thr Gly Lys Gly Ser Lys
            20                  25                  30

Ala Lys Gly Ala Leu Pro Pro Ser Pro Pro Ser Val Pro Phe Leu Gly
        35                  40                  45

His Leu His Leu Val Lys Thr Pro Phe His Ala Ala Leu Ala Arg Leu
    50                  55                  60

Ala Asp Cys His Gly Pro Val Phe Ser Leu Arg Met Gly Ala Arg Pro
65                  70                  75                  80

Ala Val Val Val Ser Ser Pro Glu His Ala Lys Glu Cys Phe Thr Glu
                85                  90                  95

His Asp Val Ala Phe Ala Asn Arg Pro Arg Phe Pro Ser Gln Gln Leu
            100                 105                 110

Ala Ser Phe Asn Gly Ala Ala Leu Gly Ser Ala Ser Tyr Gly Pro Tyr
        115                 120                 125

Trp Arg Asn Leu Arg Arg Val Ala Thr Val His Leu Leu Ser Ala His
    130                 135                 140

Arg Val Ala Cys Met Thr Gly Thr Ile Ala Ala Glu Val Arg Ala Met
145                 150                 155                 160

Val Arg Arg Met Asn Arg Ala Ala Gln Val Ala Ser Gly Gly Ala Ala
                165                 170                 175

Arg Ile Glu Leu Lys Arg Arg Leu Phe Glu Val Ser Leu Ser Val Leu
            180                 185                 190

Met Glu Thr Ile Ala Arg Thr Lys Thr Ser Arg Thr Glu Ala Asp Asp
        195                 200                 205

Asp Thr Asp Met Ser Pro Glu Ala Arg Glu Phe Lys Gln Ile Val Asp

210                 215                 220

Glu Leu Leu Pro His Leu Gly Thr Ala Asn Leu Trp Asp Tyr Met Pro
225                 230                 235                 240

Val Leu Arg Trp Phe Asp Val Phe Gly Val Arg Lys Lys Ile Val Ser
                245                 250                 255

Ala Val Arg Arg Arg Asp Ala Phe Leu Arg His Leu Val Asp Ala Glu
            260                 265                 270

Arg Thr Arg Leu Asp Asp Gly Asn Asp Ala Gly Glu Lys Lys Ser Ile
        275                 280                 285

Ile Ala Met Leu Leu Thr Leu Gln Lys Ser Glu Pro Asp Val Tyr Ser
    290                 295                 300

Asp Thr Met Ile Met Ala Leu Cys Gly Asn Leu Phe Gly Ala Gly Thr
305                 310                 315                 320

Glu Thr Thr Ser Thr Thr Thr Glu Trp Ala Met Ser Leu Leu Leu Asn
                325                 330                 335

His Pro Glu Lys Leu Arg Lys Ala Gln Ala Glu Ile Asp Ala Val Val
            340                 345                 350

Gly Thr Ser Arg Leu Leu Thr Ala Asp Asp Met Pro Arg Leu Thr Tyr
        355                 360                 365

Leu Arg Cys Ile Ile Asp Glu Thr Met Arg Leu Tyr Pro Ala Ala Pro
    370                 375                 380

Leu Leu Leu Pro His Glu Ser Ser Thr His Cys Lys Val Gly Gly Tyr
385                 390                 395                 400

Asp Val Pro Ala Gly Thr Met Leu Leu Val Asn Val Tyr Ala Ile His
                405                 410                 415

Arg Asp Pro Ala Val Trp Asp Gly Pro Thr Glu Phe Val Pro Glu Arg
            420                 425                 430

Phe Glu Asp Gly Lys Ala Glu Gly Arg Leu Leu Met Pro Phe Gly Met
        435                 440                 445

Gly Arg Arg Lys Cys Pro Gly Glu Thr Leu Ala Leu Arg Thr Ile Gly
    450                 455                 460

Leu Val Leu Gly Thr Leu Ile Gln Cys Phe Asp Trp Asp Arg Val Asp
465                 470                 475                 480

Gly Leu Glu Val Asp Met Thr Glu Ser Gly Gly Leu Thr Ile Pro Arg
                485                 490                 495

Ala Val Pro Leu Glu Ala Met Cys Arg Pro Arg Ala Thr Met Arg Glu
            500                 505                 510

Val Leu Gln Glu Leu
        515

<210>  29
<211>  4875
<212>  DNA
<213>  Artificial Sequence

<220>
<223>  Vektorfragment pAMK-TITaTDF-70SubiintronP450wheat

<400>  29

atacgtccaa ctcgtgaaaa tatttgaaac agcccccggc ccctccaccc tcacacacag      60

cgtttgatta gattagatcg gattaaccca ccaacccgca tctgaatatt tgatccctcc      120

ctgattaagc ggttgatttg ccggagggaa acagttgcag gcgtgccata aactagggtt      180

aaagaagcgt ttccatcgga tcgtcactaa cccatgcatg ccatcacttg ttaaatgagg      240

agggggaggg tcatgcttcc gtcgcgtgcc cctccaccat ctggacatgt tgttgccgtg      300

cagtgcccct ctctctctcg ccggagcgcc ccggccccta tatgtacacc ggtgccaccc      360

tgcagtgcga tctcgtcggg gggcaccgca ccggaggttg gtgcagtgcg ggggttctcg      420

agctatatat ctatcaccgt ttggctgctt gtagaagggg aggggaagga gggtggcgcc      480

ggccgggatg gggcggccgc cgtgctgcga caaggccaac gtgaagaagg ggccgtggac      540

ggcggaggag gacgccaagc tgctcgccta cacctccaac catggcaccg gcaactggac      600

ctctgttccc cagagggcag gtcggaaccc tccccccggc cggccggatc gacgtccgtc      660

atggaaatgg tgttggtctt gcatggtttc tggctgatgt gtgttgcatc atggatgcgt      720

gcgtgctgca gggctgaaga ttagccttac tagtggatcc gttcagaaga ccagagggct      780

attgagactt ttcaacaaag ggtaatatcg ggaaacctcc tcggattcca ttgcccagct      840

atctgtcact tcatcgaaag gacagtagaa aaggaagatg gcttctacaa atgccatcat      900

tgcgataaag gaaaggctat cgttcaagat gcctctaccg acagtggtcc caaagatgga      960

cccccaccca cgaggaacat cgtggaaaaa gaagacgttc caaccacgtc ttcaaagcaa      1020

gtggattgat gtgatacatg gtggagcacg acactctcgt ctactccaag aatatcaaag      1080

atacagtctc agaagaccag agggctattg agacttttca acaaagggta atatcgggaa      1140

acctcctcgg attccattgc ccagctatct gtcacttcat cgaaaggaca gtagaaaagg 1200

aagatggctt ctacaaatgc catcattgcg ataaaggaaa ggctatcgtt caagatgcct 1260

ctaccgacag tggtcccaaa gatggacccc cacccacgag gaacatcgtg gaaaaagaag 1320

acgttccaac cacgtcttca aagcaagtgg attgatgtga tatctccact gacgtaaggg 1380

atgacgcaca atcccactat ccttcgcaag acccttcctc tatataagga agttcatttc 1440

atttggagag gattagtacg ccgctcgtcc tccccccccc cccctctcta ccttctctag 1500

atcggcgttc cggtccatgg ttagggcccg gtagttctac ttctgttcat gtttgtgtta 1560

gatccgtgtt tgtgttagat ccgtgctgct agcgttcgta cacggatgcg acctgtacgt 1620

cagacacgtt ctgattgcta acttgccagt gtttctcttt ggggaatcct gggatggctc 1680

tagccgttcc gcagacggga tcgatttcat gatttttttt gtttcgttgc atagggtttg 1740

gtttgccctt ttcctttatt tcaatatatg ccgtgcactt gtttgtcggg tcatcttttc 1800

atgctttttt ttgtcttggt tgtgatgatg tggtctggtt gggcggtcgt tctagatcgg 1860

agtagaattc tgtttcaaac tacctggtgg atttattaat tttggatctg tatgtgtgtg 1920

ccatacatat tcatagttac gaattgaaga tgatggatgg aaatatcgat ctaggatagg 1980

tatacatgtt gatgcgggtt ttactgatgc atatacagag atgctttttg ttcgcttggt 2040

tgtgatgatg tggtgtggtt gggcggtcgt tcattcgttc tagatcggag tagaatactg 2100

tttcaaacta cctggtgtat ttattaattt tggaactgta tgtgtgtgtc atacatcttc 2160

atagttacga gtttaagatg gatggaaata tcgatctagg ataggtatac atgttgatgt 2220

gggttttact gatgcatata catgatggca tatgcagcat ctattcatat gctctaacct 2280

tgagtaccta tctattataa taaacaagta tgttttataa ttattttgat cttgatatac 2340

ttggatgatg gcatatgcag cagctatatg tggatttttt tagccctgcc ttcatacgct 2400

atttatttgc ttggtactgt ttcttttgtc gatgctcacc ctgttgtttg gtgttacttc 2460

tgcagattga tggacaaggc ctacgtcgcc ctgctgagct tcgcttctct gttcctgctg 2520

cactacctgg tgtctaggcg caacggcacc ggcaagggca gcaaggccaa gggcgccctg 2580

ccaccaagcc ctccaagcgt gccattcctg ggccatctgc acctggtcaa gaccccattc 2640

catgccgctc tggctaggct ggccgactgc cacggcccag tgttcagcct gaggatgggc 2700

gccaggccag ctgtggtggt gagcagccca gagcacgcca aggagtgctt cactgagcac 2760

gatgtggcct tcgccaacag gccaaggttc ccatctcagc agctcgcctc attcaacggc 2820

gctgctcttg gctctgctag ctacggccca tactggcgca atcttagaag ggtggccacc 2880

gtgcatctgc tgtctgctca cagagtggct tgcatgaccg gcactatcgc tgctgaggtg 2940

agggctatgg tgaggcgcat gaacaggcct gctcaagttg cctctggcgg cgctgctagg 3000

attgagctta agaggcgcct gttcgaggtg agcctgagcg tgctgatgga gactatcgcc 3060

aggaccaaga ccagcaggac cgaggctgac gacgacaccg atatgagccc agaggctcgc 3120

gagttcaagc agatcgtgga tgagctgctg ccacacctgg gcactgctaa cctgtgggac 3180

tacatgccag tgctgcgctg gttcgatgtg ttcggcgtgc gcaagaagat cgtcagcgcc 3240

gtcaggcgca gggatgcttt cctgaggcat ctggtggatg ccgagaggac caggctggac 3300

gatggcaacg acgccggcga gaagaagtcc atcattgcca tgctgctgac cctgcaaaag 3360

tccgagccag acgtgtacag cgacacgatg atcatggccc tgtgcggcaa cctgttcggc 3420

gctggcaccg agactacctc taccactact gagtgggcca tgagcctgct gctgaaccac 3480

ccagagaagc tgaggaaggc ccaggctgag atcgatgctg tggtgggcac ttctaggctg 3540

ctgaccgctg acgatatgcc gaggctgact tacctgcgct gcatcatcga cgagactatg 3600

aggctgtacc cagccgctcc actgctgctc ccacatgagt catctaccca ctgcaaggtg 3660

ggcggctacg atgttccagc tggcaccatg ctgctggtga acgtgtacgc tatccacagg 3720

gacccagctg tgtgggatgg cccaactgag ttcgtgccag agagattcga ggacggcaag 3780

gctgagggca ggctgctgat gccattcggc atgggcaggc gcaagtgccc aggcgagact 3840

cttgctctta ggaccatcgg cctggtgctg ggcactctga tccagtgctt cgactgggat 3900

agggtggacg gccttgaggt ggacatgacc gagtctggcg gcctgactat cccaagggct 3960

gttccacttg aggccatgtg caggccaagg gctactatgc gcgaggtgct gcaagagctg 4020

tgaatgccga atttccccga tcgttcaaac atttggcaat aaagtttctt aagattgaat 4080

cctgttgccg gtcttgcgat gattatcata taatttctgt tgaattacgt taagcatgta 4140

ataattaaca tgtaatgcat gacgttattt atgagatggg tttttatgat tagagtcccg 4200

caattataca tttaatacgc gatagaaaac aaaatatagc gcgcaaacta ggataaatta 4260

tcgcgcgcgg tgtcatctat gttactagat cgctcgaaga tcccccgggc tgcaggaatt 4320

caagcttatt gctcaagcac gagaacttca cgcaggagga ggaggagctc atcgtcaccc 4380

tccatgccat gctgggaagc aggtgcgtct ctctgtgtag tacatgcatg tttctgccat 4440

cagttacttt ctcgattttg tcatcaagtt ttcggtgaat gcgaaacttt gctatggacc 4500

gactgctgtt tctgtcatca ttcctgctat aaaccttgca tccatatact ctagcgttca 4560

gatcttatag atttatggaa gttctgaata tctcacgttg gtgtatcaga caattgctca 4620

tataccggaa atcacgtcct acttctattc aagaatttac ttcagtcgtt cgttcaaaaa 4680

tagaaagaaa tgtagtttag tccagtgacc agtctcgtgt ctacgtacgg ttcttctgaa 4740

tatatatatt ctgtttcctg gtagaatgta gtgaaaagct gaaaattcgg tgaagaacat 4800

atctgaatag ctgtcttaaa ttgatagtgt ttgacagaaa atactccaca ctgagcgtaa 4860

cgtgaaagtg cgtgt 4875

<210>  30
<211>  8630
<212>  DNA
<213>  Artificial Sequence

<220>
<223>  Fragment des Vektors pDest_ubiCAS9_ChimeraTaU6-TaTDF


<220>
<221>  misc_feature
<222>  (7519)..(7519)
<223>  n is a, c, g, or t

<400>  30
ccgctgacgc cgtcccggac tgatgggctg cctgtatcga gtggtgattt tgtgccgagc      60

tgccggtcgg ggagctgttg gctggctggt ggcaggatat attgtggtgt aaacaaattg     120

acgcttagac aacttaataa cacattgcgg acgtttttaa tgtactgaat taacgccgaa     180

ttgctctagc caatacgcaa accgcctctc cccgcgcgtt ggccgattca ttaattgcag     240

cgtgacccgg tcgtgcccct ctctagagat aatgagcatt gcatgtctaa gttataaaaa     300

attaccacat attttttttg tcacacttgt ttgaagtgca gtttatctat ctttatacat     360

atatttaaac tttactctac gaataatata atctatagta ctacaataat atcagtgttt     420

tagagaatca tataaatgaa cagttagaca tggtctaaag gacaattgag tattttgaca     480

acaggactct acagttttat ctttttagtg tgcatgtgtt ctcctttttt tttgcaaata     540

gcttcaccta tataatactt catccatttt attagtacat ccatttaggg tttagggtta     600

atggttttta tagactaatt tttttagtac atctatttta ttctatttta gcctctaaat     660

taagaaaact aaaactctat tttagttttt ttatttaata atttagatat aaaatagaat     720

aaaataaagt gactaaaaat taaacaaata ccctttaaga aattaaaaaa actaaggaaa     780

catttttctt gtttcgagta gataatgcca gcctgttaaa cgccgtcgat cgacgagtct     840

aacggacacc aaccagcgaa ccagcagcgt cgcgtcgggc caagcgaagc agacggcacg     900

gcatctctgt cgctgcctct ggacccctct cgagagttcc gctccaccgt tggacttgct     960

ccgctgtcgg catccagaaa ttgcgtggcg gagcggcaga cgtgagccgg cacggcaggc    1020

ggcctcctcc tcctctcacg gcaccggcag ctacggggga ttcctttccc accgctcctt    1080

cgctttccct tcctcgcccg ccgtaataaa tagacacccc ctccacaccc tctttcccca    1140

acctcgtgtt gttcggagcg cacacacaca caaccagatc tcccccaaat ccacccgtcg    1200

gcacctccgc ttcaaggtac gccgctcgtc ctcccccccc ccccctctct accttctcta    1260

gatcggcgtt ccggtccatg gttagggccc ggtagttcta cttctgttca tgtttgtgtt    1320

agatccgtgt ttgtgttaga tccgtgctgc tagcgttcgt acacggatgc gacctgtacg    1380

tcagacacgt tctgattgct aacttgccag tgtttctctt tggggaatcc tgggatggct 1440

ctagccgttc cgcagacggg atcgatctag gataggtata catgttgatg tgggttttac 1500

tgatgcatat acatgatggc atatgcagca tctattcata tgctctaacc ttgagtacct 1560

atctattata ataaacaagt atgttttata attattttga tcttgatata cttggatgat 1620

ggcatatgca gcagctatat gtggattttt ttagccctgc cttcatacgc tatttatttg 1680

cttggtactg tttcttttgt cgatgctcac cctgttgttt ggtgttactt ctgcaggggt 1740

accgccatgg ataagaagta ctctatcgga ctcgatatcg gaactaactc tgtgggatgg 1800

gctgtgatca ccgatgagta caaggtgcca tctaagaagt tcaaggttct cggaaacacc 1860

gataggcact ctatcaagaa aaaccttatc ggtgctctcc tcttcgattc tggtgaaact 1920

gctgaggcta ccagactcaa gagaaccgct agaagaaggt acaccagaag aaagaacagg 1980

atctgctacc tccaagagat cttctctaac gagatggcta aagtggatga ttcattcttc 2040

cacaggctcg aagagtcatt cctcgtggaa gaagataaga agcacgagag gcaccctatc 2100

ttcggaaaca tcgttgatga ggtggcatac cacgagaagt accctactat ctaccacctc 2160

agaaagaagc tcgttgattc tactgataag gctgatctca ggctcatcta cctcgctctc 2220

gctcacatga tcaagttcag aggacacttc ctcatcgagg gtgatctcaa ccctgataac 2280

tctgatgtgg ataagttgtt catccagctc gtgcagacct acaaccagct tttcgaagag 2340

aaccctatca acgcttcagg tgtggatgct aaggctatcc tctctgctag gctctctaag 2400

tcaagaaggc ttgagaacct cattgctcag ctccctggtg agaagaagaa cggacttttc 2460

ggaaacttga tcgctctctc tctcggactc acccctaact tcaagtctaa cttcgatctc 2520

gctgaggatg caaagctcca gctctcaaag gatacctacg atgatgatct cgataacctc 2580

ctcgctcaga tcggagatca gtacgctgat ttgttcctcg ctgctaagaa cctctctgat 2640

gctatcctcc tcagtgatat cctcagagtg aacaccgaga tcaccaaggc tccactctca 2700

gcttctatga tcaagagata cgatgagcac caccaggatc tcacacttct caaggctctt 2760

gttagacagc agctcccaga gaagtacaaa gagattttct tcgatcagtc taagaacgga 2820

tacgctggtt acatcgatgg tggtgcatct caagaagagt tctacaagtt catcaagcct 2880

atcctcgaga agatggatgg aaccgaggaa ctcctcgtga agctcaatag agaggatctt 2940

ctcagaaagc agaggacctt cgataacgga tctatccctc atcagatcca cctcggagag 3000

ttgcacgcta tccttagaag gcaagaggat ttctacccat tcctcaagga taacagggaa 3060

aagattgaga agattctcac cttcagaatc ccttactacg tgggacctct cgctagagga 3120

aactcaagat tcgcttggat gaccagaaag tctgaggaaa ccatcacccc ttggaacttc 3180

gaagaggtgg tggataaggg tgctagtgct cagtctttca tcgagaggat gaccaacttc 3240

gataagaacc ttccaaacga gaaggtgctc cctaagcact ctttgctcta cgagtacttc 3300

accgtgtaca acgagttgac caaggttaag tacgtgaccg agggaatgag gaagcctgct 3360

tttttgtcag gtgagcaaaa gaaggctatc gttgatctct tgttcaagac caacagaaag 3420

gtgaccgtga agcagctcaa agaggattac ttcaagaaaa tcgagtgctt cgattcagtt 3480

gagatttctg gtgttgagga taggttcaac gcatctctcg gaacctacca cgatctcctc 3540

aagatcatta aggataagga tttcttggat aacgaggaaa acgaggatat cttggaggat 3600

atcgttctta ccctcaccct ctttgaagat agagagatga ttgaagaaag gctcaagacc 3660

tacgctcatc tcttcgatga taaggtgatg aagcagttga agagaagaag atacactggt 3720

tggggaaggc tctcaagaaa gctcattaac ggaatcaggg ataagcagtc tggaaagaca 3780

atccttgatt tcctcaagtc tgatggattc gctaacagaa acttcatgca gctcatccac 3840

gatgattctc tcacctttaa agaggatatc cagaaggctc aggtttcagg acagggtgat 3900

agtctccatg agcatatcgc taacctcgct ggatctcctg caatcaagaa gggaatcctc 3960

cagactgtga aggttgtgga tgagttggtg aaggtgatgg gaaggcataa gcctgagaac 4020

atcgtgatcg aaatggctag agagaaccag accactcaga agggacagaa gaactctagg 4080

gaaaggatga agaggatcga ggaaggtatc aaagagcttg gatctcagat cctcaaagag 4140

caccctgttg agaacactca gctccagaat gagaagctct acctctacta cctccagaac 4200

ggaagggata tgtatgtgga tcaagagttg gatatcaaca ggctctctga ttacgatgtt 4260

gatcatatcg tgccacagtc attcttgaag gatgattcta tcgataacaa ggtgctcacc 4320

aggtctgata agaacagggg taagagtgat aacgtgccaa gtgaagaggt tgtgaagaaa 4380

atgaagaact attggaggca gctcctcaac gctaagctca tcactcagag aaagttcgat 4440

aacttgacta aggctgagag gggaggactc tctgaattgg ataaggcagg attcatcaag 4500

aggcagcttg tggaaaccag gcagatcact aagcacgttg cacagatcct cgattctagg 4560

atgaacacca agtacgatga gaacgataag ttgatcaggg aagtgaaggt tatcaccctc 4620

aagtcaaagc tcgtgtctga tttcagaaag gatttccaat tctacaaggt gagggaaatc 4680

aacaactacc accacgctca cgatgcttac cttaacgctg ttgttggaac cgctctcatc 4740

aagaagtatc ctaagctcga gtcagagttc gtgtacggtg attacaaggt gtacgatgtg 4800

aggaagatga tcgctaagtc tgagcaagag atcggaaagg ctaccgctaa gtatttcttc 4860

tactctaaca tcatgaattt cttcaagacc gagattaccc tcgctaacgg tgagatcaga 4920

aagaggccac tcatcgagac aaacggtgaa acaggtgaga tcgtgtggga taagggaagg 4980

gatttcgcta ccgttagaaa ggtgctctct atgccacagg tgaacatcgt taagaaaacc 5040

gaggtgcaga ccggtggatt ctctaaagag tctatcctcc ctaagaggaa ctctgataag 5100

ctcattgcta ggaagaagga ttgggaccct aagaaatacg gtggtttcga ttctcctacc 5160

gtggcttact ctgttctcgt tgtggctaag gttgagaagg gaaagagtaa gaagctcaag 5220

tctgttaagg aacttctcgg aatcactatc atggaaaggt catctttcga gaagaaccca 5280

atcgatttcc tcgaggctaa gggatacaaa gaggttaaga aggatctcat catcaagctc 5340

ccaaagtact cactcttcga actcgagaac ggtagaaaga ggatgctcgc ttctgctggt 5400

gagcttcaaa agggaaacga gcttgctctc ccatctaagt acgttaactt tctttacctc 5460

gcttctcact acgagaagtt gaagggatct ccagaagata acgagcagaa gcaacttttc 5520

gttgagcagc acaagcacta cttggatgag atcatcgagc agatctctga gttctctaaa 5580

agggtgatcc tcgctgatgc aaacctcgat aaggtgttgt ctgcttacaa caagcacaga 5640

gataagccta tcagggaaca ggcagagaac atcatccatc tcttcaccct taccaacctc 5700

ggtgctcctg ctgctttcaa gtacttcgat acaaccatcg ataggaagag atacacctct 5760

accaaagaag tgctcgatgc taccctcatc catcagtcta tcactggact ctacgagact 5820

aggatcgatc tctcacagct cggtggtgat tcaagggctg atcctaagaa gaagaggaag 5880

gtttgaggcg cgccgagctc caggcctccc agctttcgtc cgtatcatcg gtttcgacaa 5940

cgttcgtcaa gttcaatgca tcagtttcat tgcccacaca ccagaatcct actaagtttg 6000

agtattatgg cattggaaaa gctgttttct tctatcattt gttctgcttg taatttactg 6060

tgttctttca gtttttgttt tcggacatca aaatgcaaat ggatggataa gagttaataa 6120

atgatatggt ccttttgttc attctcaaat tattattatc tgttgttttt actttaatgg 6180

gttgaattta agtaagaaag gaactaacag tgtgatatta aggtgcaatg ttagacatat 6240

aaaacagtct ttcacctctc tttggttatg tcttgaattg gtttgtttct tcacttatct 6300

gtgtaatcaa gtttactatg agtctatgat caagtaatta tgcaatcaag ttaagtacag 6360

tataggcttg agctccctag gcctgttatc cctaacaagt ttgtacaaaa aagcaggctg 6420

agctcgacca agcccgttat tctgacagtt ctggtgctca acacatttat atttatcaag 6480

gagcacattg ttactcactg ctaggaggga atcgaactag gaatattgat cagaggaact 6540

acgagagagc tgaagataac tgccctctag ctctcactga tctgggcgca tagtgagatg 6600

cagcccacgt gagttcagca acggtctagc gctgggcttt taggcccgca tgatcgggct 6660

ttgtcgggtg gtcgacgtgt tcacgattgg ggagagcaac gcagcagttc ctcttagttt 6720

agtcccacct cgcctgtcca gcagagttct gaccggttta taaactcgct tgctgcatca 6780

gacttggagg ttgggcctca ggtagtgttt tagagctaga aatagcaagt taaaataagg 6840

ctagtccgtt atcaacttga aaaagtggca ccgagtcggt gctttttttc tagacccagc 6900

tttcttgtac aaagttggca ttaacccagc tttcttgtac aaagtggttc gataattccg 6960

atccagccta ggcccgggcc tgaggacgcg tccatggtta attaagacgt ccggaccgac 7020

tagtggatcc tctagagtcg acctgcaggc atgcaagctt cttcgtcaac atggtggagc 7080

acgacacgct tgtctactcc aaaaatatca aagatacagt ctcagaagac caaagggcaa 7140

ttgagacttt tcaacaaagg gtaatatccg gaaacctcct cggattccat tgcccagcta 7200

tctgtcactt tattgtgaag atagtggaaa aggaaggtgg ctcctacaaa tgccatcatt 7260

gcgataaagg aaaggccatc gttgaagatg cctctgccga cagtggtccc aaagatggac 7320

ccccacccac gaggagcatc gtggaaaaag aagacgttcc aaccacgtct tcaaagcaag 7380

tggattgatg tgatatctcc actgacgtaa gggatgacgc acaatcaatc ccactatcct 7440

tcgcaagacc cttttaaggg ggaagttcat ttcatttgga gaggacacgc tgaaatcacc 7500

agtctctctg tacaaatcna tctctctcta taatattgtg taagtagttc ccagataagg 7560

gaattagggt tcttataggg tttcgctcag ctgttgagca tataagaaac ccttagtcga 7620

tagatctgtt ggggatctac catgagccca gaacgacgcc cggccgacat ccgccgtgcc 7680

accgaggcgg acatgccggc ggtctgcacc atcgtcaacc actacatcga gacaagcacg 7740

gtcaacttcc gtaccgagcc gcaggaaccg caggagtgga cggacgacct cgtccgtctg 7800

cgggagcgct atccctggct cgtcgccgag gtggacggcg aggtcgccgg catcgcctac 7860

gcgggcccct ggaaggcacg caacgcctac gactggacgg ccgagtcgac cgtgtacgtc 7920

tccccccgcc accagcggac gggactgggc tccacgctct acacccacct gctgaagtcc 7980

ctggaggcac agggcttcaa gagcgtggtc gctgtcatcg ggctgcccaa cgacccgagc 8040

gtgcgcatgc acgaggcgct cggatatgcc ccccgcggca tgctgcgggc ggccggcttc 8100

aagcacggga actggcatga cgtgggtttc tggcagctgg acttcagcct gccggtaccg 8160

ccccgtccgg tcctgcccgt caccgagatc tgatgaccca acttagtatg tatttgtatt 8220

tgtaaaatac ttctatcaat aaaatttcta attcctaaaa ccaaaatcca ggggtaccga 8280

acaagcttgg cactggccgt cgttttacaa cgtcgtgact gggaaaaccc tggcgttacc 8340

caacttaatc gccttgcagc acatccccct ttcgccagct ggcgtaatag cgaagaggcc 8400

cgcaccgatc gcccttccca acagttgcgc agcctgaatg gcgaatgagc ttgagcttgg 8460

atcagattgt cgtttcccgc cttcagttta aactatcagt gtttgacagg atatattggc 8520

gggtaaacct aagagaaaag agcgtttatt agaataacgg atatttaaaa gggcgtgaaa 8580

aggtttatcc gttcgtccat ttgtatgtgc atgccaacca cagggttccc 8630

<210> 31
<211> 17
<212> DNA
<213> Artificial Sequence

<220>
<223> FAM markierte Sonde

<400> 31

agcatggcaa tgatgga                                                    17

<210>  32
<211>  17
<212>  DNA
<213>  Artificial Sequence

<220>
<223>  Primer

<400>  32
gacgccggcg agaagaa                                                    17

<210>  33
<211>  19
<212>  DNA
<213>  Artificial Sequence

<220>
<223>  Primer

<400>  33
tctggctcgg acttttgca                                                  19

<210>  34
<211>  18
<212>  DNA
<213>  Artificial Sequence

<220>
<223>  VIC markierte Sonde

<400>  34
tgacaaggtt cccttcgt                                                   18

<210>  35
<211>  23
<212>  DNA
<213>  Artificial Sequence

<220>
<223>  Primer

<400>  35
acctgaagaa ggtcggctac aac                                             23

<210>  36
<211>  21
<212>  DNA
<213>  Artificial Sequence

<220>
<223>  Primer

<400>  36
tcaccctcaa acccagagat g                                               21

<210>  37

<211>  20
<212>  DNA
<213>  Artificial Sequence

<220>
<223>  Primer

<400>  37
gaatttcccc gatcgttcaa                                                    20


<210>  38
<211>  23
<212>  DNA
<213>  Artificial Sequence

<220>
<223>  Primer

<400>  38
ctgtgtggta tttgacgtcg tcg                                                23